# EUROPEAN PATENT SPECIFICATION

(11) **EP 2 909 336 B1**
(45) Date of publication and mention of the grant of the patent: **16.05.2018**
(21) Application number: 13779571.2
(22) Date of filing: 17.10.2013
(51) Int. Cl.: C12Q 1/68

(54) **DETERMINATION OF REDUCED GUT BACTERIAL DIVERSITY**
BESTIMMUNG DER REDUZIERTEN DARMBAKTERIENVIELFALT
DÉTERMINATION DE LA DIVERSITÉ BACTÉRIENNE INTESTINALE RÉDUITE

(30) Priority: 17.10.2012 EP 12306282
(43) Date of publication of application: 26.08.2015
(73) Proprietor: Institut National de la Recherche Agronomique, 75007 Paris (FR); Novo Nordisk A/S, 2880 Bagsværd (DK)
(72) Inventor: LE CHATELIER (ÉPOUSE JANNIERE), Emmanuelle, 78114 Magny Les Hameaux (FR); EHRLICH, Stanislav, 91400 Orsay (FR); PEDERSEN, Oluf Borbye, 2800 Lyngby (DK); HANSEN, Torben, 2900 Hellerup (DK)
(74) Representative: Regimbeau
(86) International application number: PCT/EP2013/071765
(87) International publication number: WO 2014/060538

(56) References cited:
- WO-A2-2011/107481
- WO-A2-2011/107482
- QIN JUNJIE ET AL: "A human gut microbial gene catalogue established by metagenomic sequencing", NATURE, NATURE PUBLISHING GROUP, UNITED KINGDOM, vol. 464, no. 7285, 4 March 2010 (2010-03-04), pages 59-65, XP008132800, ISSN: 1476-4687, DOI: 10.1038/NATURE08821 cited in the application -& Junjie Qin ET AL: "A human gut microbial gene catalogue established by metagenomic sequencing: Online supplementary information", Nature, 4 March 2010 (2010-03-04), pages 1-38, XP055052903, Retrieved from the Internet: URL:http://www.nature.com/nature/journal/v 464/n7285/extref/nature08821-s1.pdf [retrieved on 2013-02-11]
- EMMANUELLE LE CHATELIER ET AL: "Richness of human gut microbiome correlates with metabolic markers", NATURE, vol. 500, no. 7464, 28 August 2013 (2013-08-28), pages 541-546, XP55087499, ISSN: 0028-0836, DOI: 10.1038/nature12506
- AURÉLIE COTILLARD ET AL: "Dietary intervention impact on gut microbial gene richness", NATURE, vol. 500, no. 7464, 28 August 2013 (2013-08-28), pages 585-588, XP55087501, ISSN: 0028-0836, DOI: 10.1038/nature12480
- MANIMOZHIYAN ARUMUGAM ET AL: "Enterotypes of the human gut microbiome", NATURE, vol. 473, no. 7346, 20 April 2011 (2011-04-20), pages 174-180, XP055018562, ISSN: 0028-0836, DOI: 10.1038/nature09944
- ADRIANA GIONGO ET AL: "Toward defining the autoimmune microbiome for type 1 diabetes", THE ISME JOURNAL, vol. 5, no. 1, 8 July 2010 (2010-07-08), pages 82-91, XP055052908, ISSN: 1751-7362, DOI: 10.1038/ismej.2010.92
- ELISEO PAPA ET AL: "Non-Invasive Mapping of the Gastrointestinal Microbiota Identifies Children with Inflammatory Bowel Disease", PLOS ONE, vol. 7, no. 6, 1 January 2012 (2012-01-01) , page e39242, XP055052909, ISSN: 1932-6203, DOI: 10.1371/journal.pone.0039242
- JOSEC CLEMENTE ET AL: "The Impact of the Gut Microbiota on Human Health: An Integrative View", CELL, vol. 148, no. 6, 16 March 2012 (2012-03-16), pages 1258-1270, XP028471681, ISSN: 0092-8674, DOI: 10.1016/J.CELL.2012.01.035 [retrieved on 2012-02-21]

## Description

Most human diseases and syndromes have long been thought to be solely due to an alteration of the human genome. However, while mutations in human genes may correlate with some pathology, they only explain a small fraction of the clinical cases. One explanation is that a genetic predisposition requires epigenetic stimuli in order to result in an abnormal phenotype. Recent research however points toward another explanation, which is that the human microbiota plays a crucial role in the predispositions of different diseases (Clemente et al.,Cell., 148(6):1258-70, 2012).

The human microbiota comprises thousands of bacterial species, among which commensal, beneficial or pathogen bacteria. Humans host microbiota in multiple locations such as skin, lung, vagina, mouth, and gut. These microbiota are different in their location and in their bacterial composition. The gut microbiota is the largest in its composition. It is generally considered that it comprises thousands of bacterial species, weighs about 1.5 kg and constitutes a rich gene repertoire on its own, also called gut microbiome, 100 times larger than the human nuclear genome.

All of them are known to play a role in maturation of the immune system and to impact immune response. The skin microbiota has for example been shown to play a role in the development of immune diseases such as asthma or atopic dermatitis (Hanski et al., Proc Natl Acad Sci USA., 109(21):8334-9, 2012).

The gut microbiota has been shown to play a role in the development of allergies, inflammatory bowel diseases, irritable bowel syndrome and possibly metabolic and degenerative disorders such as obesity, metabolic syndrome, diabetes and cancer. While normbiosis, qualifying the normal state of the microbiota, seems to guaranty homeostasis, disbiosis, which is the distortion from normbiosis, correlates with a long list of diseases.

It has been shown for example that reduced bacterial diversity in the gut microbiota is associated with metabolic diseases such as obesity (Ley et al., Nature., 444(7122):1022-3, 2006), type I diabetes (Wen et al., Nature., 455(7216): 1109-1113, 2008; Giongo et al, ISME J., 5(1):82-91, 2011), metabolic syndrome, hepatic steatosis, necroinflammation and fibrosis nonalcoholic steatohepatitis (Machado et al., Ann Hepatol., 11(4):440-9, 2012). Altered gut microbiota is also observed in inflammatory-related pathologies such as ulcerative colitis (Sasaki et al., J Signal Transduct., 2012:704953, 2012) and paediatric inflammatory bowel diseases (Comito et al., Int J Inflam., 2012:687143, 2012). Moreover, gut microbiota is at least locally affected in colorectal cancer (Sobhani et al., PLoS One., 6(1):e16393), 2011).

As growing evidence points toward the role of reduced bacterial diversity of the gut microbiota in a broad range of diseases, it is becoming critical to be technically able to assess it precisely.

However, assessing gut bacterial diversity proves complex. Indeed, only a small proportion of the bacteria species of the gut microbiota have been identified and sequenced, mostly because most gut bacteria cannot be cultured. In addition, most bacterial species are only present at a low copy number in the gut microbiota, which makes them difficult to detect (Hamady and Knight, Genome Res., 19: 1141-1152, 2009). Therefore, most of the gut bacteria have not been taxonomically assigned yet, which restrains the use as biomarkers to taxonomically known species and genes.

The determination of reduced bacterial diversity has thus been so far generally limited to measuring the relative abundance of known bacterial species or phyla, rather than determining the abundance of all of the species of the gut microbiota. For instance, with respect to type I diabetes, it is known that the proportion of *Bacteroidetes* bacteria increase over time in unhealthy (i.e., autoantibody positive, "autoimmune") subjects, while the proportion of the *Firmicutes* bacteria increases in healthy non- type I diabetes prone subjects (Wen et al., Nature., 455(7216): 1109-1113, 2008). However, the evolution of the proportion of the non-taxonomically assigned species in connection with this disease, as well as many other, remains difficult to assess.

The current methods can therefore only report differences in the proportion of known bacteria. For this reason, they are not sensitive enough and most probably underestimate the actual population of people presenting reduced gut bacterial diversity.

There is therefore still a need for a comprehensive method to accurately determine reduced gut bacterial diversity in a subject that would rely on specific markers. Such a method would then not be limited to specific techniques or equipment and could therefore be implemented broadly.

### FIGURE LEGEND

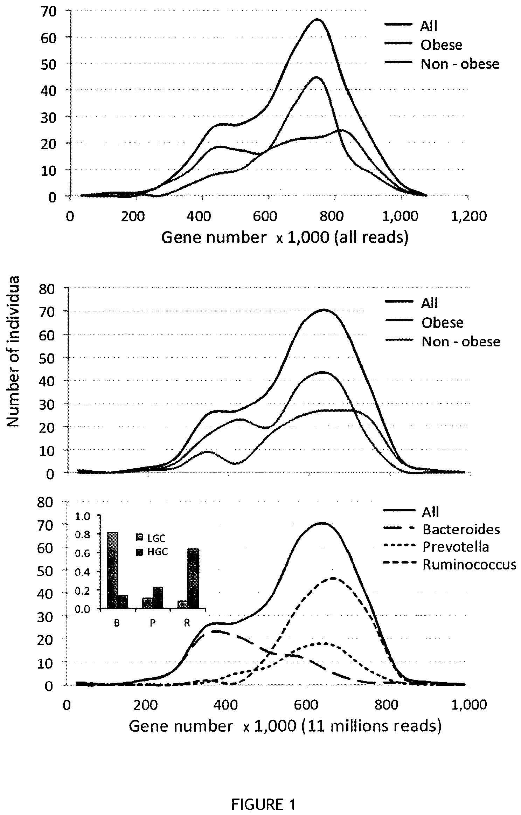

Figure 1: Distribution of low and high gene count individuals in the total population of 292 individuals. Top: Gene counts from all uniquely matched reads. Middle: Gene counts adjusted to 11 million uniquely matched reads per individual. Bottom: Gene count distributions in different enterotypes. Inset: Enterotypes of low (LGC) and high gene (HGC) individuals; B, P and R stand for Bacteroides-, Prevotella-, and Ruminococcus/Methanobrevibacter-driven enterotypes, respectively.

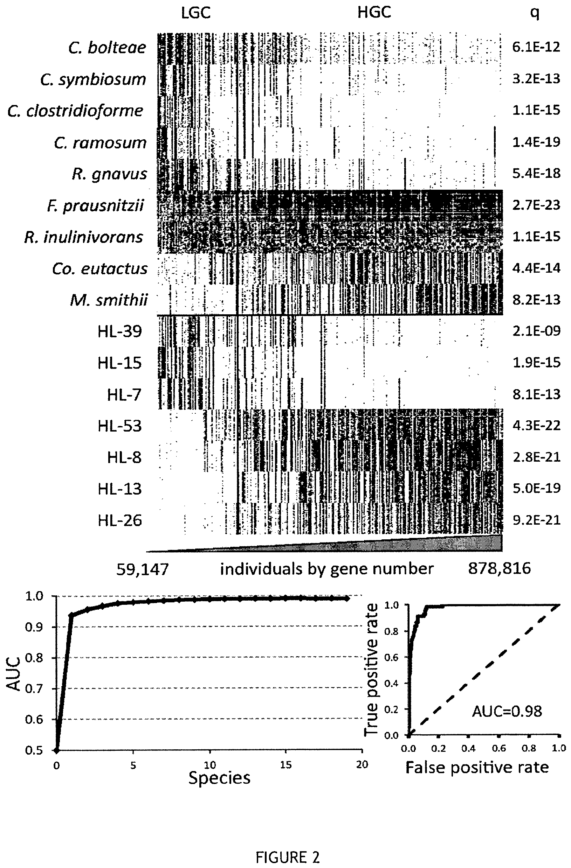

Figure 2. Bacterial species have different distribution among 292 high and low gene individuals. Top: Presence and abundance of 50 'tracer' genes nine most abundant known species and 7 unknown bacterial species. Rows correspond to genes and the relative abundance of each gene is indicated by color, increasing from light grey to intense grey; white denotes that a gene has not been detected. Columns correspond to individuals, who are ordered by increasing gene number. Values on the right side of the figure give the Wilcoxon probability (q) that a species is differentially abundant among the low and high gene individuals; the abundance of a species in an individual was computed as the mean of abundances of the tracer genes. Bottom left: AUC values obtained for the best combinations of 1 to 19 species in a ROC analysis. Bottom right: AUC for the best combination of 4 species (the 4 taxonomically unknown species with the lowest q probabilities displayed in the top part were used).

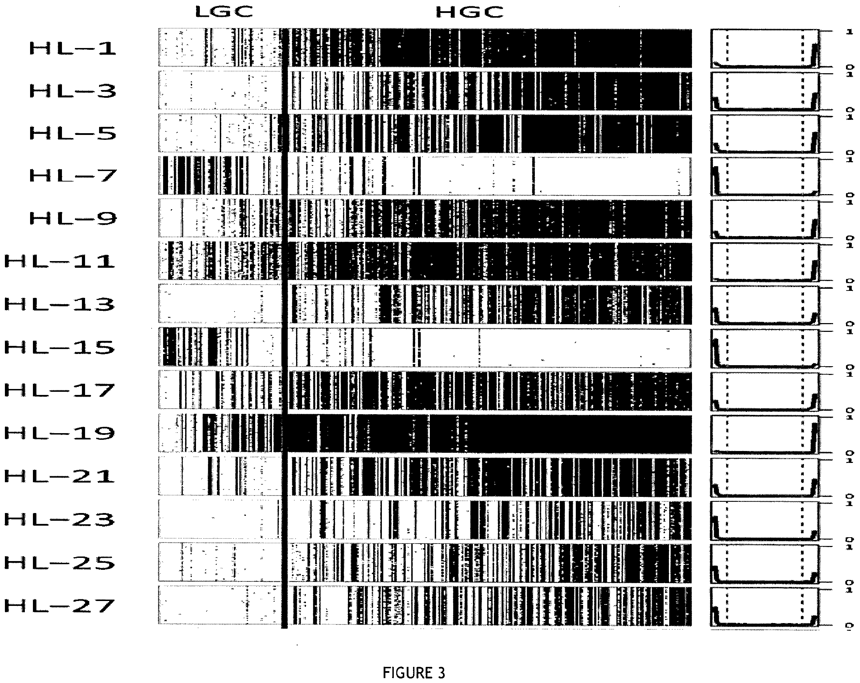

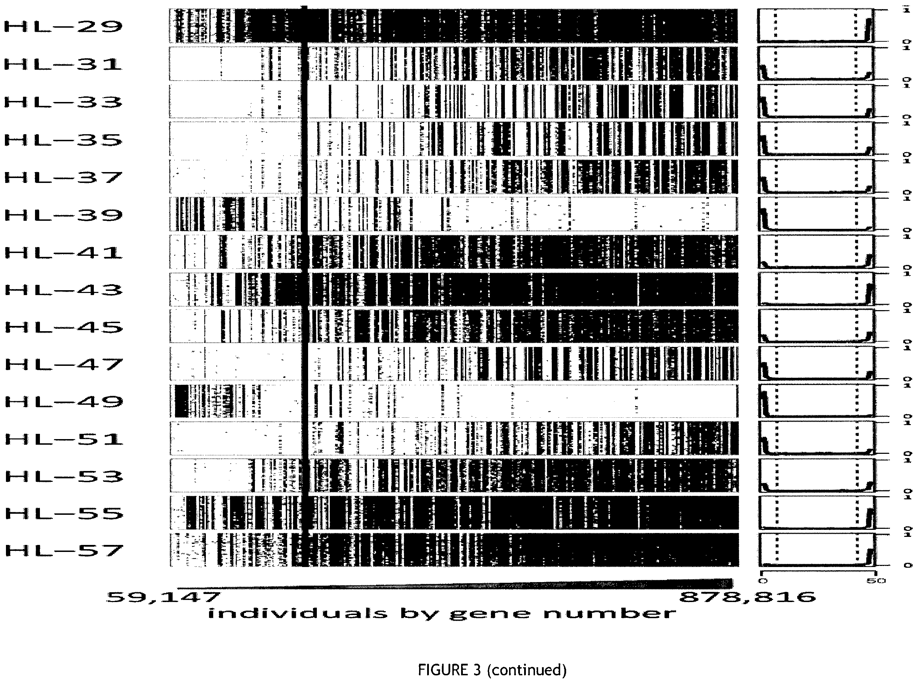

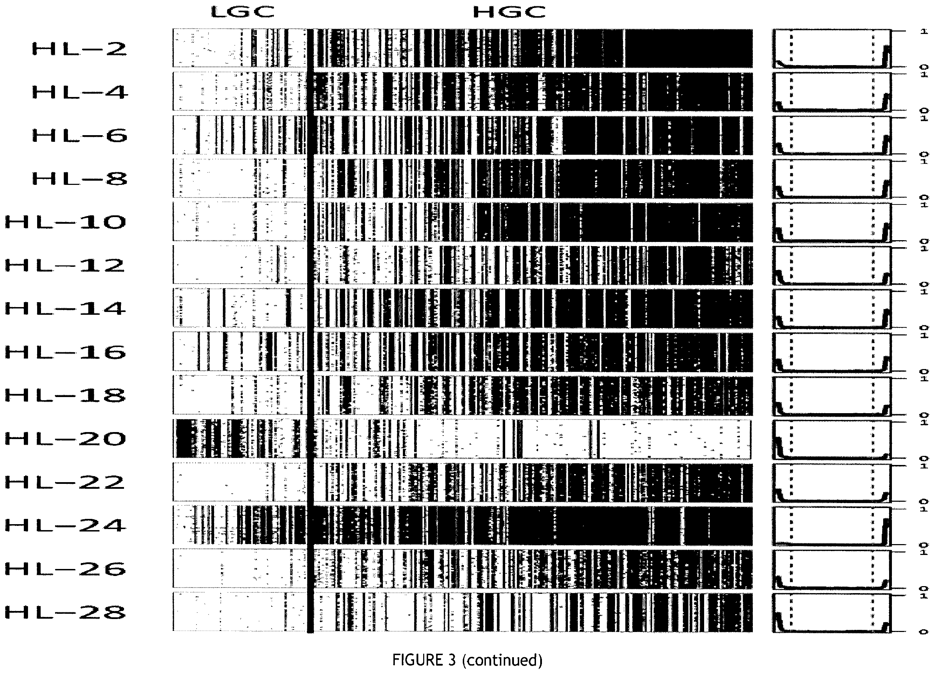

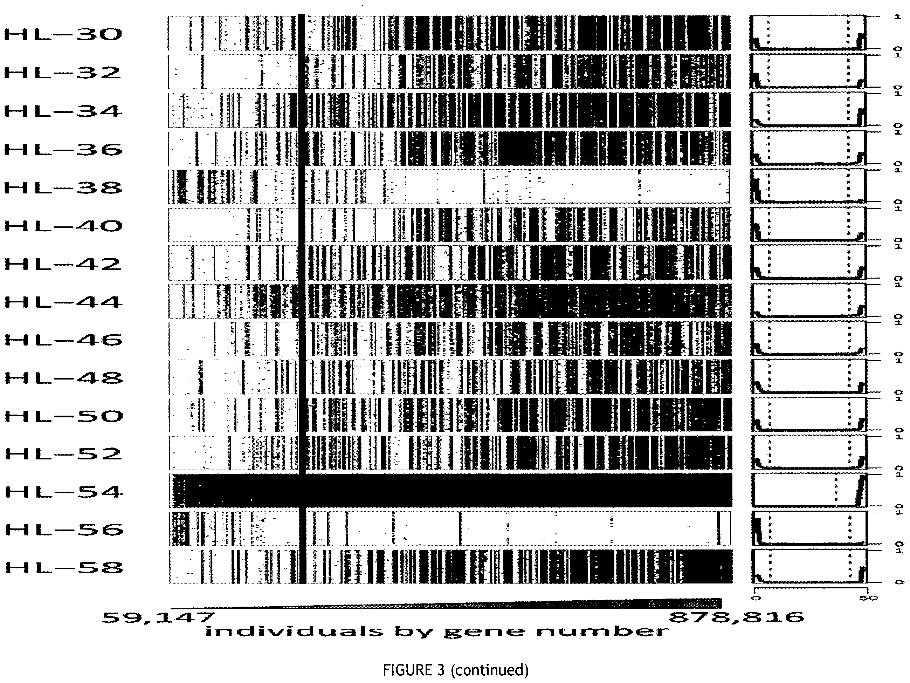

Figure 3: Presence and abundance of 50 'tracer' genes for the species significantly different in LGC and HGS individuals. Rows correspond to genes and the relative abundance of each gene is indicated by color, increasing from light grey to intense grey; white denotes undetected genes. Columns correspond to the 292 individuals of the cohort, who are ordered by increasing gene number. On the right is illustrated the fraction of individuals that have a given proportion of the tracer genes for each species; the fraction is represented in the y-axis as a percentage from 0 to 1 and the number of genes on the x-axis. Taken together 70% of individuals have none or all genes of a species; 87 have <10% or >90%.

### DESCRIPTION

The present invention is directed to a method for determining whether a subject has reduced gut bacterial diversity. Such a determination is useful, in particular for assessing whether the said subject is at risk of developing a pathology, such as e.g. type II diabetes, hyperglycemic syndrome, heart diseases, insulin resistance and hepatic stasis. The inventors have shown that it is possible to discriminate between individuals having reduced gut bacterial diversity and those having normal gut bacterial diversity by simply assessing the presence of a small number of those bacterial species in the gut.

The inventors have found a set of specific bacterial species, which presence or absence in the bacterial DNA of the faeces of a subject significantly correlates with reduced gut bacterial diversity.

By "reduced gut bacterial diversity", it is herein referred to a gut microbiota in which the number of bacterial species is reduced compared to the average normal gut microbiota.

For example, the comparison between a test microbiota and a normal gut microbiota can be achieved by the genotyping of sequences obtained from the biological samples for example with massively parallel DNA sequencing. In that case, a subject with reduced bacterial diversity can have a microbiome comprising less than 480 000 bacterial gene counts, wherein said counts were obtained by sequencing gut microbial DNA obtained from a sample of 200 mg of faeces with Illumina-based high throughput sequencing, mapping the sequences obtained onto a reference set of bacterial genome (as described in Arumugam et al., Nature., 473(7346):174-80, 2011), removing human contamination, discarding reads mapping at multiple positions, and based on the total amount of remaining matched reads.

According to the invention, a subject has either a reduced gut bacterial diversity, or a normal bacterial diversity. The skilled person would then understand easily that when the method of the invention does not determine that the overweight subject has a reduced gut bacterial diversity, said subject obviously has a normal gut bacterial diversity. By "normal gut bacterial diversity", it is herein referred to a gut microbiota in which the number of bacterial species is around the number found in the average normal gut microbiota, that is to say between 10% inferior and 10% superior to the the number of bacterial species found in the average normal gut microbiota.

By "microbiota", it is herein referred to microflora and microfauna in an ecosystem such as intestines, mouth, vagina, or lungs. In microbiology, flora (plural: floras or floræ) refers to the collective bacteria and other microorganisms in an ecosystem (e.g., some part of the body of an animal host). The "gut microbiota" consists of all the bacterial species constituting the microbiota present in the gut of an individual.

A bacterial species according to the invention encompasses not only known bacterial species but also species which have not yet been taxonomically described. Indeed, whether they already have been taxonomically described or not, bacterial species can be characterized by their genome. For example, methods for characterizing bacteria using genetic information have been described in Vandamme et al. (Microbiol. Rev. 1996, 60(2):407).

It will be obvious to the person skilled in the art that the genes of a bacterial species are physically linked as a unit rather than being independently distributed between individuals, i. e. the genome of said bacterial species comprises gene sequences which are always present or absent together among individuals. Bacterial species can therefore be defined by parts of their genome, and sequencing the entire genome of bacterial species is not necessary for proper bacterial species identification.

For instance, a method for the identification of bacterial species in a microbial composition, based on bacterial DNA sequencing and using marker genes as taxonomic references has been described in Liu et al. (BMC genomics, 12(S2):S4, 2011). The person skilled in the art may further refer to Arumugam et al. (Nature, 473(7346):174-80, 2011) or Qin et al. (Nature, 490(7418):55-60, 2012) for detailed methods for the identification of bacterial species based on bacterial DNA sequencing.

According to the present invention a "bacterial species" is a group of bacterial genes from the gut microbiome, which abundance level varies in the same proportion among different individual samples. In other words, a bacterial species according to the invention is a cluster of bacterial gene sequences which abundance levels in samples from distinct subjects are statistically linked rather than being randomly distributed. It will be immediately apparent to the skilled person that such a cluster thus corresponds to a bacterial species.

Genes of the microbiome can be ascribed to a bacterial species by several statistical methods known to the person skilled in the art. Preferably, a statistical method for testing covariance is used for testing whether two genes belong to the same cluster. To this end, the skilled person may use non-parametrical measures of statistical dependence, such as the Spearman's rank correlation coefficient for example. Most preferably, a bacterial species according to the invention is a cluster that comprises gut bacterial genes and that is determined by the method used in Qin et al. (Nature, 490(7418): 55-60, 2012) for identifying metagenomic linkage groups.

By "subject", it is herein referred to a vertebrate, preferably a mammal, and most preferably a human. There are several ways to obtain samples of the said subject's gut microbial DNA (Sokol et al., Inflamm. Bowel Dis., 14(6): 858-867, 2008). For example, it is possible to prepare mucosal specimens, or biopsies, obtained by coloscopy. However, coloscopy is an invasive procedure which is ill-defined in terms of collection procedure from study to study. Likewise, it is possible to obtain biopies through surgery. However, even more than coloscopy, surgery is an invasive procedure, which effects on the microbial population are not known. Preferred is the fecal analysis, a procedure which has been reliably been used in the art (Bullock et al., Curr Issues Intest Microbiol.; 5(2): 59-64, 2004; Manichanh et al., Gut, 55: 205-211, 2006; Bakir et al., Int J Syst Evol Microbiol, 56(5): 931-935, 2006; Manichanh et al., Nucl. Acids Res., 36(16): 5180-5188, 2008; Sokol et al., Inflamm. Bowel Dis., 14(6): 858-867, 2008). An example of this procedure is described in the Methods section of the Experimental Examples. Feces contain about 1011 bacterial cells per gram (wet weight) and bacterial cells comprise about 50 % of fecal mass. The microbiota of the feces represents primarily the microbiology of the distal large bowel. It is thus possible to isolate and analyze large quantities of microbial DNA from the feces of an individual. By "gut microbial DNA", it is herein understood the DNA from any of the resident bacterial communities of the human gut. The term "gut microbial DNA" encompasses both coding and non-coding sequences; it is in particular not restricted to complete genes, but also comprises fragments of coding sequences. Fecal analysis is thus a non-invasive procedure, which yields consistent and directly-comparable results from patient to patient.

As explained above, "gut microbiome", as used herein, refers to the set of bacterial genes from the species constituting the microbiota present in the gut of said subject. The sequences of the microbiome of the invention comprise at least gene sequences from the bacterial gene catalogue published by Qin et al. (Nature, 464: 59-65, 2010). The gene sequences from the catalogue are available from the EMBL (http:///www.bork.embl.de/∼arumugam/Qin_et_al_2010/) and BGI (http://gutmeta.genomics.org.cn) websites.

The bacterial species listed in Table 1 are absent from the gut microbiome of a significant proportion of subjects with a reduced bacterial diversity, while the bacterial species listed in Table 2 are present in the gut microbiome of a significant proportion of subjects with a reduced bacterial diversity.

These species are not limited to the ones which have already been known from prior art. Importantly, these specific bacterial species show a high correlation coefficient with reduced gut bacterial diversity. It is thus possible to determine whether a subject has reduced gut bacterial diversity with a high sensitivity. The sensitivity of a method is the proportion of actual positives which are correctly identified as such, and can be estimated by the area under the ROC (Receiver Operating Characteristic) curve, also called AUC. A receiver operating characteristic (ROC), or simply ROC curve, is a graphical plot which illustrates the performance of a binary classifier system as its discrimination threshold is varied. It is created by plotting the fraction of true positives out of the positives (TPR = true positive rate) vs. the fraction of false positives out of the negatives (FPR = false positive rate), at various threshold settings. TPR is also known as sensitivity, and FPR is one minus the specificity or true negative rate. Area Under the Curve (AUC) is a measure of a classifier/test performance across all possible values of the thresholds. The higher the AUC, the better the performance of the test.

The inventors have found that it is not necessary to determine the presence or the absence of every single species in order to assess the diversity of the gut bacterial population. Rather, said diversity can be evaluated with a high degree of confidence and accuracy by examining a very small subset of bacterial species. As shown in the experimental part, a very small number of species is a good marker of the said diversity. Indeed, even when the presence or absence of only one bacterial species is assessed, the method of the invention enables the detection of reduced bacterial diversity in a subject with an AUC of at least 0.69, and can be up to 0.936, depending of the bacterial species chosen for the test.

In comparison, a random method usually has an AUC of 0.5. Moreover, when inflammatory bowel disease, one of the pathologies associated with reduced bacterial diversity, is assessed by 16S rRNA sequencing of fecal samples, the AUC is of only 0.83 (Papa et al; PLoS One. 2012;7(6):e39242. 2012).

Therefore, the present invention is directed to a method for determining whether a subject has a reduced gut bacterial diversity according to the present set of claims.

In a first embodiment, the method of the invention is based on the determination of the presence or the absence of at least one bacterial species. Thus, according to this embodiment, the invention is directed to a method for determining whether a subject has reduced gut bacterial diversity, the said method comprising the step of detecting the presence or the absence of at least one bacterial species, preferably among the 58 bacterial species from table 1 and table 2, in the gut of the said subject. By "at least one bacterial species", it is herein meant that the presence or absence of one unique species or of more than one species is assessed. In a preferred embodiment, the method of the invention includes the detection of the presence or absence of 1, 2, 2, 4, or 5 species. Even more preferably, the said method includes the detection of the presence or absence of more than 5 species. Most preferably, the said method includes detection of the presence or absence of 58 species.

The bacterial species of the invention are chosen from the list consisting in the bacterial species of table 1 and table 2. More precisely, the bacterial species of the invention are chosen from the list consisting in HL-1, HL-2, HL-3, HL-4, HL-5, HL-6, HL-7, HL-8, HL-9, HL-10, HL-11, HL-12, HL-13, HL-14, HL-15, HL-16, HL-17, HL-18, HL-19, HL-20, HL-21, HL-22, HL-23, HL-24, HL-25, HL-26, HL-27, HL-28, HL-29, HL-30, HL-31, HL-32, HL-33, HL-34, HL-35, HL-36, HL-37, HL-38, HL-39, HL-40, HL-41, HL-42, HL-43, HL-44, HL-45, HL-46, HL-47, HL-48, HL-49, HL-50, HL-51, HL-52, HL-53, HL-54, HL-55, HL-56, HL-57, HL-58.

Most intestinal commensals cannot be cultured. Genomic strategies have been developed to overcome this limitation (Hamady and Knight, Genome Res, 19: 1141-1152, 2009). These strategies have allowed the definition of the microbiome as the collection of the genes comprised in the genomes of the microbiota (Turnbaugh et al., Nature, 449: 804-8010, 2007; Hamady and Knight, Genome Res., 19: 1141-1152, 2009). The existence of a small number of species shared by all individuals constituting the human intestinal microbiota phylogenetic core has been demonstrated (Tap et al., Environ Microbiol., 11(10): 2574-2584, 2009). Recently, a metagenomic analysis has led to the identification of an extensive catalogue of 3.3 million non-redundant microbial genes of the human gut, corresponding to 576.7 gigabases of sequence (Qin et al., Nature, 464(7285): 59-65, 2010).

It will be immediately apparent to the person of skills in the art that the presence of a bacterial species can be easily determined by detecting a nucleic acid sequence specific of the said species. The presence of gut bacterial species is usually determined by detecting 16S rRNA gene sequences. However, this method is limited to known bacterial species.

By contrast, in the method of the invention, no prior identification of the bacterial species the said gene belongs to is required. The inventors have determined a minimum set of 50 bacterial gene sequences that are non-redundant sequences for each bacterial species of table 1 and table 2, and that can be used as tracer genes.

**Table 1: bacterial species absent in subjects with reduced bacterial gut diversity**

| Bacterial species | Bacterial gene sequence |
|---|---|
| HL-1 | SEQ ID NO. 1 to 50 |
| HL-2 | SEQ ID NO. 51 to 100 |
| HL-3 | SEQ ID NO. 101 to 150 |
| HL-4 | SEQ ID NO. 151 to 200 |
| HL-5 | SEQ ID NO. 201 to 250 |
| HL-6 | SEQ ID NO. 251 to 300 |
| HL-8 | SEQ ID NO. 351 to 400 |
| HL-9 | SEQ ID NO. 401 to 450 |
| HL-10 | SEQ ID NO. 451 to 500 |
| HL-11 | SEQ ID NO. 501 to 550 |
| HL-12 | SEQ ID NO. 551 to 600 |
| HL-13 | SEQ ID NO. 601 to 650 |
| HL-14 | SEQ ID NO. 651 to 700 |
| HL-16 | SEQ ID NO. 751 to 800 |
| HL-17 | SEQ ID NO. 801 to 850 |
| HL-18 | SEQ ID NO. 851 to 900 |
| HL-19 | SEQ ID NO. 901 to 950 |
| HL-21 | SEQ ID NO. 1001 to 1050 |
| HL-22 | SEQ ID NO. 1051 to 1100 |
| HL-23 | SEQ ID NO. 1101 to 1150 |
| HL-24 | SEQ ID NO. 1151 to 1200 |
| HL-25 | SEQ ID NO. 1201 to 1250 |
| HL-26 | SEQ ID NO. 1251 to 1300 |
| HL-27 | SEQ ID NO. 1301 to 1350 |
| HL-28 | SEQ ID NO. 1351 to 1400 |
| HL-29 | SEQ ID NO. 1401 to 1450 |
| HL-30 | SEQ ID NO. 1451 to 1500 |
| HL-31 | SEQ ID NO. 1501 to 1550 |
| HL-32 | SEQ ID NO. 1551 to 1600 |
| HL-33 | SEQ ID NO. 1601 to 1650 |
| HL-34 | SEQ ID NO. 1651 to 1700 |
| HL-35 | SEQ ID NO. 1701 to 1750 |
| HL-36 | SEQ ID NO. 1751 to 1800 |
| HL-37 | SEQ ID NO. 1801 to 1850 |
| HL-40 | SEQ ID NO. 1951 to 2000 |
| HL-41 | SEQ ID NO. 2001 to 2050 |
| HL-42 | SEQ ID NO. 2051 to 2100 |
| HL-43 | SEQ ID NO. 2101 to 2150 |
| HL-44 | SEQ ID NO. 2151 to 2200 |
| HL-45 | SEQ ID NO. 2201 to 2250 |
| HL-46 | SEQ ID NO. 2251 to 2300 |
| HL-47 | SEQ ID NO. 2301 to 2350 |
| HL-48 | SEQ ID NO. 2351 to 2400 |
| HL-50 | SEQ ID NO. 2451 to 2500 |
| HL-51 | SEQ ID NO. 2501 to 2550 |
| HL-52 | SEQ ID NO. 2551 to 2600 |
| HL-53 | SEQ ID NO. 2601 to 2650 |
| HL-54 | SEQ ID NO. 2651 to 2700 |
| HL-55 | SEQ ID NO. 2701 to 2750 |
| HL-57 | SEQ ID NO. 2801 to 2850 |
| HL-58 | SEQ ID NO. 2851 to 2900 |

**Table 2: bacterial species present in subjects with reduced bacterial gut diversity**

| Bacterial species | Bacterial gene sequence |
|---|---|
| HL-7 | SEQ ID NO. 301 to 350 |
| HL-15 | SEQ ID NO. 701 to 750 |
| HL-20 | SEQ ID NO. 951 to 1000 |
| HL-38 | SEQ ID NO. 1851 to 1900 |
| HL-39 | SEQ ID NO. 1901 to 1950 |
| HL-49 | SEQ ID NO. 2401 to 2450 |
| HL-56 | SEQ ID NO. 2751 to 2800 |

It will be obvious to the person skilled in the art that the number of bacteria from a given bacterial species in a sample directly correlate with the number of copies of at least one gene sequence detected in said sample. It is thereby possible to determine the presence of at least one of the bacterial species from table 1, or the absence of at least one of the bacterial species from table 2, simply by detecting the absence of at least one bacterial gene from said species.

The invention therefore enables assessing reduced gut bacterial diversity in a subject, without the need for complex and tedious statistical analysis. Moreover, because the method of the description can rely on as little as one bacterial gene as a marker, it may be implemented by any known technique of DNA amplification or sequencing, and is not limited to a specific method or apparatus.

According to a preferred embodiment of the description, the method for determining whether a subject has a reduced gut bacterial diversity comprises a step of detecting from a gut microbial DNA sample obtained from said subject whether at least one gene from at least one bacterial species from Table 1 is absent in said sample. Alternatively, the said method comprises a step of detecting from a gut microbial DNA sample obtained from said subject whether at least one gene from at least one bacterial species from Table 2 is present in said sample. Preferably, the method of the description comprises a step of detecting from a gut microbial DNA sample obtained from said subject if at least one gene from at least one bacterial species from Table 1 is absent in said sample and at least one gene from at least one bacterial species from Table 2 is present in said sample.

Another preferred embodiment of the description is a method for determining whether a subject has a reduced gut bacterial diversity, said method comprising:
a) detecting from a gut microbial DNA sample obtained from said subject whether at least one gene from at least one bacterial species from Table 1 is absent in said sample, and
b) determining that the subject has a reduced gut bacterial diversity, if at least one gene from at least one bacterial species from Table 1 is absent in said sample.

Yet another preferred embodiment of the description is a method for determining whether a subject has a reduced gut bacterial diversity, said method comprising:
a) detecting from a gut microbial DNA sample obtained from said subject whether at least one gene from at least one bacterial species from Table 2 is present in said sample, and
b) determining that the subject has a reduced gut bacterial diversity, if at least one gene from at least one bacterial species from Table 2 is present in said sample.

In a preferred embodiment, the bacterial genes sequences of the bacterial cluster according to the description are chosen in the list consisting of sequence SEQ ID NO.1 to sequence SEQ ID NO. 2900.

Depending on the size of the sample and of the occurrence of the bacterial genes of interest, certain bacterial genes may be difficult to detect in a sample. The skilled person would thus easily conceive that, to increase the confidence of the results, it is advantageous to determine the absence of a bacterial species by detecting the average abundance of several bacterial genes from a bacterial species.

In an embodiment, detecting whether at least one bacterial gene from at least a bacterial species from table 1 is absent in said sample comprises determining the number of copies of at least 1, 2, 3, 4 or 5 bacterial gene from said bacterial species in the sample. In a preferred embodiment, detecting whether at least one bacterial gene from at least one bacterial species from table 1 is absent in said sample comprises determining the number of copies of at least 10, 20, 30, 40 or at least 50 bacterial genes from said bacterial species in the sample.

Moreover, among all of the bacterial genes, some bacterial species are more significantly correlated with reduced gut bacterial diversity than others. The detection of the presence or absence of the more correlated bacterial species can advantageously enable determining reduced gut bacterial diversity with a much better sensitivity than the methods of the prior art. For example, as shown in the experimental part, the detection of the presence or absence of one of the bacterial species HL-1, HL-57, HL-53, HL-4, HL-54, HL-2, HL-3, HL-8, HL-10, HL-45, HL-22, HL-26, HL-9, HL-5, HL-11, HL-14, HL-13, HL-18, HL-12 or HL-21 enables the detection of reduced bacterial diversity in a subject with an AUC superior to 0.83. It is thereby possible to increase the sensitivity of the method of the invention, simply by assessing the presence or absence of those specific bacterial species.

In an advantageous embodiment, the method of the description comprises a step of detecting from a gut microbial DNA sample obtained from said subject whether at least one gene from a bacterial species chosen from the list consisting in HL-1, HL-57, HL-53, HL-4, HL-54, HL-2, HL-3, HL-8, HL-10, HL-45, HL-22, HL-26, HL-9, HL-5, HL-11, HL-14, HL-13, HL-18, HL-12 HL-21 from table 1 is absent in said sample.

In a particularly advantageous embodiment, the method of the description comprises a step of detecting from a gut microbial DNA sample obtained from said subject whether at least one gene from the bacterial species HL-1 from table 1 is absent in said sample.
The person skilled in the art knows that the more distinct bacterial species from Table 1 are present in the bacterial DNA from the feces of the subject, and the more distinct bacterial species from Table 2 are absent from the bacterial DNA from the feces of the subject the higher the probability that the subjects has a reduced gut bacterial diversity. It would then be obvious to the skilled person that the sensitivity of the method of the description can be increased by assessing the presence or absence of bacterial genes from several different bacterial species from Table 1 and/or table 2. It is then possible to increase the sensitivity of the method by using bacterial genes from a linear combination of 2, 3, 4, 5 or more different bacterial species. For exemple, the combinations of 2 bacterial species from Table 1 and/or 2 enable AUC between around 0.736 and 0.955, the combinations of 3 bacterial species from Table 1 and/or 2 enable AUC between around 0.734 and 0.966, and the combinations of 4 bacterial species from Table 1 and/or 2 enable AUC between around 0.734 and 0.975. However, the inventors have surprisingly discovered that the detection of specific combinations of 2, 3 or 4 bacterial species enables for very high AUC. The more advantageous combinations of 2, 3 and 4 bacterial species are indicated in table 7, 8 and 9 respectively.

In a prefered embodiment, the method of the description comprises a step of detecting from a gut microbial DNA sample obtained from said subject whether at least one gene from each of the bacterial species of any of the bacterial species combinations indicated in table 7, 8 and/or 9 is absent and/or present in said sample.

The person skilled in the art will notice that the bacterial species combinations indicated in table 7, 8 and/or 9 are combinations of bacterial species indicated in tables 1 and 2. Therefore, the person skilled in the art will obviously understand that, detecting whether at least one gene from each of the bacterial species of a bacterial species combination indicated in table 7, 8 and/or 9 is absent and/or present in said sample corresponds to detecting wether
- at least one gene from each of the bacterial species of said combination, wherein said bacterial species is also indicated in table 1, is absent and
- at least one gene from each of the bacterial species of said combination, wherein said bacterial species is also indicated in table 2, is present,
in said sample.

The inventors have additionally selected bacterial species combinations of 2 to 20 bacterial species that enables for particularly important AUC, indicated in table 10, ranging from 0.955 to 0.982. Therefore, it is possible to achieve a great sensitivity by simply assessing the presence or absence of at least one bacterial gene from each of the bacterial species from a specific combination.

In an advantageous embodiment, the method of the description comprises a step of detecting from a gut microbial DNA sample obtained from said subject whether:
- at least one gene from each of the bacterial species HL-1 and HL-5 from table 1 are absent in said sample, or;
- at least one gene from each of the bacterial species HL-10, HL-1 and HL-5 from table 1 are absent in said sample, or;
- at least one gene from each of the bacterial species HL-8, HL-3, HL-53 and HL-26 from table 1 are absent in said sample, or;
- at least one gene from each of the bacterial species HL-10, HL-26, HL-8, HL-53 and HL-3 from table 1 are absent in said sample, or;
- at least one gene from each of the bacterial species HL-53, HL-8, HL-13, HL-3, HL-26 and HL-37 from table 1 are absent in said sample, or;
- at least one gene from each of the bacterial species HL-37, HL-26, HL-10, HL-8, HL-21, HL-53 and HL-11 from table 1 are absent in said sample, or;
- at least one gene from each of the bacterial species HL-10, HL-5, HL-26, HL-25, HL-53, HL-22, HL-8 and HL-17 from table 1 are absent in said sample, or;
- at least one gene from each of the bacterial species HL-26, HL-37, HL-21, HL-10, HL-5, HL-17, HL-16, HL-8 and HL-3 from table 1 are absent in said sample, or;
- at least one gene from each of the bacterial species HL-11, HL-27, HL-35, HL-8, HL-22, HL-47, HL-26, HL-10 and HL-37 from table 1 are absent, and at least one gene from the bacterial species HL-15 from table 2 is present, in said sample;
- at least one gene from each of the bacterial species HL-28, HL-21, HL-5, HL-27, HL-26, HL-17, HL-3, HL-40, HL-37 and HL-25 from table 1 are absent, and at least one gene from the bacterial species HL-38 from table 2 is present, in said sample;
- at least one gene from each of the bacterial species HL-8, HL-45, HL-35, HL-53, HL-17, HL-26, HL-3, HL-18, HL-10, HL-37 and HL-40 from table 1 are absent, and at least one gene from the bacterial species HL-15 from table 2 is present, in said sample;
- at least one gene from each of the bacterial species HL-33, HL-13, HL-10, HL-28, HL-36, HL-17, HL-8, HL-3, HL-22, HL-53, HL-35, HL-5 and HL-27 from table 1 are absent in said sample, or;
- at least one gene from each of the bacterial species HL-56, HL-17, HL-21, HL-35, HL-40, HL-26, HL-12, HL-13, HL-45, HL-3, HL-5, HL-10, HL-8 and HL-27 from table 1 are absent in said sample, or;
- at least one gene from each of the bacterial species HL-31, HL-11, HL-25, HL-10, HL-35, HL-12, HL-28, HL-37, HL-5, HL-33, HL-17, HL-51, HL-27 and HL-40 from table 1 are absent, and at least one gene from the bacterial species HL-15 from table 2 is present, in said sample;
- at least one gene from each of the bacterial species L-33, HL-51, HL-39, HL-27, HL-56, HL-31, HL-23, HL-10, HL-18, HL-4, HL-11, HL-8, HL-21, HL-45, HL-5 and HL-17 from table 1 are absent in said sample, or;
- at least one gene from each of the bacterial species HL-45, HL-27, HL-47, HL-5, HL-51, HL-8, HL-26, HL-3, HL-53, HL-37, HL-13, HL-11, HL-17, HL-23, HL-1 and HL-28 from table 1 are absent, and at least one gene from the bacterial species HL-38 from table 2 is present, in said sample;
- at least one gene from each of the bacterial species HL-31, HL-11, HL-33, HL-28, HL-36, HL-21, HL-22, HL-4, HL-37, HL-45, HL-27, HL-15, HL-51, HL-8 and HL-17 from table 1 are absent, and at least one gene from each of the bacterial species HL-49, HL-38 and HL-56 from table 2 are present, in said sample;
- at least one gene from each of the bacterial species, HL-18, HL-56, HL-28, HL-36, HL-45, HL-17, HL-35, HL-33, HL-11, HL-5, HL-8, HL-10, HL-12, HL-25 and HL-22 from table 1 are absent, and at least one gene from each of the bacterial species HL-39, HL-49, HL-7 and HL-15 from table 2 are present, in said sample;
- at least one gene from each of the bacterial species HL-47, HL-5, HL-36, HL-37, HL-35, HL-44, HL-11, HL-8, HL-17, HL-31, HL-18, HL-13, HL-21, HL-51, HL-4, HL-28, HL-45, HL-33 and HL-3 from table 1 are absent, and at least one gene from the bacterial species HL-15 from table 2 is present, in said sample.

A bacterial gene is absent from the sample when its number of copies in the sample is inferior to a certain threshold value. Accordingly, a bacterial gene is present in the sample when its number of copies in the sample is inferior to a certain threshold value.

According to the present invention, a "threshold value" is intended to mean a value that permits to discriminate samples in which the number of copies of the bacterial gene of interest is low or high.

In particular, if a number of copies of a bacterial gene of interest is inferior or equal to the threshold value, then the number of copies of this bacterial gene in the sample is considered low, whereas if the number of copies is superior to the threshold value, then the number of copies of this bacterial gene in the sample is considered high. A low copy number means that the bacterial gene is absent from the sample, whereas a high number of copies means that the bacterial gene is present in the sample.

For each gene, and depending on the method used for measuring the number of copies of the bacterial gene, the optimal threshold value may vary. However, it may be easily determined by a skilled person based on the analysis of the microbiome of several individuals in which the number of copies (low or high) is known for this particular bacterial gene, and on the comparison thereof with the number of copies of a control gene. Such a comparison may be facilitated by using the same amount of bacterial DNA for each of the analyzed samples, or by dividing the number of copies of the bacterial gene obtained, by the initial amount of bacterial DNA used in the test. Indeed, it is well known from the skilled person that the total amount of bacteria in the gut of a subject, and consequently in its feces, remains the same even in the case of reduced bacterial diversity. It is also possible to use a reference such as a gut bacterial species whose abundance is known not to vary between individuals with reduced and normal bacterial diversity.

According to the description, determining the number of copies of at least one bacterial gene in a sample obtained from the subject can be achieved by any technique capable of detecting and quantifying nucleic acids sequences, and include *inter alia* hybridization with a labelled probe, PCR amplification, sequencing, and all other methods known to the person of skills in the art.

In a first embodiment, determining the number of copies of at least one bacterial gene in a sample obtained from the subject is performed using sequencing. Optionally, DNA is be fragmented, for example by restriction nuclease prior to sequencing. Sequencing is done using any technique known in the state of the art, including sequencing by ligation, pyrosequencing, sequencing-by-synthesis or single-molecule sequencing. Sequencing also includes PCR-Based techniques, such as for example quantitative PCR or emulsion PCR.

Sequencing is performed on the entire DNA contained in the biological sample, or on portions of the DNA contained in the biological sample. It will be immediately clear to the skilled person that the said sample contains at least a mixture of bacterial DNA and of human DNA from the host subject. However, though the overall bacterial DNA is likely to represent the major fraction of the total DNA present in the sample, each bacterial species may only represent a small fraction of the total DNA present in the sample.

To overcome this difficulty, the skilled person can use a method that allows the quantitative genotyping of sequences obtained from the biological sample with high precision. In one embodiment of this approach, the precision is achieved by analysis of a large number (for example, millions or billions) of polynucleotides. Furthermore, the precision can be enhanced by the use of massively parallel DNA sequencing, such as, but not limited to that performed by the Illumina Genome Analyzer platform (Bentley et al. Nature; 456: 53-59, 2008), the Roche 454 platform (Margulies et al. Nature; 437: 376-380, 2005), the ABI SOLiD platform (McKernan et al., Genome Res; 19: 1527-1541, 2009), the Helicos single molecule sequencing platform (Harris et al. Science; 320: 106-109, 2008), real-time sequencing using single polymerase molecules (Science; 323: 133-138, 2009), Ion Torrent sequencing (WO 2010/008480; Rothberg et al., Nature, 475: 348-352, 2011) and nanopore sequencing (Clarke J et al. Nat Nanotechnol.; 4: 265-270, 2009).

When the skilled person relies on sequencing methods to detect the presence or absence of certain bacterial genes, the information collected from sequencing is used to determine the number of copies of nucleic acid sequences of interest via bioinformatics procedures. For example, in an embodiment, the nucleic acid sequences of said bacterial species in the gut bacterial DNA sample are identified in the global sequencing data by comparison with the nucleic acid sequences SEQ ID NO.1 to SEQ ID NO. 2900. This comparison is advantageously based on the level of sequence identity with the sequences SEQ ID NO.1 to SEQ ID NO. 2900.

Thus, a nucleic acid sequence displaying at least 90 %, at least 95 %, at least 96 %, at least 97 %, at least 98 %, at least 99 %, or 100 % identity with at least one of the nucleic acid sequences SEQ ID NO. 1 to SEQ ID NO. 2900 is identified as a sequence comprised in one of the bacterial species of the description.

Thus, in a preferred embodiment, detecting whether at least one bacterial species from table 1 is absent and/or at least one species from table 2 is present in said sample comprises determining the number of nucleic acid sequences in the gut bacterial DNA sample having at least 90 %, at least 95 %, at least 96 %, at least 97 %, at least 98 %, at least 99 %, or 100 % identity with at least one of the nucleic acid sequences SEQ ID NO. 1 to SEQ ID NO. 2900.

The term "sequence identity" herein refers to the identity between two nucleic acids sequences. Identity between sequences can be determined by comparing a position in each of the sequences which may be aligned for the purposes of comparison. When a position in the compared sequences is occupied by the same base, then the sequences are identical at that position. A degree of sequence identity between nucleic acid sequences is a function of the number of identical nucleotides at positions shared by these sequences.

To determine the percent identity of two amino acids sequences, the sequences are aligned for optimal comparison. For example, gaps can be introduced in the sequence of a first nucleic acid sequence for optimal alignment with the second nucleic acid sequence. The nucleotides at corresponding nucleotide positions are then compared. When a position in the first sequence is occupied by the same nucleotide as the corresponding position in the second sequence, the molecules are identical at that position. The percent identity between the two sequences is a function of the number of identical positions shared by the sequences. Hence % identity = number of identical positions/total number of overlapping positions X 100.

In this comparison the sequences can be the same length or can be different in length. Optimal alignment of sequences for determining a comparison window may be conducted by the local homology algorithm of Smith and Waterman (J. Theor. Biol., 91(2): 370-380, 1981), by the homology alignment algorithm of Needleman and Wunsch (J. Mol. Biol, 48(3): 443-453, 1972), by the search for similarity via the method of Pearson and Lipman (Proc. Natl. Acad. Sci. U.S.A., 85(5): 2444-2448, 1988), by computerized implementations of these algorithms (GAP, BESTFIT, FASTA and TFASTA in the Wisconsin Genetics Software Package Release 7.0, Genetic Computer Group, 575, Science Drive, Madison, Wisconsin) or by inspection. The best alignment (i.e. resulting in the highest percentage of identity over the comparison window) generated by the various methods is selected.

The term "sequence identity" thus means that two polynucleotide sequences are identical (i.e. on a nucleotide by nucleotide basis) over the window of comparison. The term "percentage of sequence identity" is calculated by comparing two optimally aligned sequences over the window of comparison, determining the number of positions at which the identical nucleic acid base (e.g. A, T, C, G, U, or I) occurs in both sequences to yield the number of matched positions, dividing the number of matched positions by the total number of positions in the window of comparison (i.e. the window size) and multiplying the result by 100 to yield the percentage of sequence identity. The same process can be applied to polypeptide sequences. The percentage of sequence identity of a nucleic acid sequence or an amino acid sequence can also be calculated using BLAST software (Version 2.06 of September 1998) with the default or user defined parameter.

In another preferred embodiment, PCR-based techniques are used to determine the number of copies of at least one bacterial gene. Preferably, the PCR technique used quantitatively measures starting amounts of DNA, cDNA, or RNA. Examples of PCR-based techniques according to the description include techniques such as, but not limited to, quantitative PCR (Q-PCR), reverse-transcriptase polymerase chain reaction (RT-PCR), quantitative reverse-transcriptase PCR (QRT-PCR), rolling circle amplification (RCA) or digital PCR. These techniques are well known and easily available technologies for those skilled in the art and do not need a precise description. In a preferred embodiment, the determination of the copy number of the bacterial genes of the description is performed by quantitative PCR.

Amplification primers specific for the genes to be tested are thus also very useful for performing the methods according to the description. The present description thus also encompasses primers for amplifying at least one gene selected from the genes of sequence SEQ ID NO. 1-2900.

In another preferred embodiment, the presence or absence of the bacterial genes according to the invention is detected by the use of a nucleic microarray.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the copy number of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

In a specific embodiment, the nucleic microarray is an oligonucleotide microarray comprising at least one oligonucleotide specific for at least one gene having a sequence selected from SEQ ID NOs 1-2900. Preferably, the said microarray comprises at least 58 oligonucleotides, each oligonucleotide being specific for one gene of a distinct cluster of the description. More preferably, the microarray of the description consists of 2900 oligonucleotides specific for each of the genes of sequences SEQ ID NOs. 1-2900.

In another embodiment, the nucleic microarray is an oligonucleotide microarray comprising at least one oligonucleotide specific for at least one gene of each of the bacterial species HL-10, HL-1 and HL-5. Preferably, the nucleic microarray is an oligonucleotide microarray comprising or consisting in oligonucleotides specific for at least 2, 3, 4, 5, 10, 20, 30 or 40 genes of each of the bacterial species HL-10, HL-1 and HL-5.

In another embodiment, the nucleic microarray is an oligonucleotide microarray comprising at least one oligonucleotide specific for at least one gene of each of the bacterial species HL-8, HL-3, HL-53 and HL-26. Preferably, the nucleic microarray is an oligonucleotide microarray comprising or consisting in oligonucleotides specific for at least 2, 3, 4, 5, 10, 20, 30 or 40 genes of each of the bacterial species HL-8, HL-3, HL-53 and HL-26.

In another embodiment, the nucleic microarray is an oligonucleotide microarray comprising at least one oligonucleotide specific for at least one gene of each of the bacterial species HL-10, HL-26, HL-8, HL-53 and HL-3. Preferably, the nucleic microarray is an oligonucleotide microarray comprising or consisting in oligonucleotides specific for at least 2, 3, 4, 5, 10, 20, 30 or 40 genes of each of the bacterial species HL-10, HL-26, HL-8, HL-53 and HL-3.

In another embodiment, the nucleic microarray is an oligonucleotide microarray comprising at least one oligonucleotide specific for at least one gene of each of the bacterial species HL-53, HL-8, HL-13, HL-3, HL-26 and HL-37. Preferably, the nucleic microarray is an oligonucleotide microarray comprising or consisting in oligonucleotides specific for at least 2, 3, 4, 5, 10, 20, 30 or 40 genes of each of the bacterial species HL-53, HL-8, HL-13, HL-3, HL-26 and HL-37.

In another embodiment, the nucleic microarray is an oligonucleotide microarray comprising at least one oligonucleotide specific for at least one gene of each of the bacterial species HL-37, HL-26, HL-10, HL-8, HL-21, HL-53 and HL-11. Preferably, the nucleic microarray is an oligonucleotide microarray comprising or consisting in oligonucleotides specific for at least 2, 3, 4, 5, 10, 20, 30 or 40 genes of each of the bacterial species HL-37, HL-26, HL-10, HL-8, HL-21, HL-53 and HL-11.

In another embodiment, the nucleic microarray is an oligonucleotide microarray comprising at least one oligonucleotide specific for at least one gene of each of the bacterial species HL-10, HL-5, HL-26, HL-25, HL-53, HL-22, HL-8 and HL-17. Preferably, the nucleic microarray is an oligonucleotide microarray comprising or consisting in oligonucleotides specific for at least 2, 3, 4, 5, 10, 20, 30 or 40 genes of each of the bacterial species HL-10, HL-5, HL-26, HL-25, HL-53, HL-22, HL-8 and HL-17.

In another embodiment, the nucleic microarray is an oligonucleotide microarray comprising at least one oligonucleotide specific for at least one gene of each of the bacterial species HL-26, HL-37, HL-21, HL-10, HL-5, HL-17, HL-16, HL-8 and HL-3 Preferably, the nucleic microarray is an oligonucleotide microarray comprising or consisting in oligonucleotides specific for at least 2, 3, 4, 5, 10, 20, 30 or 40 genes of each of the bacterial species HL-26, HL-37, HL-21, HL-10, HL-5, HL-17, HL-16, HL-8 and HL-3.

In another embodiment, the nucleic microarray is an oligonucleotide microarray comprising at least one oligonucleotide specific for at least one gene of each of the bacterial species HL-11, HL-15 HL-27, HL-35, HL-8, HL-22, HL-47, HL-26, HL-10 and HL-37. Preferably, the nucleic microarray is an oligonucleotide microarray comprising or consisting in oligonucleotides specific for at least 2, 3, 4, 5, 10, 20, 30 or 40 genes of each of the bacterial species HL-11, HL-15, HL-27, HL-35, HL-8, HL-22, HL-47, HL-26, HL-10 and HL-37.

In another embodiment, the nucleic microarray is an oligonucleotide microarray comprising at least one oligonucleotide specific for at least one gene of each of the bacterial species HL-28, HL-21, HL-5, HL-27, HL-26, HL-17, HL-3, HL-40, HL-37 HL-25 and HL-38. Preferably, the nucleic microarray is an oligonucleotide microarray comprising or consisting in oligonucleotides specific for at least 2, 3, 4, 5, 10, 20, 30 or 40 genes of each of the bacterial species HL-28, HL-21, HL-5, HL-27, HL-26, HL-17, HL-3, HL-40, HL-37 HL-25 and HL-38.

In another embodiment, the nucleic microarray is an oligonucleotide microarray comprising at least one oligonucleotide specific for at least one gene of each of the bacterial species HL-8, HL-45, HL-35, HL-53, HL-17, HL-26, HL-3, HL-18, HL-10, HL-37, HL-40 and HL-15. Preferably, the nucleic microarray is an oligonucleotide microarray comprising or consisting in oligonucleotides specific for at least 2, 3, 4, 5, 10, 20, 30 or 40 genes of each of the bacterial species HL-8, HL-45, HL-35, HL-53, HL-17, HL-26, HL-3, HL-18, HL-10, HL-37, HL-40 and HL-15.

In another embodiment, the nucleic microarray is an oligonucleotide microarray comprising at least one oligonucleotide specific for at least one gene of each of the bacterial species HL-33, HL-13, HL-10, HL-28, HL-36, HL-17, HL-8, HL-3, HL-22, HL-53, HL-35, HL-5 and HL-27. Preferably, the nucleic microarray is an oligonucleotide microarray comprising or consisting in oligonucleotides specific for at least 2, 3, 4, 5, 10, 20, 30 or 40 genes of each of the bacterial species HL-33, HL-13, HL-10, HL-28, HL-36, HL-17, HL-8, HL-3, HL-22, HL-53, HL-35, HL-5 and HL-27.

In another embodiment, the nucleic microarray is an oligonucleotide microarray comprising at least one oligonucleotide specific for at least one gene of each of the bacterial species HL-56, HL-17, HL-21, HL-35, HL-40, HL-26, HL-12, HL-13, HL-45, HL-3, HL-5, HL-10, HL-8 and HL-27. Preferably, the nucleic microarray is an oligonucleotide microarray comprising or consisting in oligonucleotides specific for at least 2, 3, 4, 5, 10, 20, 30 or 40 genes of each of the bacterial species HL-56, HL-17, HL-21, HL-35, HL-40, HL-26, HL-12, HL-13, HL-45, HL-3, HL-5, HL-10, HL-8 and HL-27.

In another embodiment, the nucleic microarray is an oligonucleotide microarray comprising at least one oligonucleotide specific for at least one gene of each of the bacterial species HL-31, HL-11, HL-25, HL-10, HL-35, HL-12, HL-28, HL-37, HL-5, HL-33, HL-17, HL-51, HL-27, HL-40 and HL-15. Preferably, the nucleic microarray is an oligonucleotide microarray comprising or consisting in oligonucleotides specific for at least 2, 3, 4, 5, 10, 20, 30 or 40 genes of each of the bacterial species HL-31, HL-11, HL-25, HL-10, HL-35, HL-12, HL-28, HL-37, HL-5, HL-33, HL-17, HL-51, HL-27, HL-40 and HL-15.

In another embodiment, the nucleic microarray is an oligonucleotide microarray comprising at least one oligonucleotide specific for at least one gene of each of the bacterial species L-33, HL-51, HL-39, HL-27, HL-56, HL-31, HL-23, HL-10, HL-18, HL-4, HL-11, HL-8, HL-21, HL-45, HL-5 and HL-17. Preferably, the nucleic microarray is an oligonucleotide microarray comprising or consisting in oligonucleotides specific for at least 2, 3, 4, 5, 10, 20, 30 or 40 genes of each of the bacterial species L-33, HL-51, HL-39, HL-27, HL-56, HL-31, HL-23, HL-10, HL-18, HL-4, HL-11, HL-8, HL-21, HL-45, HL-5 and HL-17.

In another embodiment, the nucleic microarray is an oligonucleotide microarray comprising at least one oligonucleotide specific for at least one gene of each of the bacterial species HL-45, HL-27, HL-47, HL-5, HL-51, HL-8, HL-26, HL-3, HL-53, HL-37, HL-13, HL-11, HL-17, HL-23, HL-1 and HL-28. Preferably, the nucleic microarray is an oligonucleotide microarray comprising or consisting in oligonucleotides specific for at least 2, 3, 4, 5, 10, 20, 30 or 40 genes of each of the bacterial species HL-45, HL-27, HL-47, HL-5, HL-51, HL-8, HL-26, HL-3, HL-53, HL-37, HL-13, HL-11, HL-17, HL-23, HL-1 and HL-28.

In another embodiment, the nucleic microarray is an oligonucleotide microarray comprising at least one oligonucleotide specific for at least one gene of each of the bacterial species HL-31, HL-11, HL-33, HL-28, HL-36, HL-21, HL-22, HL-4, HL-37, HL-45, HL-27, HL-15, HL-51, HL-8 and HL-17. Preferably, the nucleic microarray is an oligonucleotide microarray comprising or consisting in oligonucleotides specific for at least 2, 3, 4, 5, 10, 20, 30 or 40 genes of each of the bacterial species HL-31, HL-11, HL-33, HL-28, HL-36, HL-21, HL-22, HL-4, HL-37, HL-45, HL-27, HL-15, HL-51, HL-8 and HL-17.

In another embodiment, the nucleic microarray is an oligonucleotide microarray comprising at least one oligonucleotide specific for at least one gene of each of the bacterial species HL-18, HL-56, HL-28, HL-36, HL-45, HL-17, HL-35, HL-33, HL-11, HL-5, HL-8, HL-10, HL-12, HL-25, HL-22, HL-39,, HL-49, HL-7 and HL-15. Preferably, the nucleic microarray is an oligonucleotide microarray comprising or consisting in oligonucleotides specific for at least 2, 3, 4, 5, 10, 20, 30 or 40 genes of each of the bacterial species HL-18, HL-56, HL-28, HL-36, HL-45, HL-17, HL-35, HL-33, HL-11, HL-5, HL-8, HL-10, HL-12, HL-25, HL-22, HL-39,, HL-49, HL-7 and HL-15.

In another embodiment, the nucleic microarray is an oligonucleotide microarray comprising at least one oligonucleotide specific for at least one gene of each of the bacterial species HL-47, HL-5, HL-36, HL-37, HL-35, HL-44, HL-11, HL-8, HL-17, HL-31, HL-18, HL-13, HL-21, HL-51, HL-4, HL-28, HL-45, HL-33, HL-3 and HL-15. Preferably, the nucleic microarray is an oligonucleotide microarray comprising or consisting in oligonucleotides specific for at least 2, 3, 4, 5, 10, 20, 30 or 40 genes of each of the bacterial species HL-47, HL-5, HL-36, HL-37, HL-35, HL-44, HL-11, HL-8, HL-17, HL-31, HL-18, HL-13, HL-21, HL-51, HL-4, HL-28, HL-45, HL-33, HL-3 and HL-15.

Said microarray may further comprise at least one oligonucleotide for detecting at least one gene of at least one control bacterial species. A convenient bacterial species may be e.g. a bacterial species whose abundance does not vary between individuals with a reduced bacterial diversity and individuals with normal bacterial diversity. Preferably, the oligonucleotides are about 50 bases in length.

Suitable microarray oligonucleotides specific for any gene of SEQ ID NOs. 1-2900 may be designed, based on the genomic sequence of each gene, using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

The description further concerns a kit for the *in vitro* determination of the reduced gut bacterial diversity phenotype, comprising at least one reagent for the determination of the copy number of at least one gene having a sequence selected from SEQ ID NOs. 1-2900. By "a reagent for the determination of the copy number of at least one gene", it is meant a reagent which specifically allows for the determination of the copy number of the said gene, i.e. a reagent specifically intended for the specific determination of the copy number of at least one gene having a sequence selected from SEQ ID NOs. 1-2900. This definition excludes generic reagents useful for the determination of the expression level of any gene, such as Taq polymerase or an amplification buffer, although such reagents may also be included in a kit according to the description. Such a reagent for the determination of the copy number of at least one gene can be for example a dedicated microarray as described above or amplification primers specific for at least one gene having a sequence selected from SEQ ID NOs. 1-2900. The present description thus also relates to a kit for the *in vitro* determination of the reduced gut bacterial diversity phenotype, said kit comprising a dedicated microarray as described above or amplification primers specific for at least one gene having a sequence selected from SEQ ID NOs. 1-2900. Here also, when the kit comprises amplification primers, while said kit may comprise amplification primers specific for other genes, said kit preferably comprises at most 100, at most 75, 50, at most 40, at most 30, preferably at most 25, at most 20, at most 15, more preferably at most 10, at most 8, at most 6, even more preferably at most 5, at most 4, at most 3 or even 2 or one or even zero couples of amplification primers specific for other genes than the genes of sequences SEQ ID NOs 1-2900. For example, said kit may comprise at least a couple of amplification primers for at least one gene in addition to the primers for at least one gene having a sequence selected from SEQ ID NOs. 1-2900.

Such a kit for the in vitro determination of the reduced gut bacterial diversity phenotype may further comprise instructions for detection of the presence or absence of a responsive phenotype.

The inventors have also discovered that a low gut microbiome profile is associated with traits underlying metabolic disorders.

The inventors have compared the features of normal subjects, having a high gene count, and subjects with a reduced gut bacterial diversity, having a low gene count, and identified that the low gene count individuals, who accounted for 23% of the total study population, included a significantly higher proportion of the obese and were characterized by a more marked adiposity, as reflected by an increase in body mass index (BMI) and fat percentage. The inventors have discovered that the adiposity phenotype of low gene count individuals was associated with elevated serum leptin, decreased serum adiponectin, insulin resistance, hyperinsulinaemia, elevated levels of triglycerides and free fatty acids and a more marked inflammatory phenotype (increased C reactive protein (CRP) and elevated white blood cell count) than seen in high gene count individuals. Circulating fasting induced adipose factor (FIAF) was significantly elevated in the low gene count group - also when adjusting for BMI. These analyses indicate that the phenotypic differences of low gene count and high gene count individuals are consistent with a series of risk markers for metabolic disorders such as type II diabetes, hyperglycemic syndrome, heart diseases, insulin resistance or hepatic stasis.

The invention therefore also relates to a method to assess the risk of a subject of developing metabolic disorders, preferentially type II diabetes, hyperglycemic syndrome, heart diseases, insulin resistance or hepatic stasis, and comprising the steps of:
a) Determining whether said subject has a reduced gut bacterial diversity with a method of the invention;
b) If the subject has a reduced gut bacterial diversity, assessing the risk for said subject to develop said metabolic disorders.

Moreover, it is known from the art that a reduced gut bacterial diversity is correlated to immune disorders. In particular, it has been shown that a reduced gut bacterial diversity is associated with sensitivity to nosocomial pathogens in elderly, allergic asthma in neonatal subjects, atopic dermatitis and type I diabetes.

The invention therefore also relates to a method to assess the risk of a subject of developing immune disorders, preferentially sensitivity to nosocomial pathogens in elderly, allergic asthma in neonatal subjects, atopic dermatitis or type I diabetes, and comprising the steps of:
a) Determining whether said subject has a reduced gut bacterial diversity with a method of the invention;
b) If the subject has a reduced gut bacterial diversity, assessing the risk for said subject to develop said immune disorders.

The practice of the description employs, unless other otherwise indicated, conventional techniques or protein chemistry, molecular virology, microbiology, recombinant DNA technology, and pharmacology, which are within the skill of the art. Such techniques are explained fully in the literature. (See Ausubel et al., Current Protocols in Molecular Biology, Eds., John Wiley & Sons, Inc. New York, 1995; Remington's Pharmaceutical Sciences, 17th ed., Mack Publishing Co., Easton, Pa., 1985; and Sambrook et al., Molecular cloning: A laboratory manual 2nd edition, Cold Spring Harbor Laboratory Press - Cold Spring Harbor, NY, USA, 1989). The nomenclatures used in connection with, and the laboratory procedures and techniques of, molecular and cellular biology, protein biochemistry, enzymology and medicinal and pharmaceutical chemistry described herein are those well known and commonly used in the art.

Having generally described this invention, a further understanding of characteristics and advantages of the invention can be obtained by reference to certain specific examples and figures which are provided herein for purposes of illustration only and are not intended to be limiting unless otherwise specified.

### EXAMPLES

The abundance of known intestinal bacteria was assessed by mapping of a large number of sequencing reads from total fecal DNA onto a reference set of their genomes. The abundance of genes from the reference catalog of 292 non-obese and obese individuals was assessed.

### Study population

Study participants were recruited from the Inter99 study population. The Inter99 study is a randomized, non-pharmacological intervention study for the prevention of ischemic heart disease, and was conducted at the Research Centre for Prevention and Health in Glostrup, Denmark between 1999-2006 (clinicalTrials.gov: NCT00289237)¹. The participants in the Inter99 study were examined at baseline, after 1, 3 and 5 years depending on the type of intervention.

For the study individuals with body mass index (BMI) below 25 kg/m2 or BMI above 30 kg/m2 at year 5 in the Inter99 study were randomly selected from track records. They had no known gastro-intestinal disease, no previously bariatric surgery, no medications known to affect the immune system and no antibiotics two months prior to fecal sample collection. Individuals with type 2 diabetes at the day of examination where excluded. All together 292 non-diabetic individuals were included in the protocol. All had North European ethnicity. At the time of the current physical examination 96 (33%) of study volunteers were lean with BMI < 25 kg/m2, 27 (9 %) were overweight with BMI between 25 and 30 kg/m2, and 169 (58 %) were obese with BMI >30 kg/m2 according to World Health Organisation (WHO) definition². The study was approved by the local Ethical Committees of the Capital Region of Denmark (HC-2008-017), and was in accordance with the principals of the Declaration of Helsinki. All individuals gave written informed consent before participation in the study.

### Phenotyping

The participants were examined on two different days approximately 14 days apart. On the first day participants were examined in the morning after an over-night fast. Height was measured without shoes to the nearest 0.5 cm, and weight was measured without shoes and wearing light clothes to the nearest 0.1 kg. Hip and waist circumference were recorded using a non-expandable measuring tape to the nearest 0.5 cm. Waist circumference was measured midway between the lower rib margin and the iliac crest. Hip circumference was measured as the largest circumference between the waist and the thighs. On the second day of examination all participants delivered a stool sample collected at home and Dual-emission X-ray Absorptiometry (DXA) was performed. Analyses of data from DXA scan were conducted with the integrated software (Hologic Discovery A, Santax, USA). Sagittal height was measured at the time of the DXA scan with the use of the Holtain-Kahn abdominal Caliper at the highest point of the abdomen with the participant supine and while breathing out. Participant receiving statins, fibrates and/or ezetimibe were reported as receiving lipid lowering medication.

### Derived anthropometrical measure and indices of insulin resistance and pancreatic beta-cell function

Intra-abdominal adipose tissue (IAAT, cm²) was calculated using data from DXA scans and anthropometry using the equation³ : y = -208.2+ 4.62 (sagittal diameter, cm) + 0.75 (age, years) + 1.73 (waist, cm) + 0.78 (trunk fat, %)³. Homeostatic model assessment of insulin resistance (HOMA-IR) was calculated as: (fasting plasma glucose (mmol/l)*fasting serum insulin (mU/l))/22.5⁴.

### Biochemical measurements

All analyses were performed on blood samples drawn in the morning after an over-night fast from at least 10.00 p.m. the previous evening.

Plasma glucose was analyzed by a glucose oxidase method (Granutest, Merck, Darmstadt, Germany) with a detection limit of 0.11 mmol/l and intra- and interassay coefficients of variation (CV) of <0.8 and <1.4%, respectively. HbA1c was measured on TOSOH G7 by ion-exchange high performance liquid chromatography.

Serum insulin (excluding intact proinsulin) was measured using the AutoDELFIA insulin kit (Perkin-Elmer, Wallac, Turku, Finland) with a detection limit of 3 pmol/l and with intra- and interassay CV of <3.2% and <4.5%, respectively. Plasma total cholesterol, plasma HDL-cholesterol and plasma triglycerides were all measured on Vitros 5600 using reflect-spectrophotometrics. Blood leucocytes and white blood cell differential count were measured on Sysmex XS 1000i using flow cytometrics. Plasma alanin aminotransferase (ALT) and plasma total free fatty acids were analyzed using standard biochemical methods (Modular Evo). Plasma high sensitive C- reactive protein (hs- CRP) was analyzed by a particle-enhanced immunoturbidmetric assay on MODULAR Evo using CRPL3 kit (Roche, Mannheim, Germany) with a detection limit of 0.3 mg/l and intra- and inter CV of <4.0 % and 6.2%, respectively

Plasma adiponectin was analyzed using a two-site-sandwich ELISA kit for measuring total human adiponectin (TECO, Sissach, Switzerland). Detection limit was 0.6 ng/ml and interassay and intraassay CV were <6.72% and <4.66%, respectively. Fasting induced adipose factor (FIAF), also termed human angiopoietin like 4 (ANGPLT4) was measured using a quantitative sandwich ELISA (Adipo Bioscience, Santa Clara, USA). Detection limit was 0.6 µg/l and the inter-assay and intra-assay CV were 8% and 4%, respectively. Lipopolysaccharide binding protein was analyzed by a solid phase sandwich ELISA kit (Abnova) with an interassay CV of <17.8 % and an intraassay CV of <6.1%. Serum IL-6 and serum TNF-alfa were analysed by Luminex using the Bio-Plex Pro cytokine assay (Bio-Rad), whereas serum leptin was measured using the Bio-Plex Pro diabetes assay.

### Fecal sampling

Stool samples were obtained at the homes of each participant and samples were immediately frozen by storing them in their home freezer. Frozen samples were delivered to Steno Diabetes Center using insulating polystyrene foam containers, and stored at -80°C until analysis. The time span from sampling to delivery at the Steno Diabetes Center was aimed to be as short as possible and no more than 48 hours.

### DNA extraction

A frozen aliquot (200 mg) of each fecal sample was suspended in 250 µl of guanidine thiocyanate, 0.1 M Tris (pH 7.5) and 40 µl of 10% N-lauroyl sarcosine. Then, DNA extraction was conducted as previously described^{4,5}. The DNA concentration and its molecular size were estimated by nanodrop (Thermo Scientific) and on agarose gel electrophoresis.

### Illumina sequencing

DNA library preparation followed the manufacturer's instruction (Illumina). The workflow indicated by the provider was used to perform cluster generation, template hybridization, isothermal amplification, linearization, blocking and denaturing and hybridization of the sequencing primers. The base-calling pipeline (version IlluminaPipeline-0.3) was used to process the raw fluorescent images and call sequences.

One library (clone insert size 200 bp) was constructed for each of the first batch of 15 samples; two libraries with different clone insert sizes (135 bp and 400 bp) for each of the second batch of 70 samples, and one library (350 bp) for each of the third batch of 207 samples.

After sequencing, quality control was performed and human genome contaminant was screened. Finally, 26.0-186.1 million high-quality reads were generated for the 292 samples, with an average of 68.2 million high-quality reads. Sequencing read length of the first batch of 15 samples was 44 bp, the second batch was 75 bp, and the third batch was 75 bp and 90 bp.

### Sequence read mapping on catalogue genes

The high-quality short reads were aligned against the gene catalog using SOAP2.21⁶ by allowing at most two mismatches in the first 35-bp region and 90% identity over the read sequence. The alignment result was filtered and the uniquely -mapped pairs (paired-end reads) were counted for each gene for each sample. To reasonably and sufficiently utilize the alignment result, some of paired-end reads, one end of which was mapped on the end of a gene and the other end was missed but expected to locate on the unassembled gene region or no coding region, would be treated as correct paired-end alignment.

### Gene counting

Based on the pair-oriented counting result of each samples, the threshold of 1 read was selected for gene identification, to include the rare genes into the analysis. 91,032-1,005,488 genes were identified for the 292 samples, with an average of 670,528 genes.

### Read downsizing

To eliminate the influence of sequencing fluctuation, the alignment results were sampled and the number of mapped pairs was downsized to 11 million for each sample. After that, 59,147-878,816 genes were found for the 292 samples, with an average of 578,512 genes.

### Diversity estimate by single copy gene scoring

Genes belonging to the orthologous groups COG0085, COG0525, and COG0090 from 3,515 prokaryotic genomes were clustered to operational taxonomic units (OTUs) at 95% identity using UCLUST (Edgar, 2010) and used as a reference database. Paired-end Illumina reads from 292 metagenomic samples were mapped at 95% identity cut-off using soap2.21⁶. The numbers of fragments that were assigned to the reference sequences were counted so that each fragment's weight equals 1, i.e. a fragment assigned to N different reference sequences contributes 1/N to each reference sequence. Fragment counts of reference sequences were grouped to yield OTU counts. Samples with low sampling effort, i.e., with less than 3,000 fragments mapped to reference genes were removed leaving 229 samples for comparative analyses. OTU counts were normalized by gene length, scaled by the maximum count across all marker genes, and down-sampled using the vegan package⁷ to the minimum sum of OTU counts across all samples in order to compare species richness between high gene and low gene content groups.

### Phylogenetic microarray analysis

HITChip microarray analyses were performed as described previously⁸. In short, 16S rRNA genes were amplified the T7prom-Bact-27-for and Uni-1492-rev primers from 10 ng from fecal DNA extracts. On these amplicons an in vitro transcription and subsequent labeling with Cy3 and Cy5 dyes were performed. Labeled RNA was fragmented and hybridized on the arrays at 62.5°C for 16h in a rotation oven (Agilent Technologies, Amstelveen, The Netherlands). The arrays were washed, dried, scanned, and the signal intensity data was extracted as described (http://www.agilent.com). Microarray data normalization and analysis were carried out with a set of R-based scripts (http://r-project.org), while making use of a custom designed database, which operates under the MySQL database management system (http://www.mysql.com).

From the 3,699 unique HITChip probes, the probes that accounted for the top 99.9% of the total signal were selected. These probes were counted for each sample to measure richness, which was between 713 and 1,597 probes per sample. The probes that accounted for the lowest 0.1% of the total signal were regarded as background noise and were not taken into account for further analysis. Probe signal values were used to calculate the inverse Simpson's Diversity index for each sample.

HITChip probes specificity can be assigned to three phylogenetic levels based on 16S rRNA gene sequence similarity: order-like groups, genus-like groups (sequence similarity > 90%), and phylotype-like groups (sequence similarity > 98%)⁸. Relative abundances were calculated for each specificity level by summing all signal values of the probes targeting a group and dividing by the total of all probe signals for the corresponding sample. All comparisons between the HGC and LGC individuals were assessed with dependent 2-group Wilcoxon signed rank tests. When statistical tests were performed on a large number of variables the obtained p-values were adjusted by a Bonferroni correction. To place the gene count and BMI marker species (HL and oble, respectively) in HITChip phylogeny, Spearman correlation coefficients were calculated between the metagenomic profiling frequencies and relative abundances of the phylotype-like across 251 samples. A threshold of 0.7 was used to associate 16S to a species.

### Metagenomic microarray analysis

A 2.1 million-feature custom Roche NimbleGen microarray targeting a 700,000 genes subset of the MetaHit human gut gene catalog⁹ was designed and manufactured. The subset of genes was prioritized for genes that were observed in more than 20 of the 124 gene catalog samples. DNA extracted from fecal samples were labeled and hybridized according to standard NimbleGen protocols. Data was preprocessed and Shannon diversity index calculated using the RMA implementation under the "oligo" package and the "vegan"⁷ package, respectively, both available in the statistical programming environment R.

In order to validate the observed biomarkers for low/high gene counts found by sequencing, the data was compared to DNA microarray signals for the same samples and individuals. Thus, the tracer genes for known and unknown species indicated in Figure 2 were compared to a microarray gene set comprising more than 700,000 gut-associated genes selected from the MetaHit Gene Catalog⁹ in addition to reference genomes. Perfect matches were found for 129 tracer genes on the DNA microarray. In order to test whether a similar discrimination could be obtained from the microarray data, the samples were divided into low and high diversity sets using the Shannon diversity index. Using this index, 90 samples were categorized as low diversity, while 70 were categorized as high. Differences in DNA abundance signals between low and high diversity samples were tested for the 129 matching genes (t-test). Summarized, in terms of species the following groups were associated to high diversity, Clostridium clostridioforme, Clostridium bolteae, HL-7, HL-39, Ruminococcus gnavus, HL-15, HL-20, and Bacteroides, while HL-53 and Methanobrevibacter smithii were associated to low diversity. These DNA microarray observations support the Quantitative metagenomics results (Figure 2, Table 7 and table 7b).

### Enterotyping

For each gut microbial sample, Illumina reads were mapped to a set of 1,506 reference genomes to record genus abundances based on Bergey's taxonomy. A principal coordinate analysis was performed using JSD distance and enterotypes were assigned to each sample as described in¹⁰.

### Phylogenetic annotation

Taxonomic assignment of predicted genes for global analysis was carried out using BLASTN to assign reads to a reference genome database at a cut-off of 95% sequence identity and >100 bp overlap, unless indicated otherwise. This assignment was used as high confidence assignment on species level. As reference database we used 1,869 available reference genomes from NCBI and the set of draft gastrointestinal genomes from the DACC (http://hmpdacc.org/), both as of the 15.7.2011. The assigned reads to each taxonomic group per sample were rarefied to 5.5 million genes (the size of the smallest sample), on this rarefied matrix taxonomic groups were tested for significant differences in abundance using a Wilcoxon Ranks-Sum test. Multiple testing correction was done by controlling the False Discovery Rate (q<0.05) using the Benjamini-Hochberg method¹¹.

### Functional annotation

BLASTP was used to search the protein sequences of the predicted genes in the eggNOG database¹² and KEGG database¹³ with e-value≤1 × 10-5 as described in⁹, and the NOG/KEGG OG of the best hit was assigned to each gene. The genes annotated by COG were classified into the 25 COG categories, and genes that were annotated by KEGG were assigned to a set of manually determined gut metabolic modules [Falony et al, in prep]. The relative pathway/module abundance of higher order functional categories were calculated from rarefied KO abundances. Modules were deemed present when >=30% of the enzymes were recovered, after manual removing of overly "promiscuous" enzymes (i.e. present in multiple modules) prior to abundance calculation. For higher-level functional assignments, KO abundances were summed and distributed evenly when KOs appeared in multiple categories. Functional differences were calculated with a Wilcoxon Ranks-Sum test and multiple testing correction was done by controlling the False Discovery Rate (q<0.05) using the Benjamini-Hochberg method¹¹.

### Genes significantly different in groups of individuals

Genes significantly different in groups of individuals were identified by the Wilcoxon rank sum test coupled to a bootstrapping approach.

70% of the whole cohort (204 individuals) were randomly chosen and genes differentially abundant between LGC and HGC individuals were identified at p=<0.0001 as threshold. This test was repeated 30 times. 30 groups of randomly chosen "extreme" individuals that had <400,000 genes or >600,000 genes were composed and the same test was applied thereto. Genes common to all 60 tests were analyzed further.

For lean and obese individuals of the whole population or stratified by enterotypes, asimilar approach was used by randomly choosing 70% of individuals 30 times and using Wilcoxon rank sum test at p=<0.05.

### Gene clustering and species abundances

As only a small part (<10%) of the genes recovered as significantly different in two groups of individuals could be assigned taxonomically by sequence similarity to known reference genomes, an alternative strategy was used to cluster genes of the same species. Such genes are expected to be present at a similar abundance in an individual but at very different abundances in different individuals. The genes that vary in abundance in a coordinated way are thus likely to be from the same species. The genes were clustered according to a profile based binning strategy, using the covariance of their count profiles among the 292 individuals of the cohort. Spearman correlations coefficients were determined pairwise and all the genes that correlated above a given threshold were assigned to the same cluster.

Abundance of a given species in each individual was estimated as a mean abundance of 50 'tracer' genes of each cluster. The values were very close to the mean frequency of all the genes of a cluster.

### Receiver-Operator Characteristic (ROC) analysis

The analyses were carried out to distinguish between HGC and LGC individuals or lean and obese individuals by a combination of bacterial species. For each combination, only a single decision model was considered. In this very specific regression model weights are only allowed to take the values in. More precisely, the weight of each species in a given combination that belong to the set of the species more frequent in one group is equal to 1 while that of the species that belong to the set of species more frequent in the other group is equal to -1. The weight of each species that is outside of the combination is 0. For each individual, this model yields a score that is called the decisive-bacterial-abundance score. As opposed to the infinite number of regression models, such ternary models are finite and can be exhaustively explored. To select the best models, the cross-validated area under the ROC curve (CV-AUC) criterion¹⁴ was used, for it is well adapted to classification models for binary outcome data.

### Species correlated with the BMI change

For the entire cohort of 292 individuals, 40 individuals (14%) having the highest abundance of a species were compared with at least 125 individuals (42%) having the lowest abundance (all individuals lacking a species were included, when more numerous than 125); these numbers were chosen to allow contrasting the extremes of the distribution while keeping the sample size high enough to reduce the probability of a fortuitous difference in BMI change. For the 169 obese individuals, 30 (18%) having the highest abundance of a species were compared with at least 60 individuals (36%) having the lowest abundance (all individuals lacking a species were included, when more numerous than 60). The differences were calculated with a Student t test, the BMI changes being normally distributed, and multiple testing correction was done by controlling the False Discovery Rate (q<0.05) using the Benjamini-Hochberg method¹¹.

### Association of microbial composition and metabolic traits

We analyzed the association of 1) the high gene and low gene group and 2) gene count as a continuous trait to quantitative traits applying a linear model adjusting for age and sex.

Correlations between the quantitative traits are shown in Figure 15. P-triglycerides, P-HDL cholesterol, S-insulin, P-ALT, P-leptin and P-adiponectin and HOMA-IR were log transformed, whereas B-leucocytes, B-lymphocytes, B-monocytes, B-neutrophilocytes, P-hsCRP, S-FIAF, P-Free fatty acids, S-TNF-alfa, S-IL-6, S-lipopolysaccharide binding protein and BMI were rank normalized before analyses in the linear model. In the analyses of triglycerides, treatment with lipid lowering medications was added as a covariate to the linear model.

The data was corrected for multiple testing by the Benjamini-Hochberg method¹¹ setting the false discovery rate (FDR) at 10%. The results are displayed in Table 14.

For pair-wise analyses of the enterotype with phenotypes a linear model adjusting for age and sex was applied. The Benjamini-Hochberg method¹¹ was used to correct for multiple testing applied to the three pair-wise comparisons, again setting the false discovery rate (FDR) at10 %. The results are displayed in Table 15.

### Microbial gene abundance profiling by quantitative metagenomics

The intestinal bacterial gene content of the enrolled individuals was determined by high throughput Illumina-based sequencing of total fecal DNA. An average of 34.1 million paired-end reads were produced for each sample and, after removing human contamination (∼0.1%, on average), 19.9 ± 6.7 (s.d.) million reads were mapped at a unique position of the reference catalog of 3.3 million genes, requiring >90% identity²²; reads mapping at multiple positions (13.4 %, on average) were discarded. The abundance of a gene in a sample was estimated by dividing the number of reads that uniquely mapped to that gene by the gene length and by the total number of reads from the sample that uniquely mapped to any gene in the catalog. The resulting set of gene abundances, termed a microbial gene profile of an individual, was used for further analyses.

### A bimodal distribution of microbial genes

Comparison of gene profiles across the total study sample of 292 individuals showed a bimodal distribution of bacterial genes (Figure 1, a and b, and Table 4). 27% of individuals had <590 K genes while the remainder had more. This was even more striking among the obese individuals, whereas a broad, possibly multi-modal, distribution was observed for the non-obese (i.e. overweight and lean) individuals. We anticipate that a better insight into the fine structure of the gene distribution for the latter might be obtained when a higher number of individuals are analyzed. Nevertheless, non-obese individuals also showed a "shoulder" at <590 K genes, encompassing 20% of this group. A similar distribution was detected in obese French individuals using a different sequencing technology (Cotillard et al., accompanying paper). As the number of genes detected appeared to have some dependence on the number of matched reads (Fig 4), we compared individuals at the same number of reads. A downsizing to 11 million reads was used, and 15 individuals (5.1%) with fewer mapped reads were excluded. The bimodal distribution was again observed, both for all and for the obese individuals (Fig 1Table 4). We term hereafter the individuals with <480 K genes "low gene count" (LGC) and others "high gene count" (HGC). They had, on average, 380 K and 640 K genes, a difference of some 40% and harbored less or more rich microbiota, respectively, as shown by scoring several single copy marker genes . HITChip analysis24, based on the widely accepted 16S rDNA phylogenetic marker, confirmed both the bimodal distribution and the difference of richness of microbial communities between the LGC and HGC individuals (Fig 5).

Low richness of gut microbiota has been reported in patients with inflammatory bowel disorder (IBD) 22,25,26 and in obese individuals¹⁷, but the differences of richness within these groups or among non-obese individuals was not previously detected. As the composition of gut microbiota appears to be rather stable over long periods of adulthood²⁷ its richness may well be a characteristic feature of an individual. In mice, the richness appears to be affected by repeated antibiotic treatments (M. J. Blaser, personal communication); host genetics could also play a role, as exemplified by the knockout of the toll-like receptor 5 resulting in altered gut microbiota and the metabolic syndrome, a phenotype transmissible by fecal transplantation of the altered microbiota²⁸. Further studies, focusing specifically on the richness of the gut microbiota across broad cohorts as function of behavior, including food intake, exercise, smoking habits, other pollutants and medication over sufficiently long periods of time might help to elucidate the causes for its variation.

We determined the enterotype of the individuals in our cohort and found that enterotype distribution greatly varies with the gene count (Fig 1). Strikingly, 81.3% of the LGC individuals belonged to the Bacteroides-driven enterotype 1 while 63.4% of the HGC individuals belonged to enterotype 3 in which Ruminococcus was shown to be over-represented but which correlates even better with Methanobrevibacter in the present, larger, dataset. This distribution is significantly different (χ2 =5 x 10-22) from that expected from the distribution of enterotypes in the total study sample (29.2% of Bacteroides and 51% of Methanobrevibacter-driven enterotypes).

### HGC and LGC individuals differ by known bacterial species

Both the difference in gene number and the stratification by enterotypes indicate that the LGC and HGC individuals harbor different microbial communities. In order to assess the difference in phylogenetic composition between the two, we combined reference genome mapping with gene abundance data at phylum, genus and species level.

We first examined the general phylogenetic composition at higher taxonomic levels based upon genome size-normalized read abundances that were mapped on publicly available reference genomes and binned at genus and phylum level. 39 genera differed significantly in abundance between the HGC and LGC individuals .While Bacteroides, Parabacteroides, Ruminococcus (specifically R. torques and R. gnavus, of the Clostridium cluster XIV), Campylobacter, and Anaerostipes were more dominant in LGC, 31 genera, including Butyrivibrio, Alistipes, Akkermansia, Coprococcus, and Methanobrevibacter, were significantly linked to HGC. At the phylum level, this phylogenetic shift resulted in a higher abundance of Proteobacteria and Bacteroidetes in LGC individuals versus increased populations of Verrucomicrobia and Euryarchaeota in HGC individuals.An increased abundance of Bacteroides in the LGC individuals is congruent with the dominance of the Bacteroides-driven enterotype in this group. For clarity, it should be mentioned that the prevalent Ruminococcus in the HGC individuals and Ruminococcus/Methanobrevibacter smithii enterotype appears to be of the R.bromii-like group of the Clostridium cluster IV (HITChip results, data not shown).

Next, we studied the specific species that were differentially abundant between LGC and HGC individuals. To this aim, we used a novel, gene-centric approach that enables the visualization of individual-based patterns and avoids artifacts from incomplete genome coverage. In this approach, we identified the genes that were significantly different between the LGC and HGC individuals by the Wilcoxon rank sum test, comparing 204 (70% of total) randomly chosen individuals 30 times. We similarly compared 126 "extreme" individuals, harboring <400 K genes or >600 K genes. 120,723 genes were found in all 60 tests at p<0.0001 and were analyzed further.

We searched for genes that could belong to the same species, by comparing them to all sequenced genomes. At a threshold of 95% identity over at least 90% of the gene length, 10,225 genes (8.5%) were assigned to a total of 97 genomes representing some 73 species (Table 5). However, a vast majority (93.4%) belonged to only 9 species, which were all Firmicutes with a single exception of the main human methanogen, M. smithii. The corresponding species varied significantly in abundance between the LGC and HGC individuals, as illustrated in Fig 2, where the presence and abundance of 50 arbitrarily chosen genes from each of the 9 species in the individuals of the cohort is displayed. We suggest that such genes can be used as "tracers" of a species in any individual, as they have a sharply bi-modal distribution - 70% of individuals had either all or none of the genes from a species and thus harbored or lacked that species.The first 5 species were more frequent in LGC individuals whereas the last 4 species were more frequent in the HGC group (Fig 2).

Taken together, the analyses highlight the contrast between the distribution of antiinflammatory species, such as Faecalibacterium prausnitzii, which are more prevalent in HGC individuals and potentially pro-inflammatory, Bacteroides and R.gnavus, associated with IBD and found to be more frequent in LGC individuals.

However, a vast majority (>90%) of the 120,723 genes with significantly differing abundances in the LGC and HGC gene individuals could not be assigned to a sequenced bacterial genome, as the reference gut genome database is not yet complete. These genes must also belong to bacterial species that are present at different abundances in the two types of individuals. We thus attempted to cluster the genes from the same species by a gene abundance-based approach.

### HGC and LGC individuals differ by unknown bacterial species

We hypothesized that the genes of a given bacterial species should be present at a similar abundance in an individual but should display large variations across a cohort, as species abundance is known to vary immensely among individuals (10- to 10,000-fold)²². The genes that vary in abundance in a coordinated way are thus likely to be from the same species. We tested this hypothesis for the 10,225 taxonomically assigned genes that differ significantly between LGC and HGC individuals, by computing the Spearman correlation coefficients for each gene with all the other genes and grouping those that were correlated above a given threshold. Ninety-two clusters containing at least 2 genes and including collectively 8,594 genes (84% of the total) were found at a Spearman threshold of 0.75. A vast majority of these (8,125; 94.5%) clustered into only 8 groups that included the 9 most highly represented species shown in Fig 2 (genes assigned to Clostridium bolteae and C. clostridioforme genomes were in the same cluster). The specificity (proportion of the genes from the same species) and the sensitivity (proportion of the genes of a species included in a cluster) of clustering were very high (average of 97.8% and 91.8%, respectively) for 7 of the clusters. The specificity for the best 7 clusters increased to 99.5 % at a higher threshold (Spearman of 0.85), with the concomitant decrease of sensitivity, to 55.9%. We concluded that covariance analysis groups efficiently genes for most of the highly represented, taxonomically characterized species, and used it to cluster all the significantly different genes revealed by our rank sum analysis.

76,564 genes (63% of 120,723) were grouped into 1,440 clusters of 2 genes or more at a threshold of 0.85, used to favor the specificity of clustering, but a vast majority (68,952, 90%) was found in only 58 clusters that contained >75 genes. They included 6 of the 9 taxonomically characterized species shown in Fig 2, which grouped a total of 2,530 genes (3.7%) and clustered with an average specificity of 92% (ranging from 85.9% to 99.1%). This is somewhat lower than the values observed when 10,225 taxonomically assigned genes were clustered; possibly, some of the genes of these species were not carried on the sequenced reference genomes and were thus not taxonomically assigned. A majority of other clusters that grouped >75 genes with no clear taxonomic assignment should contain genes from previously unknown species, at a similar high specificity. To test this assumption we correlated the 16S rRNA gene sequences represented on the HITChip to these clusters. For this purpose, the abundance of each cluster, computed as the average abundance of 50 arbitrarily chosen tracer genes was compared with the hybridization signal for each of the probes contained on the HITChip across the individuals of our cohort. The 16S rRNA gene sequences correlated to 3 of the 6 clusters of known taxonomy in a congruent way and to 24 of the 52 clusters of unknown taxonomy; all of the latter were from unknown species. For the remaining 28 clusters, it is possible that the HITChip resolution of closely related genomes may have been insufficient or that the corresponding 16S sequences were lacking. We conclude that the clustering procedure grouped the genes of the same species.

Distribution of unknown species across LGC and HGC individuals of the cohort was clearly biased, as illustrated for 7 of them with 50 tracer genes (Fig 2). Genes for 10 of the species and the Bacteroides were present on the metagenomic arrays ; in all cases the HGC/LGC bias found by sequencing was also detected by the arrays (Fig 2). Characterization of the unknown species will be required in order to more fully assess the impact of the gut microbial communities on the HGC and LGC individuals.

### A small number of bacterial species allow distinguishing between HGC and LGC individuals

To test whether LGC and HGC individuals could be distinguished by bacterial species they harbour we performed a receiver-operator characteristic (ROC) analysis. First, we estimated the abundance of 58 species that were significantly different between LGC and HGC individuals (Table 4a and Table 4b). For each individual, we used these values to compute a score, named Decisive-Bacterial-Abundance (DBA) score, equal to the sum of abundances of the species more frequent in HGC individuals subtracted by the sum of the abundances of species more frequent in LGC individuals. The DBA scores were calculated exhaustively for all combinations of up to 23 species and were used in the ROC analysis; the area under curve (AUC) values for the best combinations are shown in Fig 2. The best combination of 4 species gave an AUC value of 0.98 (Fig 2); in a ten-fold cross-validation test with 90% of randomly chosen individuals the AUC value of 0.976 ± 0.02 (s.d.) was obtained for the groups of the remaining 10%, indicating the robustness of the analysis. We concluded that LGC and HGC individuals can be accurately differentiated using only a few bacterial species.

### Phenotypes of the HGC and LGC individuals

Characteristics of study materials are given in Table 10. We performed an anthropometric and biochemical phenotyping of multiple interrelated features of LGC and HGC individuals, and identified significant differences between them at a false discovery rate³⁴ of up to 10% (Table 3). This value was used to avoid missing significant associations; a less stringent level, up to 25%, was chosen in a recent and comparable study design. The LGC individuals, who represented 23% of the total study population, included a significantly higher proportion of obese participants and were as a group characterized by a more marked adiposity, as reflected by an increase in fat mass percentage and body weight (Table 3). The adiposity phenotype of LGC people was associated with elevated serum leptin, decreased serum adiponectin, insulin resistance, hyperinsulinaemia, elevated levels of triglycerides and free fatty acids (FFA), decreased HDL-cholesterol and a more marked inflammatory phenotype (increased hsCRP and higher white blood cell counts) than seen in HGC individuals (Table 3). We further tested the significance of our observations by treating the gene counts as a continuous variable and examining its correlation with the anthropometric and biochemical variables. All but two (BMI and weight) of the observed differences between LGC and HGC individuals were found significantly associated with the gene counts (Table 3). Together, these analyses suggest that the LGC individuals are featured by metabolic disturbances known to bring them at increased risk of prediabetes, type 2 diabetes and ischaemic cardiovascular disorders. Similar abnormalities were found in the accompanying paper (Cotillard et al.).

Based upon these results we hypothesize that an imbalance of potentially pro- and antiinflammatory bacterial species triggers low-grade inflammation and insulin resistance.In parallel, we suggest that an altered gut microbiota of LGC individuals induces the noted increase in serum FIAF levels, eliciting an elevated release of triglycerides and FFA (Table 3), as evidenced by studies in rodent models.

An almost perfect stratification of LGC and HGC individuals can be achieved with a very few bacterial species, suggesting that simple molecular diagnostic tests, based on our other genome, can be developed to identify individuals at risk of common morbidities. Therefore focus on our other genome, which in some respects appears to be more informative than our own, may spearhead development of stratified approaches for treatment and prevention of widespread chronic disorders.

Beyond metabolic dysfunctions, low-grade inflammation as seen in LGC individuals with and without obesity is associated with a plethora of other chronic diseases, which are steadily rising (Bach, 2002). Whether a low gut bacterial richness is common to many or even all of those, as already reported for IBD, could be revealed by exploring gut microbiota at a deep metagenomic level in a broad variety of these afflictions.

**Table 3a. Phenotypic characteristics of 292 study participants when stratified by low and high gene counts or Bacteroides/Ruminoccocus enterotypes**

| | LGC | HGC | *p* | *q* | *Gene counts p* | *Gene counts q* |
|---|---|---|---|---|---|---|
| N (men/women) | 68 (23/45) | 224 (113/111) | | | 277 (133/144) | |
| Age Yrs | 56 ± 7.5 | 57 ± 7.3 | 0.73 | 0.76 | 0.11 | 0.30 |
| BMI (kg/m²) | 32 (29 - 34) | 30 (23 - 33) | 0.035 | 0.065 | 0.06 | 0.20 |
| Weight (kg) | 91 ± 21 | 87 ± 19 | 0.019 | 0.041 | 0.15 | 0.34 |
| Fat % | 36 ± 7.8 | 31 ± 9.2 | 0.0069 | 0.024 | 0.0012 | 0.017 |
| S- Insulin (pmol/l) | 50 (35 - 91) | 44 (26 - 66) | 0.0095 | 0.03 | 0.0052 | 0.04 |
| HOMA-IR | 1.9 (1.2 - 3.3) | 1.6 (0.9 - 2.6) | 0.012 | 0.033 | 0.0059 | 0.04 |
| p-Triglycerides mmol/l | 1.3 (0.97 - 1.8) | 1.1 (0.82 - 1.6) | 0.0014 | 0.013 | 0.0007 | 0.01 |
| P-Free fatty acids (mmol/l) | 0.55 (0.39 - 0.7) | 0.48 (0.35 - 0.6) | 0.014 | 0.033 | 0.0013 | 0.017 |
| S-Leptin (µ/l) | 17 (6.7 - 33) | 8.3 (3.4 - 26) | 0.0036 | 0.02 | 0.00057 | 0.010 |
| S-Adiponectin (mg/l) | 7.5 (5.5 - 13) | 9.6 (6.7 - 14) | 0.006 | 0.024 | 0.015 | 0.079 |
| P-ALT (U/l) | 20 (14 - 30) | 19 (15 - 26) | 0.22 | 0.22 | 0.029 | 0.12 |
| B-leucocytes (10⁹/l) | 6.4 (5.2 - 7.8) | 5.6 (4.8 - 6.9) | 0.0019 | 0.013 | 0.0023 | 0.027 |
| B-Lymphocytes (10⁹/l) | 2.1 (1.6 - 2.3) | 1.8 (1.5 - 2.1) | 0.001 | 0.013 | 0.0044 | 0.0378 |
| P-CRP (mg/l) | 2.3 (1.1 - 5.7) | 1.4 (0.6 - 2.7) | 0.00088 | 0.013 | 0.0033 | 0.033 |
| S-FIAF (µg/l) | 88 (72 - 120) | 78 (60 - 100) | 0.0047 | 0.022 | 0.011 | 0.061 |

**Table 3b. Phenotypic characteristics of 292 study participants when stratified by low and high gene counts or Bacteroides/Ruminoccocus enterotypes**

| | *Bacteroides* | *Ruminococcus* | *p* | *q* |
|---|---|---|---|---|
| N (men/women) | 84 (37/47) | 148 (64/84) | | |
| Age Yrs | 56 ± 7.2 | 57 ± 7.6 | 0.33 | 0.4 |
| BMI (kg/m²) | 31 (24 - 34) | 30 (23 - 33) | 0.18 | 0.3 |
| Weight (kg) | 90 ± 21 | 85 ± 19 | 0.032 | 0.1 |
| Fat % | 34 ± 8.8 | 32 ± 9.2 | 0.058 | 0.16 |
| S-Insulin (pmol/l) | 50 (36 - 75) | 38 (23 - 64) | 0.0019 | 0.016 |
| HOMA-IR | 1.8 (1.2 - 2.8) | 1.4 (0.9 - 2.3) | 0.0019 | 0.016 |
| p-Triglycerides mmol/l | 1.2 (0.92 - 1.6) | 1.2 (0.83 - 1.6) | 0.08 | 0.19 |
| P-Free fatty acids (mmol/l) | 0.51 (0.4 - 0.65) | 0.48 (0.34 - 0.6) | 0.022 | 0.088 |
| S-Leptin (µ/l) | 11.0 (5.7 - 30) | 9.5 (3.4 - 28) | 0.08 | 0.19 |
| S-Adiponectin (mg/l) | 8.0 (5.7 - 13) | 9.9 (6.7 - 15) | 0.1 | 0.2 |
| P-ALT (U/l) | 22 (14 - 31) | 18 (15 - 24) | 0.0028 | 0.016 |
| B-leucocytes (10⁹/l) | 6.0 (4.9 - 7.4) | 5.8 (4.9 - 7) | 0.26 | 0.36 |
| B-Lymphocytes (10⁹/l) | 2.0 (1.6 - 2.3) | 1.7 (1.5 - 2) | 0.0029 | 0.016 |
| P-CRP (mg/l) | 1.8 (0.8 - 3.7) | 1.3 (0.67 - 2.6) | 0.03 | 0.1 |
| S-FIAF (µg/l) | 85 (68 - 110) | 76 (59 - 100) | 0.098 | 0.2 |

**Table 4a**

| Cluster id | Number of genes | Prevalence | Known species | Genes of known taxono my | Hit Chip Correlati on (Spearm an) | Phylotype (98% 16S sequence identity) |
|---|---|---|---|---|---|---|
| HL-1 | 14902 | HGC | | | 0.737 | Uncultured bacterium UC7-1 |
| HL-2 | 5398 | HGC | | | 0.826 | uncultured bacterium LD59 |
| HL-3 | 3807 | HGC | | | 0.940 | uncultured bacterium OLDB-C2 |
| HL-4 | 3752 | HGC | | | 0.904 | uncultured bacterium LN56 |
| HL-5 | 3237 | HGC | | | 0.933 | uncultured bacterium HuCA6 |
| HL-6 | 2784 | HGC | | | 0.819 | uncultured bacterium OLDB-H1 |
| HL-7 | 2743 | LGC | | | | |
| HL-8 | 2443 | HGC | | | | |
| HL-9 | 2105 | HGC | | | 0.895 | uncultured bacterium D726 |
| HL-10 | 1921 | HGC | | | 0.734 | bacterium adhufec101 |
| HL-11 | 1735 | HGC | | | | |
| HL-12 | 1732 | HGC | | | | |
| HL-13 | 1656 | HGC | | | 0.937 | uncultured bacterium C747 |
| HL-14 | 1642 | HGC | | | 0.932 | uncultured bacterium C736 |
| HL-15 | 1413 | LGC | | | | |
| HL-16 | 1216 | HGC | | | | |
| HL-17 | 1205 | HGC | | | 0.872 | Uncultured bacterium clone Eldhufec048 |
| HL-18 | 1118 | HGC | | | | |
| HL-19 | 1090 | HGC | | | 0.786 | bacterium adhufec269 |
| HL-20 | 1023 | LGC | *Ruminoc occus gnavus* | 963 | 0.815 | *Ruminococcus gnavus* |
| HL-21 | 938 | HGC | | | | |
| HL-22 | 902 | HGC | | | | |
| HL-23 | 894 | HGC | | | | |
| HL-24 | 867 | HGC | | | | |
| HL-25 | 744 | HGC | *Methano brevibacter smithii* | 639 | | |
| HL-26 | 733 | HGC | | | | |
| HL-27 | 513 | HGC | | | | |
| HL-28 | 454 | HGC | | | | |
| HL-29 | 403 | HGC | | | 0.850 | uncultured bacterium HuCB40 |
| HL-30 | 370 | HGC | | | 0.888 | bacterium adhufec57 |
| HL-31 | 366 | HGC | | | 0.905 | uncultured bacterium OLDB-F4 |
| HL-32 | 329 | HGC | | | 0.863 | Uncultured bacterium clone Eldhufec334 |
| HL-33 | 324 | HGC | | | | |
| HL-34 | 322 | HGC | *Coproco ccus eutactus* | 300 | 0.878 | *Coprococcus eutactus* |
| HL-35 | 286 | HGC | | | | |
| HL-36 | 277 | HGC | | | | |
| HL-37 | 260 | HGC | | | | |
| HL-38 | 235 | LGC | *Clostridi um symbiosum* | 233 | | |
| HL-39 | 234 | LGC | | | | |
| HL-40 | 230 | HGC | | | | |
| HL-41 | 214 | HGC | | | 0.804 | uncultured bacterium OLDC-E8 |
| HL-42 | 192 | HGC | | | 0.869 | uncultured bacterium OLDB-E4 |
| HL-43 | 191 | HGC | | | 0.711 | uncultured bacterium adhufec30.25 |
| HL-44 | 158 | HGC | | | 0.919 | uncultured bacterium D692 |
| HL-45 | 156 | HGC | | | | |
| HL-46 | 135 | HGC | | | | |
| HL-47 | 134 | HGC | | | 0.772 | uncultured bacterium OLDB-A9 |
| HL-48 | 126 | HGC | | | | |
| HL-49 | 125 | LGC | *Clostridiu m clostridiof orme* | 109 | 0.816 | *Clostridium clostridioforme* |
| HL-50 | 123 | HGC | | | | |
| HL-51 | 122 | HGC | | | | |
| HL-52 | 121 | HGC | | | | |
| HL-53 | 119 | HGC | | | | |
| HL-54 | 102 | HGC | | | 0.757 | uncultured bacterium C352 |
| HL-55 | 98 | HGC | | | | |
| HL-56 | 81 | LGC | *Clostridi um ramosum* | 75 | | |
| HL-57 | 77 | HGC | | | 0.719 | uncultured bacterium C352 |
| HL-58 | 75 | HGC | | | 0.862 | uncultured bacterium OLDA-H9 |

**Table 4b**

| Cluster id | Genus-like group (90% 16S sequence identity) | Order-like group | Phylum | Metagenomic arrays median p |
|---|---|---|---|---|
| HL-1 | Sporobacter termitidis et rel. | Clostridium cluster IV | Firmicutes | |
| HL-2 | Oscillospira guillermondii et rel. | Clostridium cluster IV | Firmicutes | |
| HL-3 | Uncultured Clostridiales II | Uncultured Clostridiales | Firmicutes | |
| HL-4 | Anaerovorax odorimutans et rel. | Clostridium cluster XI | Firmicutes | |
| HL-5 | Uncultured Clostridiales II | Uncultured Clostridiales | Firmicutes | |
| HL-6 | Uncultured Clostridiales II | Uncultured Clostridiales | Firmicutes | |
| HL-7 | | | | 2.5E-10 |
| HL-8 | | | | |
| HL-9 | Butyrivibrio crossotus et rel. | Clostridium cluster XIVa | Firmicutes | |
| HL-10 | Anaerotruncus colihominis et rel. | Clostridium cluster IV | Firmicutes | |
| HL-11 | | | | |
| HL-12 | | | | |
| HL-13 | Sporobacter termitidis et rel. | Clostridium cluster IV | Firmicutes | |
| HL-14 | Uncultured Clostridiales II | Uncultured Clostridiales | Firmicutes | |
| HL-15 | | | | 7.8E-06 |
| HL-16 | | | | |
| HL-17 | Bacteroides splachnicus et rel. | Bacteroidetes | Bacteroidetes | |
| HL-18 | | | | |
| HL-19 | Oscillospira guillermondii et rel. | Clostridium cluster IV | Firmicutes | |
| HL-20 | *Ruminococcus* gnavus et rel. | Clostridium cluster XIVa | Firmicutes | 4.0E-08 |
| HL-21 | | | | |
| HL-22 | | | | |
| HL-23 | | | | |
| HL-24 | | | | |
| HL-25 | | | | 8.9E-03 |
| HL-26 | | | | |
| HL-27 | | | | |
| HL-28 | | | | |
| HL-29 | Butyrivibrio crossotus et rel. | Clostridium cluster XIVa | Firmicutes | |
| HL-30 | Coprococcus eutactus et rel. | Clostridium cluster XIVa | Firmicutes | |
| HL-31 | Uncultured Clostridiales II | Uncultured Clostridiales | Firmicutes | |
| HL-32 | Uncultured Clostridiales II | Uncultured Clostridiales | Firmicutes | |
| HL-33 | | | | |
| HL-34 | Coprococcus eutactus et rel. | Clostridium cluster XIVa | Firmicutes | |
| HL-35 | | | | |
| HL-36 | | | | |
| HL-37 | | | | |
| HL-38 | | | | |
| HL-39 | | | | 8.5E-05 |
| HL-40 | | | | |
| HL-41 | Sporobacter termitidis et rel. | Clostridium cluster IV | Firmicutes | |
| HL-42 | Clostridium cellulosi et rel. | Clostridium cluster IV | Firmicutes | |
| HL-43 | Ruminococcus obeum et rel. | Clostridium cluster XIVa | Firmicutes | |
| HL-44 | Butyrivibrio crossotus et rel. | Clostridium cluster XIVa | Firmicutes | |
| HL-45 | | | | |
| HL-46 | | | | |
| HL-47 | Uncultured Clostridiales I | Uncultured Clostridiales | Firmicutes | |
| HL-48 | | | | |
| HL-49 | Clostridium svmbiosum et rel. | Clostridium cluster XIVa | Firmicutes | 1.5E-10 |
| HL-50 | | | | |
| HL-51 | | | | |
| HL-52 | | | | |
| HL-53 | | | | 1.4E-03 |
| HL-54 | Sporobacter termitidis et rel. | Clostridium cluster IV | Firmicutes | |
| HL-55 | | | | |
| HL-56 | | | | |
| HL-57 | Sporobacter termitidis et rel. | Clostridium cluster IV | Firmicutes | |
| HL-58 | Uncultured Clostridiales I | Uncultured Clostridiales | Firmicutes | |

**Table 5**

| | ***Low gene*** | ***High gene*** | | | ***Gene count*** | |
|---|---|---|---|---|---|---|
| | | | ***p*** | ***q*** | ***p*** | ***q*** |
| *N* | 64 (22/42) | 224 (113/111) | | | 277 (133/144) | |
| Age (vrs) | 56 (50 - 62) | 57 (50 - 61) | 0.9 | 0.9 | 0.81 | 0.84 |
| BMI (kg/m²) | 32 (28 - 34) | 30 (23 - 33) | 0.092 | 0.14 | 0.11 | 0.18 |
| Weight (kg) | 95 (75 - 100) | 86 (71 - 100) | **0.046** | **0.079** | 0.12 | 0.18 |
| Whole body fat percentage (%) | 35 (29 - 42) | 31 (25 - 39) | **0.017** | **0.057** | **0.0024** | **0.014** |
| Waist/hip ratio | 0.90 (0.85 - 0.96) | 0.90 (0.84 - 0.97) | 0.089 | 0.14 | **0.044** | **0.079** |
| Sagittal diameter (cm) | 24 (20 - 25) | 22 (18 - 26) | 0.4 | 0.47 | 0.21 | 0.26 |
| IAAT (cm²) | 150 (110 - 170) | 150 (93 - 180) | 0.43 | 0.48 | 0.19 | 0.24 |
| P-Glucose (mmol/l) | 5.8 (5.4 - 6.2) | 5.7 (5.4 - 6.1) | 0.3 | 0.39 | 0.19 | 0.24 |
| HbAlc (%) | 5.5 (5.3 - 5.7) | 5.6 (5.3 - 5.7) | 0.84 | 0.9 | 0.88 | 0.88 |
| S-Insulin (pmol/l) | 47 (34 - 84) | 44 (26 - 66) | **0.041** | **0.079** | **0.0052** | **0.018** |
| HOMA-IR | 1.8 (1.2 - 3.0) | 1.6 (0.9 - 2.6) | **0.044** | **0.079** | **0.0059** | **0.018** |
| P-Cholesterol (mmol/l) | 5.5 (4.8 - 6.1) | 5.4 (4.8 - 6.1) | 0.87 | 0.9 | 0.79 | 0.81 |
| P-HDL cholesterol (mmol/l) | 1.3 (1.1 - 1.7) | 1.5 (1.2 - 1.8) | **0.047** | **0.079** | 0.24 | 0.28 |
| P-Triglycerides (mmol/l) | 1.3 (1.0 - 1.8) | 1.1 (0.8 - 1.6) | **0.0028** | **0.036** | **0.00073** | **0.0062** |
| P-Free fatty acids (mmol/l) | 0.52 (0.39 - 0.69) | 0.48 (0.35 - 0.60) | **0.026** | **0.064** | **0.00042** | **0.0062** |
| P-ALT (U/l) | 20(14-31) | 19 (15 - 26) | 0.15 | 0.2 | **0.029** | **0.06** |
| S-Leptin (µ/l) | 15.0 (6.7 - 32.0) | 8.3 (3.4 - 26.0) | **0.0071** | **0.036** | **0.00058** | **0.0062** |
| S-Adiponectin (mg/l) | 7.6 (5.6 - 13.0) | 9.6 (6.7 - 14.0) | **0.032** | **0.072** | **0.016** | **0.036** |
| B-Leucocytes (10⁹/l) | 6.4 (5.2 - 7.8) | 5.6 (4.8 - 6.9) | **0.0053** | **0.036** | **0.0026** | **0.014** |
| B-Lymphocytes (10⁹/l) | 2.1 (1.6 - 2.3) | 1.8 (1.5 - 2.1) | **0.0014** | **0.036** | **0.0037** | **0.015** |
| B-Neutrophilocytes (10⁹/l) | 3.7 (2.8 - 4.8) | 3.1 (2.5 - 4.1) | **0.019** | **0.057** | **0.0092** | **0.023** |
| B-Monocytes (10⁹/l) | 0.5 (0.4 - 0.6) | 0.4 (0.4 - 0.6) | **0.0081** | **0.036** | 0.12 | 0.18 |
| P-CRP (mg/l) | 1.9 (1.0 - 5.4) | 1.4 (0.6 - 2.7) | **0.0041** | **0.036** | **0.0038** | **0.015** |
| S-IL-6 (ng/l) | 17.0 (11.0 - 31.0) | 13.0 (6.3 - 24.0) | **0.023** | **0.062** | **0.044** | **0.079** |
| S-TNFalfa (ng/l) | 13.0 (0.04 - 54.0) | 8.6 (0.04 - 32.0) | 0.13 | 0.18 | 0.46 | 0.52 |
| S-FIAF (µg/l) | 87 (72 - 120) | 78 (60 - 100) | **0.012** | **0.046** | **0.0088** | **0.023** |
| S-LBP (µg/l) | 19 (15 - 25) | 19 (15 - 23) | 0.4 | 0.47 | 0.16 | 0.23 |

**Table 6: AUC obtained with determining the presence or absence of all of the 50 genes from a given bacterial species.**

| **Bacterial species** | **AUC** | **Bacterial species** | **AUC** |
|---|---|---|---|
| HL-1 | 0.936055672268908 | HL-55 | 0.789653361344538 |
| HL-57 | 0.904083508403361 | HL-50 | 0.787847951680672 |
| HL-53 | 0.895680147058824 | HL-58 | 0.785057773109244 |
| HL-4 | 0.895647321428571 | HL-34 | 0.784729516806723 |
| HL-54 | 0.894826680672269 | HL-25 | 0.778886554621849 |
| HL-2 | 0.889705882352941 | HL-6 | 0.775013130252101 |
| HL-3 | 0.883009453781513 | HL-51 | 0.7734375 |
| HL-8 | 0.8828125 | HL-19 | 0.765887605042017 |
| HL-10 | 0.877002363445378 | HL-27 | 0.76437762605042 |
| HL-45 | 0.876838235294118 | HL-36 | 0.761357668067227 |
| HL-22 | 0.876050420168067 | HL-52 | 0.756171218487395 |
| HL-26 | 0.875525210084034 | HL-48 | 0.754759716386555 |
| HL-9 | 0.872242647058823 | HL-29 | 0.75108324579832 |
| HL-5 | 0.866530987394958 | HL-24 | 0.749015231092437 |
| HL-11 | 0.858357405462185 | HL-47 | 0.745765493697479 |
| HL-14 | 0.857110031512605 | HL-42 | 0.745338760504202 |
| HL-13 | 0.849954044117647 | HL-43 | 0.743106617647059 |
| HL-18 | 0.847098214285714 | HL-15 | 0.742450105042017 |
| HL-12 | 0.846474527310924 | HL-49 | 0.742089023109244 |
| HL-21 | 0.836594012605042 | HL-35 | 0.741530987394958 |
| HL-41 | 0.825728728991597 | HL-30 | 0.736935399159664 |
| HL-32 | 0.81906512605042 | HL-40 | 0.72780987394958 |
| HL-17 | 0.816307773109244 | HL-28 | 0.726529674369748 |
| HL-44 | 0.815027573529412 | HL-23 | 0.723608193277311 |
| HL-20 | 0.814732142857143 | HL-56 | 0.723542542016807 |
| HL-46 | 0.811548056722689 | HL-7 | 0.711758140756303 |
| HL-31 | 0.806492909663866 | HL-33 | 0.707851890756303 |
| HL-16 | 0.799041491596639 | HL-39 | 0.702271533613445 |
| HL-37 | 0.794708508403361 | HL-38 | 0.690388655462185 |

**Table 7: AUC above 0.9 are obtained when determining low gut diversity by detecting the presence or absence of 50 bacterial genes from the given combination of 2 bacterial species.**

| Combination of bacterial species | AUC | Combination of bacterial species | AUC |
|---|---|---|---|
| HL-1 and HL-5 | 0.955685399159664 | HL-10 and HL-32 | 0.91281512605042 |
| HL-1 and HL-10 | 0.953551733193277 | HL-12 and HL-13 | 0.912749474789916 |
| HL-1 and HL-3 | 0.949973739495798 | HL-13 and HL-22 | 0.912683823529412 |
| HL-1 and HL-8 | 0.946625525210084 | HL-8 and HL-25 | 0.912618172268908 |
| HL-1 and HL-28 | 0.945542279411765 | HL-5 and HL-54 | 0.912552521008403 |
| HL-1 and HL-47 | 0.945214023109244 | HL-4 and HL-7 | 0.9124868697479 |
| HL-8 and HL-26 | 0.943014705882353 | HL-31 and HL-57 | 0.912486869747899 |
| HL-1 and HL-13 | 0.942883403361345 | HL-4 and HL-15 | 0.912421218487395 |
| HL-5 and HL-57 | 0.942883403361344 | HL-18 and HL-21 | 0.912421218487395 |
| HL-1 and HL-22 | 0.942620798319328 | HL-34 and HL-54 | 0.912421218487395 |
| HL-1 and HL-35 | 0.942587972689076 | HL-25 and HL-45 | 0.912289915966386 |
| HL-1 and HL-26 | 0.94202993697479 | HL-2 and HL-53 | 0.912257090336134 |
| HL-1 and HL-25 | 0.941832983193278 | HL-10 and HL-35 | 0.912092962184874 |
| HL-1 and HL-33 | 0.941307773109244 | HL-10 and HL-49 | 0.912092962184874 |
| HL-3 and HL-53 | 0.940848214285714 | HL-18 and HL-57 | 0.912092962184874 |
| HL-1 and HL-12 | 0.940716911764706 | HL-44 and HL-57 | 0.912092962184874 |
| HL-1 and HL-27 | 0.940651260504202 | HL-26 and HL-45 | 0.911961659663866 |
| HL-10 and HL-53 | 0.940651260504202 | HL-37 and HL-57 | 0.911961659663866 |
| HL-1 and HL-4 | 0.940388655462185 | HL-11 and HL-14 | 0.911928834033613 |
| HL-1 and HL-18 | 0.940290178571429 | HL-2 and HL-22 | 0.911896008403361 |
| HL-1 and HL-23 | 0.940290178571429 | HL-12 and HL-21 | 0.911863182773109 |
| HL-10 and HL-11 | 0.939863445378151 | HL-31 and HL-53 | 0.911830357142857 |
| HL-4 and HL-26 | 0.939764968487395 | HL-10 and HL-25 | 0.911699054621849 |
| HL-26 and HL-53 | 0.939239758403361 | HL-3 and HL-27 | 0.911633403361345 |
| HL-1 and HL-37 | 0.939141281512605 | HL-4 and HL-46 | 0.911633403361344 |
| HL-1 and HL-51 | 0.939108455882353 | HL-8 and HL-27 | 0.911633403361344 |
| HL-1 and HL-53 | 0.938813025210084 | HL-40 and HL-57 | 0.911633403361344 |
| HL-8 and HL-53 | 0.938025210084034 | HL-11 and HL-13 | 0.911502100840336 |
| HL-1 and HL-31 | 0.93795955882353 | HL-13 and HL-57 | 0.911436449579832 |
| HL-10 and HL-21 | 0.937959558823529 | HL-27 and HL-57 | 0.911370798319328 |
| HL-1 and HL-21 | 0.937204569327731 | HL-49 and HL-57 | 0.911305147058824 |
| HL-3 and HL-26 | 0.937138918067227 | HL-2 and HL-47 | 0.911305147058823 |
| HL-1 and HL-46 | 0.93671218487395 | HL-5 and HL-44 | 0.911305147058823 |
| HL-1 and HL-32 | 0.936383928571429 | HL-10 and HL-56 | 0.911272321428572 |
| HL-13 and HL-53 | 0.936351102941176 | HL-2 and HL-46 | 0.911272321428571 |
| HL-8 and HL-11 | 0.935924369747899 | HL-21 and HL-32 | 0.911239495798319 |
| HL-8 and HL-21 | 0.935497636554622 | HL-2 and HL-8 | 0.911173844537815 |
| HL-5 and HL-11 | 0.93546481092437 | HL-12 and HL-22 | 0.911173844537815 |
| HL-1 and HL-40 | 0.935202205882353 | HL-46 and HL-53 | 0.911173844537815 |
| HL-3 and HL-4 | 0.934939600840336 | HL-7 and HL-53 | 0.911108193277311 |
| HL-1 andHL-11 | 0.934808298319328 | HL-26 and HL-51 | 0.911108193277311 |
| HL-1 and HL-9 | 0.934414390756303 | HL-22 and HL-25 | 0.911042542016807 |
| HL-22 and HL-26 | 0.933626575630252 | HL-38 and HL-57 | 0.910911239495799 |
| HL-8 and HL-45 | 0.933560924369748 | HL-23 and HL-57 | 0.910911239495798 |
| HL-8 and HL-9 | 0.933035714285714 | HL-8 and HL-44 | 0.910878413865546 |
| HL-3 and HL-5 | 0.932707457983193 | HL-1 and HL-20 | 0.910845588235294 |
| HL-5 and HL-13 | 0.932280724789916 | HL-33 and HL-57 | 0.91077993697479 |
| HL-10 and HL-22 | 0.93218224789916 | HL-4 and HL-28 | 0.910484506302521 |
| HL-3 and HL-8 | 0.932116596638656 | HL-10 and HL-15 | 0.910386029411764 |
| HL-4 and HL-8 | 0.931755514705882 | HL-13 and HL-37 | 0.910254726890756 |
| HL-13 and HL-26 | 0.931427258403361 | HL-32 and HL-53 | 0.910254726890756 |
| HL-4 and HL-10 | 0.931263130252101 | HL-2 and HL-17 | 0.910189075630252 |
| HL-5 and HL-45 | 0.931131827731092 | HL-3 and HL-15 | 0.91015625 |
| HL-1 and HL-36 | 0.931066176470588 | HL-35 and HL-57 | 0.910123424369748 |
| HL-3 and HL-18 | 0.930015756302521 | HL-11 and HL-18 | 0.909959296218487 |
| HL-4 and HL-5 | 0.930015756302521 | HL-26 and HL-37 | 0.909959296218487 |
| HL-2 and HL-5 | 0.929982930672269 | HL-13 and HL-49 | 0.909860819327731 |
| HL-1 and HL-49 | 0.92985162815126 | HL-15 and HL-53 | 0.909860819327731 |
| HL-1 and HL-45 | 0.929785976890757 | HL-4 and HL-9 | 0.909762342436975 |
| HL-1 and HL-14 | 0.929359243697479 | HL-25 and HL-57 | 0.909663865546218 |
| HL-1 and HL-2 | 0.929326418067227 | HL-29 and HL-54 | 0.909663865546218 |
| HL-1 and HL-15 | 0.929326418067227 | HL-3 and HL-36 | 0.909466911764706 |
| HL-5 and HL-17 | 0.929096638655462 | HL-22 and HL-57 | 0.909466911764706 |
| HL-8 and HL-18 | 0.929096638655462 | HL-2 and HL-31 | 0.909401260504202 |
| HL-3 and HL-10 | 0.929030987394958 | HL-2 and HL-21 | 0.909269957983193 |
| HL-21 and HL-53 | 0.92889968487395 | HL-8 and HL-36 | 0.908974527310924 |
| HL-3 and HL-11 | 0.928768382352941 | HL-5 and HL-46 | 0.908941701680672 |
| HL-10 and HL-45 | 0.928669905462185 | HL-16 and HL-57 | 0.908941701680672 |
| HL-1 and HL-57 | 0.927980567226891 | HL-35 and HL-45 | 0.908876050420168 |
| HL-10 and HL-57 | 0.927980567226891 | HL-1 and HL-48 | 0.908843224789916 |
| HL-10 and HL-18 | 0.927947741596639 | HL-12 and HL-18 | 0.908810399159664 |
| HL-25 and HL-53 | 0.927914915966387 | HL-8 and HL-49 | 0.908613445378151 |
| HL-4 and HL-21 | 0.927849264705882 | HL-36 and HL-57 | 0.908613445378151 |
| HL-8 and HL-13 | 0.927849264705882 | HL-45 and HL-47 | 0.908580619747899 |
| HL-3 and HL-45 | 0.927422531512605 | HL-5 and HL-41 | 0.908547794117647 |
| HL-1 and HL-17 | 0.927127100840336 | HL-9 and HL-26 | 0.908547794117647 |
| HL-10 and HL-17 | 0.927061449579832 | HL-2 and HL-57 | 0.908482142857143 |
| HL-4 and HL-11 | 0.927061449579831 | HL-10 and HL-27 | 0.908416491596639 |
| HL-5 and HL-26 | 0.92702862394958 | HL-51 and HL-53 | 0.908416491596639 |
| HL-4 and HL-53 | 0.925814075630252 | HL-4 and HL-49 | 0.908416491596638 |
| HL-18 and HL-53 | 0.925551470588235 | HL-51 and HL-57 | 0.908416491596638 |
| HL-10 and HL-13 | 0.925518644957983 | HL-8 and HL-32 | 0.908383665966387 |
| HL-1 and HL-56 | 0.925485819327731 | HL-3 and HL-25 | 0.908350840336134 |
| HL-3 and HL-21 | 0.925420168067227 | HL-12 and HL-45 | 0.908350840336134 |
| HL-1 and HL-16 | 0.925190388655463 | HL-9 and HL-53 | 0.908318014705882 |
| HL-3 and HL-22 | 0.92515756302521 | HL-28 and HL-53 | 0.90828518907563 |
| HL-4 and HL-13 | 0.924960609243697 | HL-14 and HL-57 | 0.908219537815126 |
| HL-5 and HL-21 | 0.924960609243697 | HL-8 and HL-15 | 0.908088235294118 |
| HL-1 and HL-7 | 0.924796481092437 | HL-9 and HL-13 | 0.907924107142857 |
| HL-4 and HL-22 | 0.924730829831933 | HL-7 and HL-8 | 0.907891281512605 |
| HL-5 and HL-10 | 0.924698004201681 | HL-26 and HL-31 | 0.907825630252101 |
| HL-10 and HL-46 | 0.924665178571429 | HL-21 and HL-27 | 0.907694327731092 |
| HL-4 and HL-14 | 0.924501050420168 | HL-21 and HL-37 | 0.907694327731092 |
| HL-5 and HL-25 | 0.924435399159664 | HL-22 and HL-47 | 0.907694327731092 |
| HL-35 and HL-53 | 0.924402573529412 | HL-23 and HL-53 | 0.907628676470588 |
| HL-8 and HL-22 | 0.924238445378151 | HL-2 and HL-9 | 0.907563025210084 |
| HL-12 and HL-26 | 0.924041491596639 | HL-8 and HL-31 | 0.907563025210084 |
| HL-5 and HL-14 | 0.924008665966386 | HL-14 and HL-22 | 0.907563025210084 |
| HL-3 and HL-17 | 0.923713235294118 | HL-5 and HL-47 | 0.90749737394958 |
| HL-9 and HL-10 | 0.923384978991596 | HL-9 and HL-14 | 0.90749737394958 |
| HL-22 and HL-53 | 0.923319327731092 | HL-8 and HL-37 | 0.907431722689076 |
| HL-18 and HL-26 | 0.923253676470588 | HL-32 and HL-57 | 0.907431722689075 |
| HL-11 and HL-57 | 0.922859768907563 | HL-21 and HL-45 | 0.907300420168067 |
| HL-1 and HL-44 | 0.922794117647059 | HL-27 and HL-45 | 0.906906512605042 |
| HL-5 and HL-22 | 0.922728466386554 | HL-4 and HL-12 | 0.906808035714286 |
| HL-1 and HL-38 | 0.92266281512605 | HL-3 and HL-44 | 0.906775210084034 |
| HL-10 and HL-26 | 0.922465861344538 | HL-4 and HL-20 | 0.906709558823529 |
| HL-4 and HL-25 | 0.922137605042017 | HL-15 and HL-57 | 0.906709558823529 |
| HL-2 and HL-3 | 0.921973476890756 | HL-14 and HL-32 | 0.906578256302521 |
| HL-5 and HL-8 | 0.921842174369748 | HL-11 and HL-27 | 0.906545430672269 |
| HL-4 and HL-35 | 0.921678046218487 | HL-28 and HL-45 | 0.906446953781513 |
| HL-5 and HL-9 | 0.921448266806723 | HL-2 and HL-25 | 0.906315651260504 |
| HL-4 and HL-27 | 0.921382615546219 | HL-2 and HL-27 | 0.90625 |
| HL-4 and HL-45 | 0.921382615546218 | HL-4 and HL-17 | 0.90625 |
| HL-8 and HL-10 | 0.921382615546218 | HL-13 and HL-56 | 0.905921743697479 |
| HL-5 and HL-53 | 0.92062762605042 | HL-10 and HL-47 | 0.905856092436975 |
| HL-4 and HL-47 | 0.920594800420168 | HL-41 and HL-57 | 0.905856092436975 |
| HL-8 and HL-17 | 0.920594800420168 | HL-11 and HL-45 | 0.90579044117647 |
| HL-18 and HL-22 | 0.920529149159664 | HL-2 and HL-4 | 0.905593487394958 |
| HL-8 and HL-57 | 0.920430672268908 | HL-10 and HL-31 | 0.905593487394958 |
| HL-9 and HL-57 | 0.920299369747899 | HL-4 and HL-23 | 0.905560661764706 |
| HL-22 and HL-35 | 0.920299369747899 | HL-9 and HL-45 | 0.905527836134454 |
| HL-8 and HL-47 | 0.920102415966387 | HL-46 and HL-57 | 0.905462184873949 |
| HL-8 and HL-20 | 0.92000393907563 | HL-4 and HL-51 | 0.905396533613446 |
| HL-21 and HL-26 | 0.919971113445378 | HL-13 and HL-46 | 0.905265231092437 |
| HL-3 and HL-9 | 0.919774159663865 | HL-2 and HL-35 | 0.905199579831933 |
| HL-8 and HL-35 | 0.919511554621849 | HL-14 and HL-45 | 0.905166754201681 |
| HL-14 and HL-26 | 0.91938025210084 | HL-18 and HL-32 | 0.905035451680672 |
| HL-26 and HL-35 | 0.919248949579832 | HL-9 and HL-18 | 0.904969800420168 |
| HL-4 and HL-18 | 0.919051995798319 | HL-11 and HL-26 | 0.904936974789916 |
| HL-11 and HL-53 | 0.919051995798319 | HL-2 and HL-12 | 0.904805672268908 |
| HL-37 and HL-53 | 0.919019170168067 | HL-4 and HL-56 | 0.904805672268908 |
| HL-1 and HL-39 | 0.918887867647059 | HL-22 and HL-51 | 0.904674369747899 |
| HL-1 and HL-6 | 0.918855042016807 | HL-11 and HL-12 | 0.904477415966386 |
| HL-5 and HL-35 | 0.91875656512605 | HL-13 and HL-31 | 0.904411764705882 |
| HL-17 and HL-57 | 0.918723739495799 | HL-18 and HL-25 | 0.904411764705882 |
| HL-3 and HL-20 | 0.918658088235294 | HL-1 and HL-42 | 0.904313287815127 |
| HL-26 and HL-27 | 0.918625262605042 | HL-2 and HL-33 | 0.904017857142857 |
| HL-47 and HL-53 | 0.918526785714286 | HL-2 and HL-51 | 0.903952205882353 |
| HL-25 and HL-26 | 0.918461134453782 | HL-13 and HL-32 | 0.903952205882353 |
| HL-3 and HL-32 | 0.918264180672269 | HL-8 and HL-33 | 0.903886554621849 |
| HL-14 and HL-53 | 0.917870273109244 | HL-33 and HL-45 | 0.903820903361345 |
| HL-12 and HL-53 | 0.917837447478991 | HL-2 and HL-14 | 0.903820903361344 |
| HL-8 and HL-46 | 0.917804621848739 | HL-8 and HL-28 | 0.903623949579832 |
| HL-53 and HL-57 | 0.917607668067227 | HL-10 and HL-37 | 0.90359112394958 |
| HL-4 and HL-31 | 0.917279411764706 | HL-9 and HL-27 | 0.903394170168067 |
| HL-27 and HL-53 | 0.917246586134454 | HL-1 and HL-50 | 0.903361344537816 |
| HL-3 and HL-56 | 0.917148109243697 | HL-5 and HL-37 | 0.903230042016807 |
| HL-33 and HL-53 | 0.917082457983193 | HL-54 and HL-55 | 0.903230042016807 |
| HL-2 and HL-45 | 0.916819852941176 | HL-11 and HL-22 | 0.903098739495798 |
| HL-3 and HL-57 | 0.916819852941176 | HL-20 and HL-53 | 0.902836134453781 |
| HL-21 and HL-22 | 0.916622899159664 | HL-49 and HL-53 | 0.902540703781513 |
| HL-1 and HL-52 | 0.916491596638656 | HL-56 and HL-57 | 0.90250787815126 |
| HL-2 and HL-11 | 0.916491596638655 | HL-8 and HL-16 | 0.902048319327731 |
| HL-5 and HL-18 | 0.916491596638655 | HL-22 and HL-27 | 0.902048319327731 |
| HL-13 and HL-18 | 0.916261817226891 | HL-54 and HL-57 | 0.902048319327731 |
| HL-4 and HL-33 | 0.916130514705882 | HL-7 and HL-21 | 0.902015493697479 |
| HL-2 and HL-13 | 0.915966386554622 | HL-21 and HL-31 | 0.901917016806723 |
| HL-5 and HL-12 | 0.915966386554622 | HL-10 and HL-54 | 0.901785714285715 |
| HL-5 and HL-20 | 0.915966386554622 | HL-22 and HL-49 | 0.901785714285714 |
| HL-26 and HL-47 | 0.915900735294118 | HL-2 and HL-56 | 0.90172006302521 |
| HL-3 and HL-37 | 0.915703781512605 | HL-10 and HL-44 | 0.90172006302521 |
| HL-26 and HL-32 | 0.915703781512605 | HL-23 and HL-45 | 0.901588760504202 |
| HL-4 and HL-57 | 0.915572478991596 | HL-3 and HL-7 | 0.901457457983194 |
| HL-3 and HL-13 | 0.915506827731092 | HL-22 and HL-23 | 0.901457457983193 |
| HL-8 and HL-56 | 0.915506827731092 | HL-13 and HL-15 | 0.901391806722689 |
| HL-45 and HL-57 | 0.915441176470588 | HL-4 and HL-40 | 0.901358981092437 |
| HL-45 and HL-53 | 0.91530987394958 | HL-5 and HL-36 | 0.901326155462185 |
| HL-10 and HL-14 | 0.915112920168067 | HL-4 and HL-38 | 0.901227678571428 |
| HL-18 and HL-45 | 0.915112920168067 | HL-2 and HL-37 | 0.901194852941176 |
| HL-26 and HL-57 | 0.915047268907563 | HL-48 and HL-54 | 0.901129201680672 |
| HL-2 and HL-10 | 0.914915966386555 | HL-53 and HL-56 | 0.900997899159664 |
| HL-2 and HL-18 | 0.914915966386555 | HL-3 and HL-16 | 0.900965073529412 |
| HL-3 and HL-46 | 0.914522058823529 | HL-4 and HL-16 | 0.90093224789916 |
| HL-2 and HL-26 | 0.914390756302521 | HL-8 and HL-39 | 0.90093224789916 |
| HL-13 and HL-21 | 0.914325105042017 | HL-9 and HL-25 | 0.900768119747899 |
| HL-8 and HL-14 | 0.914292279411765 | HL-2 and HL-32 | 0.90063681722689 |
| HL-3 and HL-31 | 0.914259453781513 | HL-20 and HL-57 | 0.900603991596638 |
| HL-4 and HL-32 | 0.9140625 | HL-21 and HL-35 | 0.900571165966387 |
| HL-47 and HL-57 | 0.913996848739496 | HL-48 and HL-57 | 0.900538340336134 |
| HL-1 and HL-41 | 0.913931197478992 | HL-10 and HL-41 | 0.900505514705882 |
| HL-8 and HL-12 | 0.913799894957983 | HL-3 and HL-14 | 0.90047268907563 |
| HL-4 and HL-37 | 0.913635766806723 | HL-42 and HL-54 | 0.90047268907563 |
| HL-13 and HL-45 | 0.91343881302521 | HL-47 and HL-54 | 0.900407037815126 |
| HL-28 and HL-57 | 0.91327468487395 | HL-5 and HL-42 | 0.900341386554622 |
| HL-21 and HL-57 | 0.913209033613445 | HL-50 and HL-57 | 0.900341386554622 |
| HL-3 and HL-12 | 0.913044905462185 | HL-7 and HL-54 | 0.900275735294118 |
| HL-39 and HL-57 | 0.913012079831933 | HL-13 and HL-20 | 0.900275735294118 |
| HL-10 and HL-12 | 0.912946428571429 | HL-5 and HL-32 | 0.900210084033613 |
| HL-10 and HL-20 | 0.912946428571429 | HL-3 and HL-35 | 0.900177258403361 |
| HL-12 and HL-57 | 0.912946428571428 | HL-7 and HL-10 | 0.90014443277311 |
| HL-11 and HL-37 | 0.912880777310924 | HL-2 and HL-41 | 0.900144432773109 |
| HL-3 and HL-49 | 0.912847951680672 | HL-52 and HL-54 | 0.900013130252101 |
| HL-7 and HL-57 | 0.91281512605042 | | |

**Table 8: AUC above 0.95 are obtained when determining low gut diversity by detecting the presence or absence of 50 bacterial genes from the given combination of 3 bacterial species.**

| Combination of bacterial species | AUC | Combination of bacterial species | AUC |
|---|---|---|---|
| HL-8 and HL-26 and HL-53 | 0.966123949579832 | HL-1 and HL-3 and HL-13 | 0.953321953781513 |
| HL-1 and HL-5 and HL-10 | 0.964778098739496 | HL-1 and HL-5 and HL-14 | 0.953256302521009 |
| HL-8 and HL-13 and HL-26 | 0.96297268907563 | HL-4 and HL-5 and HL-17 | 0.953157825630253 |
| HL-3 and HL-8 and HL-26 | 0.962841386554622 | HL-4 and HL-10 and HL-26 | 0.953125 |
| HL-1 and HL-3 and HL-5 | 0.962349002100841 | HL-10 and HL-21 and HL-27 | 0.953125 |
| HL-1 and HL-5 and HL-28 | 0.961790966386555 | HL-3 and HL-10 and HL-11 | 0.953059348739495 |
| HL-1 and HL-5 and HL-17 | 0.960740546218487 | HL-8 and HL-11 and HL-26 | 0.952993697478992 |
| HL-1 and HL-5 and HL-35 | 0.960707720588236 | HL-3 and HL-21 and HL-53 | 0.952960871848739 |
| HL-1 and HL-5 and HL-47 | 0.960707720588236 | HL-1 and HL-3 and HL-33 | 0.952928046218487 |
| HL-1 and HL-5 and HL-8 | 0.96031381302521 | HL-1 and HL-3 and HL-23 | 0.952895220588235 |
| HL-1 and HL-4 and HL-5 | 0.959887079831933 | HL-1 and HL-28 and HL-47 | 0.952862394957984 |
| HL-3 and HL-26 and HL-53 | 0.959722951680672 | HL-4 and HL-5 and HL-11 | 0.952862394957983 |
| HL-1 and HL-3 and HL-10 | 0.959591649159664 | HL-10 and HL-35 and HL-53 | 0.952862394957983 |
| HL-1 and HL-5 and HL-11 | 0.959361869747899 | HL-1 and HL-8 and HL-47 | 0.952796743697479 |
| HL-1 and HL-5 and HL-25 | 0.959132090336135 | HL-10 and HL-13 and HL-26 | 0.952796743697479 |
| HL-1 and HL-5 and HL-33 | 0.959099264705883 | HL-8 and HL-25 and HL-26 | 0.952731092436975 |
| HL-1 and HL-5 and HL-26 | 0.958967962184874 | HL-8 and HL-26 and HL-45 | 0.952731092436975 |
| HL-4 and HL-8 and HL-26 | 0.958967962184874 | HL-1 and HL-3 and HL-25 | 0.952698266806723 |
| HL-8 and HL-21 and HL-53 | 0.95890231092437 | HL-1 and HL-8 and HL-28 | 0.952665441176471 |
| HL-10 and HL-21 and HL-53 | 0.95890231092437 | HL-1 and HL-10 and HL-36 | 0.952665441176471 |
| HL-1 and HL-5 and HL-22 | 0.958869485294118 | HL-10 and HL-11 and HL-53 | 0.95266544117647 |
| HL-1 and HL-8 and HL-10 | 0.958869485294118 | HL-1 and HL-3 and HL-27 | 0.952566964285715 |
| HL-5 and HL-10 and HL-17 | 0.958803834033613 | HL-7 and HL-10 and HL-21 | 0.952534138655463 |
| HL-1 and HL-5 and HL-12 | 0.95873818277311 | HL-3 and HL-5 and HL-10 | 0.952534138655462 |
| HL-1 and HL-5 and HL-13 | 0.958377100840337 | HL-13 and HL-35 and HL-53 | 0.952402836134454 |
| HL-1 and HL-5 and HL-27 | 0.958377100840337 | HL-10 and HL-27 and HL-53 | 0.952370010504202 |
| HL-1 and HL-10 and HL-28 | 0.95811449579832 | HL-3 and HL-13 and HL-53 | 0.95233718487395 |
| HL-1 and HL-10 and HL-47 | 0.958048844537815 | HL-10 and HL-11 and HL-37 | 0.952337184873949 |
| HL-8 and HL-10 and HL-11 | 0.957983193277311 | HL-10 and HL-13 and HL-21 | 0.952304359243697 |
| HL-8 and HL-21 and HL-26 | 0.957753413865546 | HL-7 and HL-8 and HL-21 | 0.952140231092437 |
| HL-8 and HL-26 and HL-47 | 0.95765493697479 | HL-3 and HL-8 and HL-45 | 0.952074579831933 |
| HL-1 and HL-5 and HL-32 | 0.957589285714286 | HL-10 and HL-11 and HL-26 | 0.952074579831933 |
| HL-1 and HL-5 and HL-9 | 0.957523634453781 | HL-1 and HL-8 and HL-26 | 0.951943277310924 |
| HL-8 and HL-13 and HL-53 | 0.957523634453781 | HL-5 and HL-11 and HL-26 | 0.951943277310924 |
| HL-1 and HL-10 and HL-22 | 0.957425157563025 | HL-1 and HL-8 and HL-35 | 0.951844800420168 |
| HL-13 and HL-26 and HL-53 | 0.957359506302521 | HL-10 and HL-11 and HL-13 | 0.951811974789916 |
| HL-1 and HL-5 and HL-53 | 0.957326680672269 | HL-5 and HL-8 and HL-26 | 0.951779149159664 |
| HL-1 and HL-5 and HL-23 | 0.957293855042017 | HL-5 and HL-10 and HL-13 | 0.951779149159664 |
| HL-8 and HL-12 and HL-26 | 0.957293855042017 | HL-1 and HL-3 and HL-4 | 0.951680672268907 |
| HL-3 and HL-8 and HL-11 | 0.957261029411765 | HL-1 and HL-5 and HL-39 | 0.951647846638656 |
| HL-1 and HL-10 and HL-35 | 0.957195378151261 | HL-1 and HL-3 and HL-12 | 0.951615021008404 |
| HL-3 and HL-8 and HL-53 | 0.956998424369748 | HL-1 and HL-3 and HL-37 | 0.951615021008403 |
| HL-3 and HL-10 and HL-53 | 0.956998424369748 | HL-1 and HL-10 and HL-11 | 0.951615021008403 |
| HL-1 and HL-5 and HL-21 | 0.956965598739496 | HL-1 and HL-3 and HL-51 | 0.951582195378151 |
| HL-1 and HL-10 and HL-13 | 0.956932773109244 | HL-1 and HL-3 and HL-53 | 0.951582195378151 |
| HL-1 and HL-10 and HL-27 | 0.956899947478992 | HL-1 and HL-5 and HL-7 | 0.951549369747899 |
| HL-5 and HL-8 and HL-11 | 0.95686712184874 | HL-10 and HL-11 and HL-27 | 0.951549369747899 |
| HL-5 and HL-8 and HL-17 | 0.956801470588235 | HL-1 and HL-10 and HL-46 | 0.951516544117647 |
| HL-8 and HL-26 and HL-3 5 | 0.956801470588235 | HL-1 and HL-13 and HL-47 | 0.951450892857143 |
| HL-1 and HL-5 and HL-51 | 0.956702993697479 | HL-1 and HL-8 and HL-13 | 0.951418067226891 |
| HL-1 and HL-5 and HL-36 | 0.956670168067227 | HL-1 and HL-5 and HL-6 | 0.951352415966387 |
| HL-1 and HL-5 and HL-37 | 0.956538865546219 | HL-1 and HL-8 and HL-22 | 0.951221113445378 |
| HL-1 and HL-4 and HL-10 | 0.956473214285714 | HL-4 and HL-13 and HL-26 | 0.951221113445378 |
| HL-1 and HL-3 and HL-8 | 0.956309086134454 | HL-5 and HL-13 and HL-17 | 0.951221113445378 |
| HL-1 and HL-5 and HL-40 | 0.956276260504202 | HL-10 and HL-11 and HL-22 | 0.951221113445378 |
| HL-10 and HL-21 and HL-26 | 0.956210609243697 | HL-1 and HL-22 and HL-47 | 0.951188287815126 |
| HL-1 and HL-10 and HL-12 | 0.956177783613446 | HL-1 and HL-3 and HL-18 | 0.951155462184874 |
| HL-8 and HL-26 and HL-27 | 0.956079306722689 | HL-8 and HL-26 and HL-32 | 0.951155462184874 |
| HL-1 and HL-10 and HL-26 | 0.955980829831933 | HL-1 and HL-8 and HL-33 | 0.95108981092437 |
| HL-5 and HL-10 and HL-11 | 0.955882352941176 | HL-5 and HL-8 and HL-13 | 0.951024159663865 |
| HL-1 and HL-10 and HL-23 | 0.955816701680672 | HL-5 and HL-13 and HL-26 | 0.950958508403361 |
| HL-3 and HL-5 and HL-17 | 0.955816701680672 | HL-4 and HL-26 and HL-53 | 0.950892857142857 |
| HL-8 and HL-18 and HL-26 | 0.95578387605042 | HL-8 and HL-26 and HL-31 | 0.950892857142857 |
| HL-3 and HL-18 and HL-26 | 0.955751050420168 | HL-8 and HL-27 and HL-53 | 0.950892857142857 |
| HL-1 and HL-5 and HL-45 | 0.955718224789917 | HL-22 and HL-26 and HL-47 | 0.950892857142857 |
| HL-8 and HL-10 and HL-53 | 0.955685399159664 | HL-1 and HL-5 and HL-16 | 0.950860031512605 |
| HL-10 and HL-13 and HL-53 | 0.955652573529412 | HL-1 and HL-13 and HL-28 | 0.950794380252101 |
| HL-1 and HL-5 and HL-31 | 0.955586922268908 | HL-1 and HL-26 and HL-28 | 0.950794380252101 |
| HL-1 and HL-10 and HL-33 | 0.955586922268908 | HL-1 and HL-26 and HL-47 | 0.950794380252101 |
| HL-1 and HL-3 and HL-28 | 0.955455619747899 | HL-26 and HL-27 and HL-53 | 0.950794380252101 |
| HL-4 and HL-10 and HL-21 | 0.955389968487395 | HL-1 and HL-3 and HL-32 | 0.950761554621849 |
| HL-3 and HL-5 and HL-26 | 0.955225840336134 | HL-8 and HL-10 and HL-26 | 0.950761554621849 |
| HL-1 and HL-10 and HL-37 | 0.955127363445378 | HL-8 and HL-10 and HL-21 | 0.950728728991596 |
| HL-1 and HL-3 and HL-22 | 0.955028886554622 | HL-8 and HL-18 and HL-53 | 0.950728728991596 |
| HL-1 and HL-5 and HL-18 | 0.954930409663866 | HL-13 and HL-21 and HL-53 | 0.950728728991596 |
| HL-1 and HL-10 and HL-25 | 0.954930409663866 | HL-3 and HL-10 and HL-18 | 0.950695903361345 |
| HL-1 and HL-10 and HL-51 | 0.954930409663866 | HL-10 and HL-17 and HL-53 | 0.950695903361345 |
| HL-25 and HL-26 and HL-53 | 0.954766281512605 | HL-1 and HL-5 and HL-49 | 0.950695903361344 |
| HL-1 and HL-10 and HL-32 | 0.954634978991597 | HL-1 and HL-8 and HL-27 | 0.950663077731093 |
| HL-1 and HL-10 and HL-21 | 0.954569327731093 | HL-1 and HL-5 and HL-15 | 0.950663077731092 |
| HL-3 and HL-4 and HL-26 | 0.95453650210084 | HL-5 and HL-11 and HL-25 | 0.950630252100841 |
| HL-8 and HL-22 and HL-26 | 0.954306722689076 | HL-1 and HL-10 and HL-45 | 0.950498949579832 |
| HL-1 and HL-10 and HL-40 | 0.954273897058824 | HL-8 and HL-11 and HL-13 | 0.950498949579832 |
| HL-10 and HL-26 and HL-53 | 0.954175420168067 | HL-10 and HL-11 and HL-21 | 0.950498949579832 |
| HL-1 and HL-5 and HL-57 | 0.954109768907563 | HL-1 and HL-8 and HL-25 | 0.95046612394958 |
| HL-1 and HL-5 and HL-46 | 0.953978466386555 | HL-4 and HL-26 and HL-35 | 0.950433298319328 |
| HL-1 and HL-3 and HL-26 | 0.953847163865546 | HL-8 and HL-26 and HL-28 | 0.950433298319328 |
| HL-1 and HL-3 and HL-35 | 0.953814338235294 | HL-8 and HL-26 and HL-51 | 0.950433298319328 |
| HL-3 and HL-10 and HL-21 | 0.953715861344538 | HL-10 and HL-22 and HL-53 | 0.950433298319328 |
| HL-5 and HL-11 and HL-13 | 0.953715861344538 | HL-5 and HL-11 and HL-57 | 0.950367647058824 |
| HL-10 and HL-25 and HL-53 | 0.953715861344538 | HL-8 and HL-11 and HL-53 | 0.950367647058824 |
| HL-3 and HL-18 and HL-53 | 0.953617384453781 | HL-3 and HL-8 and HL-21 | 0.950367647058823 |
| HL-4 and HL-10 and HL-11 | 0.953584558823529 | HL-26 and HL-35 and HL-53 | 0.950367647058823 |
| HL-1 and HL-10 and HL-53 | 0.953551733193278 | HL-8 and HL-35 and HL-53 | 0.950334821428571 |
| HL-1 and HL-10 and HL-31 | 0.953551733193277 | HL-1 and HL-22 and HL-28 | 0.950269170168067 |
| HL-5 and HL-10 and HL-57 | 0.953518907563025 | HL-22 and HL-25 and HL-26 | 0.950236344537815 |
| HL-10 and HL-21 and HL-22 | 0.953518907563025 | HL-1 and HL-3 and HL-40 | 0.950203518907563 |
| HL-1 and HL-3 and HL-47 | 0.953486081932774 | HL-1 and HL-9 and HL-10 | 0.950105042016807 |
| HL-1 and HL-10 and HL-17 | 0.953387605042017 | HL-3 and HL-8 and HL-18 | 0.950105042016807 |
| HL-1 and HL-10 and HL-18 | 0.953387605042017 | HL-5 and HL-8 and HL-21 | 0.950105042016807 |
| HL-3 and HL-22 and HL-26 | 0.953354779411765 | HL-3 and HL-8 and HL-9 | 0.950105042016806 |
| HL-3 and HL-5 and HL-11 | 0.953354779411764 | HL-5 and HL-17 and HL-45 | 0.950039390756303 |

**Table 9: AUC above 0.96 are obtained when determining low gut diversity by detecting the presence or absence of 50 bacterial genes from the given combination of 4 bacterial species.**

| Combination of clusters | AUC |
|---|---|
| HL-3; HL-8; HL-26 and HL-53 | 0.975380777310924 |
| HL-8; HL-13; HL-26 and HL-53 | 0.974658613445378 |
| HL-8; HL-13; HL-26 and HL-35 | 0.972098214285714 |
| HL-8; HL-26; HL-27 and HL-53 | 0.971179096638655 |
| HL-8; HL-25; HL-26 and HL-53 | 0.970522584033613 |
| HL-1; HL-5; HL-10 and HL-17 | 0.970391281512605 |
| HL-8; HL-26; HL-35 and HL-53 | 0.970161502100841 |
| HL-8; HL-13; HL-26 and HL-47 | 0.969800420168067 |
| HL-8; HL-10; HL-26 and HL-53 | 0.969669117647059 |
| HL-8; HL-21; HL-26 and HL-53 | 0.969669117647059 |
| HL-3; HL-8; HL-26 and HL-51 | 0.969603466386555 |
| HL-5; HL-8; HL-10 and HL-17 | 0.96937368697479 |
| HL-8; HL-13; HL-26 and HL-51 | 0.969209558823529 |
| HL-8; HL-26; HL-33 and HL-53 | 0.968881302521009 |
| HL-8; HL-13; HL-18 and HL-26 | 0.968881302521008 |
| HL-8; HL-26; HL-47 and HL-53 | 0.968815651260504 |
| HL-5; HL-10; HL-17 and HL-22 | 0.968553046218488 |
| HL-1; HL-4; HL-5 and HL-10 | 0.968290441176471 |
| HL-1; HL-3; HL-5 and HL-17 | 0.968224789915966 |
| HL-8; HL-12; HL-26 and HL-53 | 0.968224789915966 |
| HL-5; HL-8; HL-17 and HL-45 | 0.968159138655462 |
| HL-8; HL-10; HL-13 and HL-26 | 0.968159138655462 |
| HL-3; HL-8; HL-13 and HL-26 | 0.968093487394958 |
| HL-8; HL-26; HL-51 and HL-53 | 0.968027836134454 |
| HL-1; HL-5; HL-8 and HL-17 | 0.96796218487395 |
| HL-3; HL-8; HL-26 and HL-35 | 0.967929359243697 |
| HL-8; HL-26; HL-37 and HL-53 | 0.967863707983194 |
| HL-8; HL-26; HL-27 and HL-35 | 0.967732405462185 |
| HL-8; HL-10; HL-21 and HL-53 | 0.967699579831933 |
| HL-1; HL-3; HL-5 and HL-10 | 0.967633928571429 |
| HL-3; HL-5; HL-10 and HL-17 | 0.967633928571429 |
| HL-1; HL-5; HL-8 and HL-10 | 0.967568277310925 |
| HL-8; HL-26; HL-31 and HL-53 | 0.96750262605042 |
| HL-1; HL-5; HL-10 and HL-27 | 0.967469800420168 |
| HL-5; HL-10; HL-13 and HL-17 | 0.967436974789916 |
| HL-3; HL-8; HL-10 and HL-26 | 0.967305672268908 |
| HL-1; HL-5; HL-10 and HL-35 | 0.967240021008404 |
| HL-1; HL-5; HL-10 and HL-32 | 0.967240021008403 |
| HL-1; HL-5; HL-10 and HL-47 | 0.967108718487395 |
| HL-5; HL-10; HL-17 and HL-27 | 0.966911764705883 |
| HL-1; HL-5; HL-10 and HL-36 | 0.966911764705882 |
| HL-3; HL-8; HL-26 and HL-47 | 0.966911764705882 |
| HL-8; HL-10; HL-27 and HL-53 | 0.966911764705882 |
| HL-4; HL-8; HL-26 and HL-53 | 0.966878939075631 |
| HL-3; HL-8; HL-18 and HL-26 | 0.966846113445378 |
| HL-1; HL-5; HL-10 and HL-26 | 0.966813287815126 |
| HL-8; HL-10; HL-13 and HL-53 | 0.966813287815126 |
| HL-1; HL-5; HL-10 and HL-12 | 0.966780462184874 |
| HL-1; HL-5; HL-17 and HL-28 | 0.966780462184874 |
| HL-3; HL-4; HL-8 and HL-26 | 0.966780462184874 |
| HL-1; HL-5; HL-10 and HL-13 | 0.96671481092437 |
| HL-8; HL-10; HL-17 and HL-53 | 0.966583508403361 |
| HL-3; HL-8; HL-10 and HL-53 | 0.966550682773109 |
| HL-1; HL-5; HL-10 and HL-28 | 0.966485031512605 |
| HL-5; HL-8; HL-13 and HL-17 | 0.966320903361345 |
| HL-3; HL-5; HL-8 and HL-17 | 0.966320903361344 |
| HL-8; HL-13; HL-26 and HL-28 | 0.966320903361344 |
| HL-8; HL-26; HL-27 and HL-47 | 0.966320903361344 |
| HL-1; HL-5; HL-10 and HL-22 | 0.966255252100841 |
| HL-1; HL-5; HL-17 and HL-47 | 0.96625525210084 |
| HL-8; HL-13; HL-26 and HL-37 | 0.966156775210084 |
| HL-8; HL-22; HL-26 and HL-53 | 0.966156775210084 |
| HL-8; HL-10; HL-11 and HL-21 | 0.966123949579832 |
| HL-1; HL-3; HL-5 and HL-28 | 0.96609112394958 |
| HL-1; HL-5; HL-10 and HL-25 | 0.966058298319328 |
| HL-10; HL-25; HL-26 and HL-53 | 0.966058298319328 |
| HL-5; HL-8; HL-11 and HL-26 | 0.965926995798319 |
| HL-8; HL-13; HL-25 and HL-26 | 0.965926995798319 |
| HL-5; HL-10; HL-17 and HL-45 | 0.965861344537815 |
| HL-10; HL-13; HL-21 and HL-53 | 0.965762867647059 |
| HL-1; HL-3; HL-5 and HL-35 | 0.965730042016807 |
| HL-4; HL-5; HL-10 and HL-17 | 0.965730042016807 |
| HL-4; HL-8; HL-13 and HL-26 | 0.965730042016807 |
| HL-8; HL-10; HL-11 and HL-26 | 0.965730042016807 |
| HL-8; HL-10; HL-11 and HL-27 | 0.965730042016806 |
| HL-1; HL-5; HL-10 and HL-21 | 0.965664390756303 |
| HL-3; HL-8; HL-12 and HL-26 | 0.965664390756303 |
| HL-3; HL-8; HL-22 and HL-26 | 0.965664390756302 |
| HL-1; HL-5; HL-10 and HL-23 | 0.965598739495799 |
| HL-3; HL-8; HL-21 and HL-53 | 0.965598739495798 |
| HL-3; HL-10; HL-21 and HL-53 | 0.965598739495798 |
| HL-4; HL-10; HL-21 and HL-27 | 0.965598739495798 |
| HL-8; HL-18; HL-26 and HL-53 | 0.965598739495798 |
| HL-1; HL-5; HL-10 and HL-33 | 0.965565913865547 |
| HL-1; HL-5; HL-10 and HL-37 | 0.965500262605042 |
| HL-3; HL-8; HL-26 and HL-37 | 0.965500262605042 |
| HL-4; HL-8; HL-26 and HL-35 | 0.965500262605042 |
| HL-3; HL-8; HL-26 and HL-32 | 0.96546743697479 |
| HL-4; HL-10; HL-21 and HL-26 | 0.96546743697479 |
| HL-1; HL-5; HL-10 and HL-51 | 0.965434611344538 |
| HL-1; HL-5; HL-28 and HL-47 | 0.965401785714286 |
| HL-8; HL-13; HL-26 and HL-33 | 0.965401785714286 |
| HL-1; HL-5; HL-10 and HL-40 | 0.965368960084034 |
| HL-3; HL-13; HL-26 and HL-53 | 0.965368960084034 |
| HL-5; HL-10; HL-17 and HL-25 | 0.965368960084034 |
| HL-1; HL-4; HL-5 and HL-28 | 0.965336134453782 |
| HL-1; HL-5; HL-10 and HL-11 | 0.965336134453781 |
| HL-8; HL-26; HL-40 and HL-53 | 0.965336134453781 |
| HL-5; HL-8; HL-11 and HL-17 | 0.965270483193278 |
| HL-8; HL-21; HL-26 and HL-27 | 0.965270483193277 |
| HL-4; HL-5; HL-8 and HL-17 | 0.965139180672269 |
| HL-4; HL-10; HL-21 and HL-53 | 0.965106355042017 |
| HL-5; HL-10; HL-17 and HL-26 | 0.965106355042017 |
| HL-10; HL-13; HL-26 and HL-53 | 0.965073529411765 |
| HL-3; HL-8; HL-26 and HL-45 | 0.965007878151261 |
| HL-1; HL-3; HL-4 and HL-5 | 0.964942226890757 |
| HL-5; HL-10; HL-11 and HL-17 | 0.964942226890757 |
| HL-3; HL-8; HL-26 and HL-40 | 0.964942226890756 |
| HL-5; HL-8; HL-17 and HL-26 | 0.964942226890756 |
| HL-5; HL-10; HL-17 and HL-53 | 0.964942226890756 |
| HL-8; HL-13; HL-35 and HL-53 | 0.964942226890756 |
| HL-1; HL-5; HL-10 and HL-53 | 0.964909401260505 |
| HL-1; HL-5; HL-8 and HL-28 | 0.964876575630253 |
| HL-4; HL-8; HL-21 and HL-26 | 0.964876575630252 |
| HL-8; HL-26; HL-28 and HL-53 | 0.964876575630252 |
| HL-1; HL-3; HL-5 and HL-8 | 0.96484375 |
| HL-3; HL-5; HL-8 and HL-26 | 0.96484375 |
| HL-3; HL-8; HL-11 and HL-26 | 0.964810924369748 |
| HL-8; HL-10; HL-11 and HL-35 | 0.964810924369748 |
| HL-8; HL-12; HL-13 and HL-26 | 0.964810924369748 |
| HL-8; HL-12; HL-26 and HL-35 | 0.964810924369748 |
| HL-8; HL-13; HL-22 and HL-26 | 0.964810924369748 |
| HL-8; HL-23; HL-26 and HL-53 | 0.964810924369748 |
| HL-10; HL-13; HL-18 and HL-26 | 0.964745273109244 |
| HL-1; HL-5; HL-28 and HL-35 | 0.964712447478992 |
| HL-1; HL-5; HL-10 and HL-31 | 0.96467962184874 |
| HL-1; HL-5; HL-10 and HL-45 | 0.96467962184874 |
| HL-4; HL-8; HL-26 and HL-47 | 0.964679621848739 |
| HL-1; HL-3; HL-5 and HL-26 | 0.964646796218488 |
| HL-1; HL-5; HL-8 and HL-35 | 0.964613970588236 |
| HL-3; HL-8; HL-11 and HL-37 | 0.964613970588235 |
| HL-3; HL-5; HL-8 and HL-11 | 0.964548319327731 |
| HL-8; HL-18; HL-26 and HL-35 | 0.964548319327731 |
| HL-1; HL-3; HL-5 and HL-22 | 0.964482668067227 |
| HL-1; HL-3; HL-5 and HL-32 | 0.964482668067227 |
| HL-1; HL-5; HL-17 and HL-26 | 0.964482668067227 |
| HL-5; HL-10; HL-17 and HL-31 | 0.964482668067227 |
| HL-10; HL-21; HL-26 and HL-53 | 0.964482668067227 |
| HL-1; HL-3; HL-5 and HL-47 | 0.964449842436975 |
| HL-5; HL-8; HL-13 and HL-26 | 0.964449842436975 |
| HL-1; HL-5; HL-35 and HL-47 | 0.964417016806723 |
| HL-3; HL-5; HL-13 and HL-17 | 0.964417016806723 |
| HL-3; HL-8; HL-26 and HL-27 | 0.964417016806723 |
| HL-1; HL-3; HL-5 and HL-33 | 0.964384191176471 |
| HL-10; HL-13; HL-21 and HL-26 | 0.96438419117647 |
| HL-1; HL-5; HL-17 and HL-45 | 0.964351365546219 |
| HL-3; HL-10; HL-17 and HL-53 | 0.964351365546219 |
| HL-10; HL-21; HL-22 and HL-26 | 0.964351365546219 |
| HL-3; HL-8; HL-21 and HL-26 | 0.964351365546218 |
| HL-3; HL-10; HL-21 and HL-26 | 0.964351365546218 |
| HL-8; HL-21; HL-26 and HL-31 | 0.964351365546218 |
| HL-1; HL-5; HL-26 and HL-28 | 0.964285714285715 |
| HL-1; HL-5; HL-12 and HL-17 | 0.964285714285714 |
| HL-4; HL-8; HL-26 and HL-27 | 0.964252888655462 |
| HL-8; HL-22; HL-26 and HL-47 | 0.964252888655462 |
| HL-10; HL-21; HL-26 and HL-27 | 0.964252888655462 |
| HL-1; HL-4; HL-5 and HL-17 | 0.96422006302521 |
| HL-1; HL-4; HL-5 and HL-35 | 0.96422006302521 |
| HL-1; HL-5; HL-17 and HL-33 | 0.96422006302521 |
| HL-3; HL-10; HL-26 and HL-53 | 0.96422006302521 |
| HL-8; HL-10; HL-11 and HL-37 | 0.96422006302521 |
| HL-13; HL-26; HL-47 and HL-53 | 0.96422006302521 |
| HL-1; HL-3; HL-5 and HL-27 | 0.964187237394958 |
| HL-1; HL-5; HL-12 and HL-28 | 0.964154411764706 |
| HL-8; HL-10; HL-11 and HL-13 | 0.964154411764706 |
| HL-10; HL-15; HL-21 and HL-53 | 0.964154411764706 |
| HL-10; HL-21; HL-22 and HL-53 | 0.964154411764706 |
| HL-10; HL-21; HL-27 and HL-53 | 0.964154411764706 |
| HL-1; HL-3; HL-5 and HL-25 | 0.964121586134454 |
| HL-3; HL-18; HL-26 and HL-32 | 0.964121586134454 |
| HL-1; HL-3; HL-5 and HL-36 | 0.964088760504202 |
| HL-1; HL-5; HL-13 and HL-28 | 0.964088760504202 |
| HL-3; HL-8; HL-26 and HL-28 | 0.964088760504202 |
| HL-3; HL-8; HL-10 and HL-11 | 0.964088760504201 |
| HL-1; HL-3; HL-5 and HL-13 | 0.96405593487395 |
| HL-1; HL-5; HL-13 and HL-17 | 0.964023109243697 |
| HL-8; HL-10; HL-26 and HL-47 | 0.964023109243697 |
| HL-8; HL-13; HL-21 and HL-26 | 0.964023109243697 |
| HL-8; HL-13; HL-21 and HL-53 | 0.964023109243697 |
| HL-1; HL-5; HL-27 and HL-28 | 0.963990283613446 |
| HL-5; HL-10; HL-17 and HL-37 | 0.963990283613446 |
| HL-5; HL-8; HL-10 and HL-13 | 0.963990283613445 |
| HL-1; HL-4; HL-5 and HL-47 | 0.963957457983193 |
| HL-1; HL-5; HL-17 and HL-27 | 0.963957457983193 |
| HL-1; HL-5; HL-17 and HL-35 | 0.963957457983193 |
| HL-1; HL-5; HL-22 and HL-28 | 0.963924632352942 |
| HL-8; HL-10; HL-21 and HL-26 | 0.963924632352941 |
| HL-1; HL-5; HL-17 and HL-53 | 0.963891806722689 |
| HL-1; HL-5; HL-28 and HL-32 | 0.963891806722689 |
| HL-3; HL-8; HL-26 and HL-33 | 0.963891806722689 |
| HL-8; HL-10; HL-17 and HL-45 | 0.963891806722689 |
| HL-3; HL-8; HL-11 and HL-35 | 0.963826155462185 |
| HL-3; HL-18; HL-26 and HL-53 | 0.963826155462185 |
| HL-7; HL-10; HL-21 and HL-53 | 0.963826155462185 |
| HL-1; HL-3; HL-5 and HL-11 | 0.963760504201681 |
| HL-1; HL-4; HL-5 and HL-8 | 0.963760504201681 |
| HL-1; HL-5; HL-8 and HL-9 | 0.963760504201681 |
| HL-1; HL-5; HL-10 and HL-18 | 0.963760504201681 |
| HL-10; HL-21; HL-37 and HL-53 | 0.963760504201681 |
| HL-4; HL-8; HL-10 and HL-26 | 0.96376050420168 |
| HL-1; HL-3; HL-5 and HL-12 | 0.963727678571429 |
| HL-1; HL-5; HL-8 and HL-47 | 0.963727678571429 |
| HL-5; HL-10; HL-11 and HL-26 | 0.963694852941177 |
| HL-8; HL-13; HL-18 and HL-53 | 0.963694852941176 |
| HL-8; HL-13; HL-26 and HL-45 | 0.963694852941176 |
| HL-1; HL-5; HL-8 and HL-25 | 0.963662027310925 |
| HL-3; HL-7; HL-10 and HL-21 | 0.963662027310925 |
| HL-1; HL-3; HL-5 and HL-53 | 0.963596376050421 |
| HL-1; HL-5; HL-8 and HL-33 | 0.963596376050421 |
| HL-1; HL-5; HL-8 and HL-53 | 0.963596376050421 |
| HL-1; HL-5; HL-25 and HL-47 | 0.963596376050421 |
| HL-5; HL-10; HL-17 and HL-35 | 0.96359637605042 |
| HL-1; HL-5; HL-11 and HL-17 | 0.963563550420169 |
| HL-1; HL-5; HL-17 and HL-23 | 0.963563550420168 |
| HL-1; HL-5; HL-28 and HL-36 | 0.963563550420168 |
| HL-7; HL-8; HL-21 and HL-26 | 0.963563550420168 |
| HL-8; HL-10; HL-11 and HL-53 | 0.963563550420168 |
| HL-8; HL-13; HL-26 and HL-40 | 0.963563550420168 |
| HL-10; HL-13; HL-35 and HL-53 | 0.963563550420168 |
| HL-1; HL-5; HL-26 and HL-47 | 0.963530724789917 |
| HL-8; HL-12; HL-26 and HL-47 | 0.963530724789916 |
| HL-3; HL-5; HL-17 and HL-26 | 0.963497899159664 |
| HL-5; HL-8; HL-11 and HL-21 | 0.963497899159664 |
| HL-8; HL-26; HL-31 and HL-47 | 0.963497899159664 |
| HL-1; HL-5; HL-8 and HL-26 | 0.963465073529412 |
| HL-1; HL-5; HL-17 and HL-22 | 0.96343224789916 |
| HL-3; HL-8; HL-11 and HL-21 | 0.96343224789916 |
| HL-3; HL-22; HL-26 and HL-47 | 0.96343224789916 |
| HL-3; HL-25; HL-26 and HL-53 | 0.96343224789916 |
| HL-5; HL-8; HL-17 and HL-36 | 0.96343224789916 |
| HL-8; HL-11; HL-26 and HL-53 | 0.96343224789916 |
| HL-3; HL-8; HL-25 and HL-26 | 0.963432247899159 |
| HL-8; HL-13; HL-26 and HL-31 | 0.963432247899159 |
| HL-8; HL-21; HL-26 and HL-35 | 0.963432247899159 |
| HL-1; HL-5; HL-26 and HL-35 | 0.963399422268908 |
| HL-1; HL-5; HL-33 and HL-47 | 0.963399422268908 |
| HL-5; HL-8; HL-11 and HL-13 | 0.963366596638656 |
| HL-7; HL-8; HL-21 and HL-53 | 0.963366596638656 |
| HL-1; HL-5; HL-11 and HL-26 | 0.963366596638655 |
| HL-3; HL-5; HL-17 and HL-45 | 0.963366596638655 |
| HL-3; HL-8; HL-11 and HL-53 | 0.963366596638655 |
| HL-8; HL-21; HL-26 and HL-51 | 0.963366596638655 |
| HL-13; HL-26; HL-35 and HL-53 | 0.963366596638655 |
| HL-4; HL-8; HL-25 and HL-26 | 0.963333771008403 |
| HL-1; HL-5; HL-8 and HL-27 | 0.963300945378152 |
| HL-1; HL-5; HL-17 and HL-25 | 0.963300945378151 |
| HL-3; HL-4; HL-26 and HL-53 | 0.963300945378151 |
| HL-8; HL-13; HL-18 and HL-35 | 0.963300945378151 |
| HL-1; HL-5; HL-12 and HL-47 | 0.9632681197479 |
| HL-1; HL-5; HL-17 and HL-40 | 0.963235294117647 |
| HL-1; HL-5; HL-32 and HL-47 | 0.963235294117647 |
| HL-3; HL-5; HL-11 and HL-26 | 0.963235294117647 |
| HL-8; HL-22; HL-26 and HL-35 | 0.963235294117647 |
| HL-8; HL-10; HL-17 and HL-27 | 0.963202468487395 |
| HL-1; HL-4; HL-5 and HL-33 | 0.963169642857143 |
| HL-4; HL-8; HL-26 and HL-33 | 0.963169642857143 |
| HL-8; HL-10; HL-11 and HL-47 | 0.963169642857143 |
| HL-10; HL-21; HL-25 and HL-53 | 0.963169642857142 |
| HL-1; HL-5; HL-8 and HL-13 | 0.963136817226891 |
| HL-5; HL-10; HL-17 and HL-23 | 0.963136817226891 |
| HL-1; HL-5; HL-11 and HL-47 | 0.963103991596639 |
| HL-8; HL-10; HL-11 and HL-40 | 0.963103991596639 |
| HL-8; HL-10; HL-35 and HL-53 | 0.963103991596639 |
| HL-1; HL-5; HL-21 and HL-28 | 0.963071165966387 |
| HL-3; HL-26; HL-47 and HL-53 | 0.963071165966387 |
| HL-1; HL-5; HL-11 and HL-21 | 0.963038340336135 |
| HL-1; HL-5; HL-22 and HL-47 | 0.963038340336135 |
| HL-1; HL-5; HL-28 and HL-33 | 0.963038340336135 |
| HL-3; HL-5; HL-11 and HL-37 | 0.963038340336135 |
| HL-1; HL-4; HL-5 and HL-25 | 0.963038340336134 |
| HL-3; HL-5; HL-22 and HL-26 | 0.963038340336134 |
| HL-8; HL-13; HL-26 and HL-32 | 0.963038340336134 |
| HL-8; HL-21; HL-27 and HL-53 | 0.963038340336134 |
| HL-8; HL-26; HL-27 and HL-51 | 0.963038340336134 |
| HL-1; HL-5; HL-13 and HL-35 | 0.963005514705883 |
| HL-1; HL-5; HL-13 and HL-47 | 0.963005514705883 |
| HL-1; HL-5; HL-27 and HL-35 | 0.963005514705883 |
| HL-1; HL-4; HL-5 and HL-26 | 0.962972689075631 |
| HL-1; HL-5; HL-8 and HL-12 | 0.962972689075631 |
| HL-1; HL-3; HL-8 and HL-10 | 0.96297268907563 |
| HL-1; HL-5; HL-9 and HL-17 | 0.96297268907563 |
| HL-3; HL-22; HL-26 and HL-53 | 0.96297268907563 |
| HL-4; HL-10; HL-11 and HL-21 | 0.96297268907563 |
| HL-5; HL-10; HL-11 and HL-13 | 0.96297268907563 |
| HL-8; HL-15; HL-21 and HL-53 | 0.96297268907563 |
| HL-10; HL-21; HL-22 and HL-27 | 0.96297268907563 |
| HL-1; HL-3; HL-10 and HL-22 | 0.962939863445378 |
| HL-3; HL-26; HL-37 and HL-53 | 0.962939863445378 |
| HL-5; HL-8; HL-10 and HL-11 | 0.962907037815126 |
| HL-5; HL-10; HL-13 and HL-26 | 0.962874212184874 |
| HL-8; HL-18; HL-21 and HL-53 | 0.962874212184873 |
| HL-1; HL-3; HL-5 and HL-9 | 0.962841386554622 |
| HL-1; HL-3; HL-5 and HL-23 | 0.962841386554622 |
| HL-5; HL-8; HL-11 and HL-35 | 0.962841386554622 |
| HL-5; HL-10; HL-17 and HL-28 | 0.962841386554622 |
| HL-5; HL-10; HL-17 and HL-47 | 0.962841386554622 |
| HL-8; HL-18; HL-26 and HL-27 | 0.962841386554622 |
| HL-1; HL-5; HL-33 and HL-35 | 0.96280856092437 |
| HL-3; HL-4; HL-5 and HL-17 | 0.96280856092437 |
| HL-1; HL-5; HL-11 and HL-35 | 0.962775735294118 |
| HL-1; HL-5; HL-28 and HL-37 | 0.962775735294118 |
| HL-5; HL-8; HL-11 and HL-45 | 0.962775735294118 |
| HL-5; HL-8; HL-17 and HL-31 | 0.962775735294118 |
| HL-5; HL-10; HL-17 and HL-36 | 0.962775735294118 |
| HL-8; HL-12; HL-21 and HL-53 | 0.962775735294118 |
| HL-1; HL-5; HL-11 and HL-28 | 0.962775735294117 |
| HL-1; HL-3; HL-5 and HL-40 | 0.962742909663866 |
| HL-1; HL-5; HL-25 and HL-28 | 0.962742909663866 |
| HL-8; HL-18; HL-26 and HL-47 | 0.962742909663865 |
| HL-1; HL-5; HL-8 and HL-22 | 0.962710084033614 |
| HL-1; HL-5; HL-12 and HL-35 | 0.962710084033614 |
| HL-1; HL-5; HL-17 and HL-37 | 0.962710084033614 |
| HL-1; HL-5; HL-25 and HL-35 | 0.962710084033614 |
| HL-5; HL-8; HL-17 and HL-27 | 0.962710084033614 |
| HL-1; HL-5; HL-7 and HL-17 | 0.962710084033613 |
| HL-3; HL-8; HL-18 and HL-53 | 0.962710084033613 |
| HL-3; HL-8; HL-26 and HL-31 | 0.962710084033613 |
| HL-8; HL-10; HL-26 and HL-27 | 0.962710084033613 |
| HL-8; HL-13; HL-26 and HL-49 | 0.962710084033613 |
| HL-8; HL-21; HL-37 and HL-53 | 0.962710084033613 |
| HL-1; HL-3; HL-5 and HL-37 | 0.962677258403362 |
| HL-1; HL-5; HL-23 and HL-28 | 0.962677258403362 |
| HL-1; HL-5; HL-27 and HL-47 | 0.962677258403362 |
| HL-1; HL-5; HL-28 and HL-51 | 0.962677258403362 |
| HL-8; HL-10; HL-18 and HL-26 | 0.962677258403361 |
| HL-1; HL-3; HL-5 and HL-51 | 0.96264443277311 |
| HL-1; HL-5; HL-8 and HL-11 | 0.962644432773109 |
| HL-1; HL-5; HL-9 and HL-10 | 0.962644432773109 |
| HL-5; HL-8; HL-11 and HL-27 | 0.962644432773109 |
| HL-5; HL-8; HL-17 and HL-22 | 0.962644432773109 |
| HL-8; HL-10; HL-33 and HL-53 | 0.962644432773109 |
| HL-8; HL-13; HL-26 and HL-27 | 0.962644432773109 |
| HL-8; HL-21; HL-26 and HL-37 | 0.962644432773109 |
| HL-1; HL-8; HL-10 and HL-47 | 0.962611607142857 |
| HL-1; HL-5; HL-11 and HL-40 | 0.962578781512606 |
| HL-1; HL-5; HL-17 and HL-21 | 0.962578781512605 |
| HL-3; HL-8; HL-11 and HL-40 | 0.962578781512605 |
| HL-4; HL-10; HL-11 and HL-26 | 0.962578781512605 |
| HL-5; HL-10; HL-12 and HL-17 | 0.962578781512605 |
| HL-8; HL-21; HL-35 and HL-53 | 0.962578781512605 |
| HL-1; HL-5; HL-25 and HL-26 | 0.962545955882353 |
| HL-1; HL-4; HL-5 and HL-27 | 0.962513130252101 |
| HL-1; HL-5; HL-32 and HL-35 | 0.962513130252101 |
| HL-3; HL-21; HL-26 and HL-53 | 0.962513130252101 |
| HL-10; HL-21; HL-35 and HL-53 | 0.962480304621849 |
| HL-3; HL-8; HL-13 and HL-53 | 0.962480304621848 |
| HL-1; HL-4; HL-5 and HL-13 | 0.962447478991597 |
| HL-1; HL-5; HL-8 and HL-36 | 0.962447478991597 |
| HL-1; HL-5; HL-17 and HL-36 | 0.962447478991597 |
| HL-1; HL-5; HL-22 and HL-35 | 0.962447478991597 |
| HL-3; HL-7; HL-21 and HL-53 | 0.962447478991597 |
| HL-3; HL-13; HL-18 and HL-26 | 0.962447478991597 |
| HL-1; HL-3; HL-5 and HL-21 | 0.962414653361345 |
| HL-3; HL-18; HL-22 and HL-26 | 0.962414653361344 |
| HL-5; HL-10; HL-17 and HL-40 | 0.962381827731093 |
| HL-1; HL-4; HL-5 and HL-12 | 0.962381827731092 |
| HL-1; HL-4; HL-5 and HL-22 | 0.962381827731092 |
| HL-1; HL-5; HL-11 and HL-27 | 0.962381827731092 |
| HL-3; HL-10; HL-11 and HL-37 | 0.962381827731092 |
| HL-8; HL-10; HL-11 and HL-33 | 0.962381827731092 |
| HL-1; HL-5; HL-23 and HL-35 | 0.962316176470589 |
| HL-1; HL-5; HL-28 and HL-40 | 0.962316176470589 |
| HL-4; HL-5; HL-11 and HL-26 | 0.962316176470589 |
| HL-4; HL-7; HL-10 and HL-21 | 0.962316176470589 |
| HL-10; HL-13; HL-15 and HL-21 | 0.962316176470589 |
| HL-1; HL-4; HL-5 and HL-53 | 0.962316176470588 |
| HL-1; HL-5; HL-17 and HL-31 | 0.962316176470588 |
| HL-5; HL-8; HL-11 and HL-37 | 0.962316176470588 |
| HL-8; HL-10; HL-12 and HL-26 | 0.962316176470588 |
| HL-4; HL-8; HL-21 and HL-53 | 0.962283350840335 |
| HL-1; HL-5; HL-6 and HL-10 | 0.962250525210084 |
| HL-1; HL-5; HL-35 and HL-36 | 0.962250525210084 |
| HL-1; HL-5; HL-36 and HL-47 | 0.962250525210084 |
| HL-3; HL-5; HL-17 and HL-36 | 0.962250525210084 |
| HL-5; HL-11; HL-13 and HL-26 | 0.962250525210084 |
| HL-8; HL-10; HL-16 and HL-17 | 0.962250525210084 |
| HL-8; HL-26; HL-32 and HL-53 | 0.962250525210084 |
| HL-8; HL-26; HL-40 and HL-47 | 0.962250525210084 |
| HL-8; HL-26; HL-47 and HL-51 | 0.962250525210084 |
| HL-10; HL-18; HL-21 and HL-53 | 0.962217699579831 |
| HL-1; HL-5; HL-17 and HL-18 | 0.96218487394958 |
| HL-1; HL-5; HL-17 and HL-51 | 0.96218487394958 |
| HL-1; HL-5; HL-26 and HL-33 | 0.96218487394958 |
| HL-3; HL-8; HL-11 and HL-27 | 0.96218487394958 |
| HL-4; HL-21; HL-26 and HL-27 | 0.96218487394958 |
| HL-5; HL-8; HL-17 and HL-53 | 0.96218487394958 |
| HL-8; HL-10; HL-11 and HL-45 | 0.96218487394958 |
| HL-8; HL-10; HL-21 and HL-27 | 0.96218487394958 |
| HL-1; HL-3; HL-10 and HL-13 | 0.962119222689076 |
| HL-1; HL-5; HL-11 and HL-12 | 0.962119222689076 |
| HL-1; HL-10; HL-22 and HL-28 | 0.962119222689076 |
| HL-8; HL-10; HL-11 and HL-22 | 0.962119222689075 |
| HL-8; HL-21; HL-26 and HL-40 | 0.962119222689075 |
| HL-1; HL-3; HL-10 and HL-26 | 0.962086397058824 |
| HL-1; HL-5; HL-8 and HL-40 | 0.962086397058824 |
| HL-1; HL-5; HL-22 and HL-26 | 0.962086397058824 |
| HL-1; HL-3; HL-10 and HL-28 | 0.962053571428572 |
| HL-1; HL-5; HL-13 and HL-33 | 0.962053571428572 |
| HL-3; HL-5; HL-11 and HL-17 | 0.962053571428572 |
| HL-3; HL-8; HL-35 and HL-53 | 0.962053571428571 |
| HL-4; HL-8; HL-10 and HL-11 | 0.962053571428571 |
| HL-8; HL-22; HL-25 and HL-26 | 0.962053571428571 |
| HL-10; HL-12; HL-21 and HL-53 | 0.962053571428571 |
| HL-1; HL-5; HL-12 and HL-25 | 0.96202074579832 |
| HL-3; HL-4; HL-10 and HL-21 | 0.962020745798319 |
| HL-8; HL-12; HL-21 and HL-26 | 0.962020745798319 |
| HL-10; HL-22; HL-25 and HL-53 | 0.962020745798319 |
| HL-1; HL-5; HL-10 and HL-49 | 0.961987920168068 |
| HL-1; HL-5; HL-8 and HL-32 | 0.961987920168067 |
| HL-1; HL-5; HL-11 and HL-13 | 0.961987920168067 |
| HL-1; HL-8; HL-10 and HL-27 | 0.961987920168067 |
| HL-3; HL-8; HL-17 and HL-45 | 0.961987920168067 |
| HL-5; HL-10; HL-22 and HL-25 | 0.961987920168067 |
| HL-1; HL-8; HL-10 and HL-22 | 0.961955094537815 |
| HL-1; HL-8; HL-10 and HL-26 | 0.961955094537815 |
| HL-1; HL-3; HL-10 and HL-35 | 0.961922268907563 |
| HL-3; HL-5; HL-8 and HL-10 | 0.961922268907563 |
| HL-5; HL-10; HL-17 and HL-51 | 0.961922268907563 |
| HL-8; HL-10; HL-26 and HL-45 | 0.961922268907563 |
| HL-10; HL-13; HL-26 and HL-47 | 0.961922268907563 |
| HL-10; HL-21; HL-26 and HL-32 | 0.961889443277311 |
| HL-1; HL-8; HL-10 and HL-35 | 0.961856617647059 |
| HL-3; HL-4; HL-21 and HL-26 | 0.961856617647059 |
| HL-3; HL-5; HL-11 and HL-13 | 0.961856617647059 |
| HL-3; HL-8; HL-11 and HL-47 | 0.961856617647059 |
| HL-3; HL-26; HL-35 and HL-53 | 0.961856617647059 |
| HL-8; HL-18; HL-25 and HL-26 | 0.961856617647059 |
| HL-10; HL-11; HL-26 and HL-53 | 0.961856617647059 |
| HL-10; HL-13; HL-25 and HL-26 | 0.961856617647059 |
| HL-5; HL-10; HL-11 and HL-37 | 0.961856617647058 |
| HL-1; HL-5; HL-12 and HL-33 | 0.961823792016807 |
| HL-1; HL-5; HL-13 and HL-25 | 0.961823792016807 |
| HL-1; HL-5; HL-47 and HL-53 | 0.961823792016807 |
| HL-8; HL-26; HL-35 and HL-40 | 0.961823792016807 |
| HL-8; HL-21; HL-26 and HL-33 | 0.961823792016806 |
| HL-1; HL-3; HL-5 and HL-31 | 0.961790966386555 |
| HL-1; HL-4; HL-8 and HL-10 | 0.961790966386555 |
| HL-1; HL-5; HL-9 and HL-47 | 0.961790966386555 |
| HL-1; HL-5; HL-11 and HL-25 | 0.961790966386555 |
| HL-3; HL-23; HL-26 and HL-53 | 0.961790966386555 |
| HL-13; HL-22; HL-26 and HL-47 | 0.961790966386554 |
| HL-1; HL-5; HL-12 and HL-26 | 0.961758140756303 |
| HL-1; HL-5; HL-28 and HL-31 | 0.961758140756303 |
| HL-8; HL-26; HL-32 and HL-47 | 0.961758140756303 |
| HL-10; HL-21; HL-26 and HL-37 | 0.961758140756303 |
| HL-4; HL-10; HL-15 and HL-21 | 0.961758140756302 |
| HL-1; HL-3; HL-10 and HL-27 | 0.961725315126051 |
| HL-1; HL-3; HL-10 and HL-47 | 0.961725315126051 |
| HL-1; HL-5; HL-27 and HL-33 | 0.961725315126051 |
| HL-5; HL-8; HL-17 and HL-40 | 0.961725315126051 |
| HL-7; HL-8; HL-13 and HL-21 | 0.961725315126051 |
| HL-7; HL-13; HL-21 and HL-53 | 0.961725315126051 |
| HL-8; HL-11; HL-26 and HL-47 | 0.961725315126051 |
| HL-8; HL-13; HL-23 and HL-26 | 0.961725315126051 |
| HL-4; HL-10; HL-11 and HL-27 | 0.96172531512605 |
| HL-5; HL-8; HL-13 and HL-35 | 0.96172531512605 |
| HL-8; HL-10; HL-26 and HL-35 | 0.96172531512605 |
| HL-8; HL-21; HL-25 and HL-26 | 0.96172531512605 |
| HL-8; HL-26; HL-31 and HL-35 | 0.96172531512605 |
| HL-3; HL-5; HL-10 and HL-26 | 0.961692489495798 |
| HL-1; HL-5; HL-13 and HL-26 | 0.961659663865547 |
| HL-5; HL-11; HL-13 and HL-35 | 0.961659663865547 |
| HL-1; HL-5; HL-26 and HL-32 | 0.961659663865546 |
| HL-1; HL-8; HL-10 and HL-13 | 0.961659663865546 |
| HL-3; HL-8; HL-9 and HL-26 | 0.961659663865546 |
| HL-3; HL-8; HL-21 and HL-36 | 0.961659663865546 |
| HL-5; HL-10; HL-11 and HL-27 | 0.961659663865546 |
| HL-5; HL-11; HL-13 and HL-39 | 0.961659663865546 |
| HL-8; HL-10; HL-11 and HL-25 | 0.961659663865546 |
| HL-8; HL-21; HL-33 and HL-53 | 0.961659663865546 |
| HL-5; HL-11; HL-13 and HL-17 | 0.961594012605043 |
| HL-1; HL-3; HL-10 and HL-23 | 0.961594012605042 |
| HL-1; HL-5; HL-26 and HL-27 | 0.961594012605042 |
| HL-3; HL-5; HL-10 and HL-11 | 0.961594012605042 |
| HL-5; HL-10; HL-11 and HL-35 | 0.961594012605042 |
| HL-8; HL-11; HL-26 and HL-27 | 0.961594012605042 |
| HL-8; HL-11; HL-26 and HL-37 | 0.961594012605042 |
| HL-8; HL-27; HL-35 and HL-53 | 0.961594012605042 |
| HL-10; HL-21; HL-33 and HL-53 | 0.961594012605042 |
| HL-10; HL-27; HL-35 and HL-53 | 0.961594012605042 |
| HL-13; HL-25; HL-26 and HL-53 | 0.961594012605042 |
| HL-1; HL-5; HL-35 and HL-53 | 0.96156118697479 |
| HL-3; HL-7; HL-8 and HL-21 | 0.96156118697479 |
| HL-5; HL-8; HL-26 and HL-53 | 0.96156118697479 |
| HL-5; HL-10; HL-17 and HL-21 | 0.96156118697479 |
| HL-5; HL-10; HL-17 and HL-33 | 0.96156118697479 |
| HL-8; HL-10; HL-27 and HL-35 | 0.96156118697479 |
| HL-1; HL-5; HL-8 and HL-23 | 0.961528361344538 |
| HL-1; HL-5; HL-28 and HL-45 | 0.961528361344538 |
| HL-1; HL-5; HL-32 and HL-33 | 0.961528361344538 |
| HL-1; HL-10; HL-22 and HL-47 | 0.961528361344538 |
| HL-3; HL-5; HL-13 and HL-26 | 0.961528361344538 |
| HL-3; HL-5; HL-17 and HL-22 | 0.961528361344538 |
| HL-3; HL-8; HL-23 and HL-26 | 0.961528361344538 |
| HL-8; HL-13; HL-27 and HL-53 | 0.961528361344538 |
| HL-10; HL-11; HL-37 and HL-53 | 0.961528361344538 |
| HL-1; HL-5; HL-11 and HL-53 | 0.961462710084034 |
| HL-1; HL-5; HL-25 and HL-32 | 0.961462710084034 |
| HL-3; HL-5; HL-11 and HL-35 | 0.961462710084034 |
| HL-3; HL-10; HL-18 and HL-26 | 0.961462710084034 |
| HL-8; HL-21; HL-22 and HL-26 | 0.961462710084034 |
| HL-8; HL-26; HL-35 and HL-51 | 0.961462710084034 |
| HL-10; HL-11; HL-21 and HL-53 | 0.961462710084034 |
| HL-8; HL-26; HL-37 and HL-47 | 0.961462710084033 |
| HL-1; HL-3; HL-5 and HL-45 | 0.96139705882353 |
| HL-1; HL-5; HL-12 and HL-22 | 0.96139705882353 |
| HL-1; HL-5; HL-35 and HL-51 | 0.96139705882353 |
| HL-5; HL-8; HL-11 and HL-12 | 0.96139705882353 |
| HL-5; HL-10; HL-17 and HL-32 | 0.96139705882353 |
| HL-1; HL-3; HL-10 and HL-32 | 0.961397058823529 |
| HL-3; HL-10; HL-11 and HL-26 | 0.961397058823529 |
| HL-5; HL-8; HL-17 and HL-21 | 0.961397058823529 |
| HL-8; HL-10; HL-25 and HL-53 | 0.961397058823529 |
| HL-10; HL-11; HL-26 and HL-27 | 0.961397058823529 |
| HL-1; HL-5; HL-25 and HL-33 | 0.961364233193278 |
| HL-10; HL-15; HL-21 and HL-22 | 0.961364233193278 |
| HL-3; HL-10; HL-35 and HL-53 | 0.961364233193277 |
| HL-1; HL-5; HL-8 and HL-21 | 0.961331407563026 |
| HL-1; HL-5; HL-23 and HL-47 | 0.961331407563026 |
| HL-1; HL-5; HL-12 and HL-36 | 0.961331407563025 |
| HL-1; HL-5; HL-25 and HL-36 | 0.961331407563025 |
| HL-3; HL-4; HL-21 and HL-53 | 0.961331407563025 |
| HL-3; HL-5; HL-17 and HL-53 | 0.961331407563025 |
| HL-3; HL-8; HL-11 and HL-33 | 0.961331407563025 |
| HL-3; HL-10; HL-11 and HL-53 | 0.961331407563025 |
| HL-4; HL-8; HL-11 and HL-26 | 0.961331407563025 |
| HL-4; HL-8; HL-26 and HL-28 | 0.961331407563025 |
| HL-4; HL-10; HL-25 and HL-26 | 0.961331407563025 |
| HL-5; HL-8; HL-17 and HL-35 | 0.961331407563025 |
| HL-5; HL-10; HL-11 and HL-25 | 0.961331407563025 |
| HL-7; HL-10; HL-21 and HL-22 | 0.961331407563025 |
| HL-8; HL-18; HL-21 and HL-26 | 0.961331407563025 |
| HL-10; HL-21; HL-26 and HL-51 | 0.961331407563025 |
| HL-13; HL-15; HL-21 and HL-53 | 0.961331407563025 |
| HL-1; HL-5; HL-8 and HL-37 | 0.961298581932774 |
| HL-1; HL-5; HL-22 and HL-27 | 0.961298581932774 |
| HL-1; HL-5; HL-25 and HL-27 | 0.961298581932774 |
| HL-1; HL-5; HL-35 and HL-37 | 0.961298581932774 |
| HL-1; HL-5; HL-35 and HL-40 | 0.961298581932774 |
| HL-1; HL-5; HL-10 and HL-15 | 0.961298581932773 |
| HL-1; HL-10; HL-28 and HL-47 | 0.961298581932773 |
| HL-3; HL-8; HL-45 and HL-53 | 0.961298581932773 |
| HL-10; HL-21; HL-51 and HL-53 | 0.961298581932773 |
| HL-1; HL-8; HL-10 and HL-17 | 0.961265756302521 |
| HL-3; HL-4; HL-5 and HL-11 | 0.961265756302521 |
| HL-5; HL-8; HL-17 and HL-28 | 0.961265756302521 |
| HL-5; HL-11; HL-25 and HL-26 | 0.961265756302521 |
| HL-8; HL-10; HL-11 and HL-36 | 0.961265756302521 |
| HL-10; HL-11; HL-13 and HL-26 | 0.961265756302521 |
| HL-10; HL-21; HL-26 and HL-31 | 0.961265756302521 |
| HL-1; HL-5; HL-37 and HL-47 | 0.961232930672269 |
| HL-1; HL-10; HL-13 and HL-47 | 0.961232930672269 |
| HL-1; HL-3; HL-4 and HL-10 | 0.961200105042017 |
| HL-1; HL-4; HL-5 and HL-21 | 0.961200105042017 |
| HL-1; HL-4; HL-5 and HL-40 | 0.961200105042017 |
| HL-1; HL-5; HL-22 and HL-25 | 0.961200105042017 |
| HL-1; HL-5; HL-28 and HL-53 | 0.961200105042017 |
| HL-1; HL-5; HL-47 and HL-51 | 0.961200105042017 |
| HL-3; HL-7; HL-21 and HL-26 | 0.961200105042017 |
| HL-3; HL-10; HL-25 and HL-53 | 0.961200105042017 |
| HL-4; HL-8; HL-26 and HL-37 | 0.961200105042017 |
| HL-5; HL-10; HL-11 and HL-21 | 0.961200105042017 |
| HL-10; HL-11; HL-21 and HL-37 | 0.961200105042017 |
| HL-3; HL-4; HL-8 and HL-11 | 0.961200105042016 |
| HL-1; HL-5; HL-10 and HL-46 | 0.961167279411765 |
| HL-1; HL-5; HL-12 and HL-27 | 0.961167279411765 |
| HL-1; HL-4; HL-5 and HL-23 | 0.961134453781513 |
| HL-1; HL-5; HL-11 and HL-36 | 0.961134453781513 |
| HL-3; HL-8; HL-11 and HL-15 | 0.961134453781513 |
| HL-8; HL-11; HL-12 and HL-26 | 0.961134453781513 |
| HL-8; HL-12; HL-26 and HL-40 | 0.961134453781513 |
| HL-1; HL-5; HL-33 and HL-36 | 0.961134453781512 |
| HL-3; HL-13; HL-21 and HL-53 | 0.961134453781512 |
| HL-5; HL-8; HL-35 and HL-45 | 0.961134453781512 |
| HL-8; HL-21; HL-31 and HL-53 | 0.961134453781512 |
| HL-1; HL-3; HL-10 and HL-51 | 0.961101628151261 |
| HL-1; HL-5; HL-22 and HL-33 | 0.961101628151261 |
| HL-3; HL-26; HL-33 and HL-53 | 0.96110162815126 |
| HL-8; HL-26; HL-28 and HL-47 | 0.961068802521008 |
| HL-1; HL-5; HL-8 and HL-51 | 0.961003151260505 |
| HL-1; HL-5; HL-11 and HL-18 | 0.961003151260504 |
| HL-1; HL-5; HL-11 and HL-37 | 0.961003151260504 |
| HL-1; HL-10; HL-22 and HL-35 | 0.961003151260504 |
| HL-3; HL-5; HL-25 and HL-26 | 0.961003151260504 |
| HL-4; HL-5; HL-8 and HL-11 | 0.961003151260504 |
| HL-5; HL-8; HL-11 and HL-47 | 0.961003151260504 |
| HL-8; HL-11; HL-13 and HL-26 | 0.961003151260504 |
| HL-8; HL-11; HL-21 and HL-26 | 0.961003151260504 |
| HL-1; HL-5; HL-13 and HL-27 | 0.960970325630253 |
| HL-1; HL-5; HL-40 and HL-47 | 0.960970325630253 |
| HL-1; HL-5; HL-13 and HL-22 | 0.960970325630252 |
| HL-1; HL-10; HL-27 and HL-28 | 0.960970325630252 |
| HL-1; HL-3; HL-5 and HL-46 | 0.960937500000001 |
| HL-1; HL-5; HL-15 and HL-17 | 0.960937500000001 |
| HL-1; HL-5; HL-12 and HL-32 | 0.9609375 |
| HL-1; HL-5; HL-17 and HL-32 | 0.9609375 |
| HL-1; HL-5; HL-17 and HL-39 | 0.9609375 |
| HL-3; HL-5; HL-8 and HL-45 | 0.9609375 |
| HL-4; HL-8; HL-26 and HL-31 | 0.9609375 |
| HL-5; HL-8; HL-11 and HL-25 | 0.9609375 |
| HL-5; HL-8; HL-17 and HL-37 | 0.9609375 |
| HL-5; HL-10; HL-11 and HL-45 | 0.9609375 |
| HL-8; HL-11; HL-26 and HL-35 | 0.9609375 |
| HL-8; HL-21; HL-25 and HL-53 | 0.9609375 |
| HL-10; HL-11; HL-13 and HL-53 | 0.9609375 |
| HL-1; HL-8; HL-10 and HL-28 | 0.960904674369748 |
| HL-1; HL-10; HL-27 and HL-47 | 0.960904674369748 |
| HL-8; HL-10; HL-12 and HL-53 | 0.960904674369748 |
| HL-1; HL-3; HL-10 and HL-17 | 0.960871848739496 |
| HL-1; HL-4; HL-5 and HL-51 | 0.960871848739496 |
| HL-1; HL-5; HL-11 and HL-22 | 0.960871848739496 |
| HL-1; HL-5; HL-11 and HL-33 | 0.960871848739496 |
| HL-1; HL-5; HL-13 and HL-53 | 0.960871848739496 |
| HL-3; HL-4; HL-10 and HL-26 | 0.960871848739496 |
| HL-3; HL-8; HL-11 and HL-13 | 0.960871848739496 |
| HL-3; HL-18; HL-26 and HL-35 | 0.960871848739496 |
| HL-7; HL-8; HL-13 and HL-26 | 0.960871848739496 |
| HL-8; HL-13; HL-15 and HL-21 | 0.960871848739496 |
| HL-8; HL-26; HL-35 and HL-45 | 0.960871848739496 |
| HL-10; HL-11; HL-27 and HL-37 | 0.960871848739496 |
| HL-1; HL-5; HL-18 and HL-28 | 0.960839023109244 |
| HL-3; HL-26; HL-27 and HL-53 | 0.960839023109243 |
| HL-3; HL-8; HL-9 and HL-45 | 0.960806197478992 |
| HL-7; HL-10; HL-13 and HL-21 | 0.960806197478992 |
| HL-8; HL-10; HL-11 and HL-17 | 0.960806197478992 |
| HL-10; HL-26; HL-27 and HL-53 | 0.960806197478992 |
| HL-3; HL-8; HL-27 and HL-53 | 0.960806197478991 |
| HL-5; HL-17; HL-28 and HL-45 | 0.960806197478991 |
| HL-8; HL-26; HL-35 and HL-47 | 0.960806197478991 |
| HL-10; HL-18; HL-21 and HL-26 | 0.960806197478991 |
| HL-1; HL-3; HL-5 and HL-18 | 0.96077337184874 |
| HL-1; HL-10; HL-28 and HL-35 | 0.96077337184874 |
| HL-8; HL-12; HL-18 and HL-26 | 0.96077337184874 |
| HL-5; HL-8; HL-17 and HL-25 | 0.960773371848739 |
| HL-5; HL-8; HL-17 and HL-47 | 0.960773371848739 |
| HL-5; HL-10; HL-17 and HL-18 | 0.960773371848739 |
| HL-1; HL-5; HL-11 and HL-15 | 0.960740546218488 |
| HL-1; HL-5; HL-23 and HL-25 | 0.960740546218488 |
| HL-1; HL-5; HL-26 and HL-53 | 0.960740546218488 |
| HL-10; HL-17; HL-25 and HL-53 | 0.960740546218488 |
| HL-1; HL-4; HL-10 and HL-26 | 0.960740546218487 |
| HL-1; HL-5; HL-9 and HL-35 | 0.960740546218487 |
| HL-3; HL-8; HL-11 and HL-18 | 0.960740546218487 |
| HL-3; HL-8; HL-18 and HL-45 | 0.960740546218487 |
| HL-3; HL-18; HL-21 and HL-53 | 0.960740546218487 |
| HL-3; HL-26; HL-51 and HL-53 | 0.960740546218487 |
| HL-4; HL-10; HL-13 and HL-21 | 0.960740546218487 |
| HL-4; HL-10; HL-13 and HL-26 | 0.960740546218487 |
| HL-5; HL-10; HL-21 and HL-22 | 0.960740546218487 |
| HL-8; HL-10; HL-11 and HL-12 | 0.960740546218487 |
| HL-8; HL-10; HL-11 and HL-18 | 0.960740546218487 |
| HL-8; HL-10; HL-17 and HL-31 | 0.960740546218487 |
| HL-8; HL-12; HL-25 and HL-26 | 0.960740546218487 |
| HL-8; HL-21; HL-51 and HL-53 | 0.960740546218487 |
| HL-8; HL-25; HL-26 and HL-40 | 0.960740546218487 |
| HL-1; HL-8; HL-10 and HL-12 | 0.960707720588236 |
| HL-1; HL-8; HL-10 and HL-40 | 0.960707720588236 |
| HL-5; HL-7; HL-10 and HL-17 | 0.960707720588236 |
| HL-1; HL-5; HL-8 and HL-31 | 0.960674894957984 |
| HL-1; HL-5; HL-12 and HL-13 | 0.960674894957984 |
| HL-4; HL-5; HL-11 and HL-17 | 0.960674894957984 |
| HL-1; HL-4; HL-5 and HL-37 | 0.960674894957983 |
| HL-1; HL-4; HL-10 and HL-28 | 0.960674894957983 |
| HL-1; HL-5; HL-11 and HL-45 | 0.960674894957983 |
| HL-1; HL-10; HL-26 and HL-28 | 0.960674894957983 |
| HL-3; HL-13; HL-18 and HL-53 | 0.960674894957983 |
| HL-3; HL-18; HL-25 and HL-26 | 0.960674894957983 |
| HL-5; HL-8; HL-12 and HL-17 | 0.960674894957983 |
| HL-5; HL-10; HL-13 and HL-35 | 0.960674894957983 |
| HL-8; HL-10; HL-13 and HL-45 | 0.960674894957983 |
| HL-8; HL-10; HL-35 and HL-45 | 0.960674894957983 |
| HL-8; HL-12; HL-26 and HL-37 | 0.960674894957983 |
| HL-8; HL-13; HL-47 and HL-53 | 0.960674894957983 |
| HL-8; HL-13; HL-51 and HL-53 | 0.960674894957983 |
| HL-8; HL-21; HL-53 and HL-56 | 0.960674894957983 |
| HL-13; HL-22; HL-26 and HL-53 | 0.960674894957983 |
| HL-1; HL-3; HL-10 and HL-33 | 0.960642069327731 |
| HL-8; HL-12; HL-26 and HL-31 | 0.960642069327731 |
| HL-1; HL-5; HL-9 and HL-12 | 0.960609243697479 |
| HL-1; HL-5; HL-21 and HL-36 | 0.960609243697479 |
| HL-5; HL-8; HL-26 and HL-45 | 0.960609243697479 |
| HL-8; HL-10; HL-17 and HL-36 | 0.960609243697479 |
| HL-8; HL-21; HL-26 and HL-47 | 0.960609243697479 |
| HL-8; HL-26; HL-35 and HL-37 | 0.960609243697479 |
| HL-13; HL-21; HL-26 and HL-53 | 0.960609243697479 |
| HL-1; HL-3; HL-5 and HL-39 | 0.960576418067227 |
| HL-1; HL-5; HL-33 and HL-51 | 0.960576418067227 |
| HL-1; HL-10; HL-13 and HL-28 | 0.960576418067227 |
| HL-1; HL-10; HL-22 and HL-27 | 0.960576418067227 |
| HL-4; HL-10; HL-26 and HL-53 | 0.960576418067227 |
| HL-10; HL-21; HL-27 and HL-37 | 0.960576418067227 |
| HL-1; HL-5; HL-9 and HL-13 | 0.960543592436975 |
| HL-1; HL-5; HL-9 and HL-26 | 0.960543592436975 |
| HL-1; HL-10; HL-26 and HL-47 | 0.960543592436975 |
| HL-3; HL-5; HL-17 and HL-28 | 0.960543592436975 |
| HL-3; HL-5; HL-26 and HL-32 | 0.960543592436975 |
| HL-4; HL-5; HL-11 and HL-13 | 0.960543592436975 |
| HL-4; HL-13; HL-26 and HL-53 | 0.960543592436975 |
| HL-5; HL-10; HL-11 and HL-22 | 0.960543592436975 |
| HL-8; HL-26; HL-33 and HL-35 | 0.960543592436975 |
| HL-8; HL-26; HL-33 and HL-47 | 0.960543592436975 |
| HL-1; HL-3; HL-8 and HL-22 | 0.960510766806723 |
| HL-1; HL-5; HL-12 and HL-53 | 0.960510766806723 |
| HL-1; HL-5; HL-25 and HL-37 | 0.960510766806723 |
| HL-13; HL-26; HL-37 and HL-53 | 0.960510766806723 |
| HL-5; HL-8; HL-10 and HL-21 | 0.960510766806722 |
| HL-1; HL-3; HL-10 and HL-40 | 0.960477941176471 |
| HL-1; HL-4; HL-5 and HL-45 | 0.960477941176471 |
| HL-1; HL-5; HL-12 and HL-23 | 0.960477941176471 |
| HL-3; HL-5; HL-11 and HL-25 | 0.960477941176471 |
| HL-3; HL-11; HL-37 and HL-53 | 0.960477941176471 |
| HL-5; HL-8; HL-16 and HL-17 | 0.960477941176471 |
| HL-5; HL-17; HL-25 and HL-45 | 0.960477941176471 |
| HL-8; HL-26; HL-45 and HL-53 | 0.960477941176471 |
| HL-10; HL-11; HL-22 and HL-27 | 0.960477941176471 |
| HL-10; HL-21; HL-25 and HL-26 | 0.960477941176471 |
| HL-8; HL-13; HL-22 and HL-53 | 0.96047794117647 |
| HL-8; HL-22; HL-26 and HL-27 | 0.96047794117647 |
| HL-10; HL-13; HL-25 and HL-53 | 0.96047794117647 |
| HL-1; HL-5; HL-22 and HL-23 | 0.960445115546219 |
| HL-1; HL-3; HL-10 and HL-37 | 0.960445115546218 |
| HL-1; HL-10; HL-13 and HL-35 | 0.960445115546218 |
| HL-3; HL-4; HL-10 and HL-53 | 0.960445115546218 |
| HL-8; HL-12; HL-26 and HL-27 | 0.960445115546218 |
| HL-8; HL-12; HL-26 and HL-51 | 0.960445115546218 |
| HL-8; HL-13; HL-37 and HL-53 | 0.960445115546218 |
| HL-13; HL-26; HL-33 and HL-53 | 0.960445115546218 |
| HL-1; HL-5; HL-31 and HL-47 | 0.960412289915967 |
| HL-8; HL-10; HL-11 and HL-49 | 0.960412289915967 |
| HL-1; HL-4; HL-10 and HL-47 | 0.960412289915966 |
| HL-1; HL-5; HL-7 and HL-21 | 0.960412289915966 |
| HL-3; HL-4; HL-8 and HL-53 | 0.960412289915966 |
| HL-3; HL-4; HL-13 and HL-26 | 0.960412289915966 |
| HL-3; HL-8; HL-11 and HL-39 | 0.960412289915966 |
| HL-7; HL-21; HL-25 and HL-53 | 0.960412289915966 |
| HL-10; HL-11; HL-37 and HL-38 | 0.960412289915966 |
| HL-10; HL-13; HL-47 and HL-53 | 0.960412289915966 |
| HL-13; HL-18; HL-35 and HL-53 | 0.960412289915966 |
| HL-1; HL-5; HL-8 and HL-45 | 0.960379464285715 |
| HL-1; HL-5; HL-10 and HL-14 | 0.960379464285715 |
| HL-1; HL-5; HL-21 and HL-25 | 0.960379464285715 |
| HL-1; HL-5; HL-26 and HL-51 | 0.960379464285715 |
| HL-1; HL-8; HL-10 and HL-51 | 0.960379464285715 |
| HL-3; HL-10; HL-22 and HL-53 | 0.960379464285714 |
| HL-1; HL-5; HL-21 and HL-47 | 0.960346638655463 |
| HL-1; HL-4; HL-5 and HL-11 | 0.960346638655462 |
| HL-1; HL-5; HL-10 and HL-57 | 0.960346638655462 |
| HL-3; HL-4; HL-10 and HL-11 | 0.960346638655462 |
| HL-3; HL-4; HL-21 and HL-27 | 0.960346638655462 |
| HL-3; HL-8; HL-12 and HL-53 | 0.960346638655462 |
| HL-5; HL-8; HL-11 and HL-22 | 0.960346638655462 |
| HL-8; HL-9; HL-10 and HL-45 | 0.960346638655462 |
| HL-8; HL-10; HL-31 and HL-53 | 0.960346638655462 |
| HL-8; HL-22; HL-26 and HL-51 | 0.960346638655462 |
| HL-10; HL-11; HL-13 and HL-37 | 0.960346638655462 |
| HL-15; HL-21; HL-25 and HL-53 | 0.960346638655462 |
| HL-1; HL-5; HL-25 and HL-53 | 0.960313813025211 |
| HL-1; HL-3; HL-10 and HL-12 | 0.96031381302521 |
| HL-1; HL-5; HL-23 and HL-33 | 0.96031381302521 |
| HL-1; HL-8; HL-10 and HL-33 | 0.96031381302521 |
| HL-8; HL-12; HL-26 and HL-33 | 0.96031381302521 |
| HL-1; HL-5; HL-9 and HL-27 | 0.960280987394958 |
| HL-1; HL-5; HL-13 and HL-32 | 0.960280987394958 |
| HL-1; HL-5; HL-33 and HL-37 | 0.960280987394958 |
| HL-1; HL-8; HL-10 and HL-25 | 0.960280987394958 |
| HL-3; HL-5; HL-11 and HL-22 | 0.960280987394958 |
| HL-3; HL-10; HL-13 and HL-53 | 0.960280987394958 |
| HL-4; HL-8; HL-15 and HL-21 | 0.960280987394958 |
| HL-5; HL-8; HL-11 and HL-40 | 0.960280987394958 |
| HL-5; HL-8; HL-21 and HL-26 | 0.960280987394958 |
| HL-5; HL-11; HL-26 and HL-27 | 0.960280987394958 |
| HL-8; HL-11; HL-21 and HL-53 | 0.960280987394958 |
| HL-10; HL-11; HL-13 and HL-49 | 0.960280987394958 |
| HL-10; HL-11; HL-27 and HL-53 | 0.960280987394958 |
| HL-10; HL-21; HL-32 and HL-53 | 0.960280987394958 |
| HL-1; HL-10; HL-12 and HL-22 | 0.960248161764706 |
| HL-1; HL-10; HL-23 and HL-47 | 0.960248161764706 |
| HL-1; HL-10; HL-35 and HL-47 | 0.960248161764706 |
| HL-3; HL-5; HL-10 and HL-22 | 0.960248161764706 |
| HL-4; HL-5; HL-10 and HL-21 | 0.960248161764706 |
| HL-5; HL-8; HL-12 and HL-26 | 0.960248161764706 |
| HL-10; HL-26; HL-35 and HL-53 | 0.960248161764706 |
| HL-1; HL-4; HL-5 and HL-31 | 0.960215336134454 |
| HL-1; HL-5; HL-9 and HL-25 | 0.960215336134454 |
| HL-1; HL-5; HL-11 and HL-23 | 0.960215336134454 |
| HL-1; HL-5; HL-25 and HL-51 | 0.960215336134454 |
| HL-1; HL-5; HL-33 and HL-40 | 0.960215336134454 |
| HL-1; HL-8; HL-10 and HL-32 | 0.960215336134454 |
| HL-1; HL-10; HL-13 and HL-22 | 0.960215336134454 |
| HL-1; HL-10; HL-27 and HL-35 | 0.960215336134454 |
| HL-8; HL-10; HL-22 and HL-26 | 0.960215336134454 |
| HL-8; HL-18; HL-26 and HL-51 | 0.960215336134454 |
| HL-8; HL-21; HL-22 and HL-53 | 0.960215336134454 |
| HL-10; HL-11; HL-13 and HL-15 | 0.960215336134454 |
| HL-3; HL-10; HL-15 and HL-21 | 0.960215336134453 |
| HL-4; HL-8; HL-18 and HL-26 | 0.960215336134453 |
| HL-1; HL-5; HL-7 and HL-10 | 0.960182510504202 |
| HL-1; HL-5; HL-25 and HL-40 | 0.960182510504202 |
| HL-8; HL-18; HL-26 and HL-33 | 0.960182510504202 |
| HL-3; HL-10; HL-18 and HL-53 | 0.960182510504201 |
| HL-1; HL-4; HL-10 and HL-27 | 0.96014968487395 |
| HL-1; HL-5; HL-27 and HL-32 | 0.96014968487395 |
| HL-3; HL-8; HL-12 and HL-45 | 0.96014968487395 |
| HL-3; HL-21; HL-26 and HL-32 | 0.96014968487395 |
| HL-4; HL-5; HL-17 and HL-45 | 0.96014968487395 |
| HL-8; HL-18; HL-26 and HL-31 | 0.96014968487395 |
| HL-8; HL-21; HL-26 and HL-32 | 0.96014968487395 |
| HL-3; HL-10; HL-37 and HL-53 | 0.960149684873949 |
| HL-5; HL-8; HL-11 and HL-53 | 0.960149684873949 |
| HL-5; HL-11; HL-17 and HL-45 | 0.960149684873949 |
| HL-1; HL-3; HL-5 and HL-7 | 0.960116859243698 |
| HL-1; HL-5; HL-13 and HL-23 | 0.960116859243698 |
| HL-1; HL-5; HL-23 and HL-26 | 0.960116859243698 |
| HL-4; HL-5; HL-13 and HL-17 | 0.960116859243698 |
| HL-4; HL-8; HL-22 and HL-26 | 0.960116859243697 |
| HL-8; HL-25; HL-26 and HL-27 | 0.960116859243697 |
| HL-1; HL-4; HL-5 and HL-9 | 0.960084033613446 |
| HL-3; HL-8; HL-11 and HL-36 | 0.960084033613446 |
| HL-1; HL-5; HL-22 and HL-32 | 0.960084033613445 |
| HL-3; HL-10; HL-11 and HL-22 | 0.960084033613445 |
| HL-3; HL-10; HL-21 and HL-27 | 0.960084033613445 |
| HL-3; HL-26; HL-31 and HL-53 | 0.960084033613445 |
| HL-4; HL-5; HL-8 and HL-26 | 0.960084033613445 |
| HL-4; HL-5; HL-25 and HL-26 | 0.960084033613445 |
| HL-5; HL-8; HL-11 and HL-39 | 0.960084033613445 |
| HL-8; HL-10; HL-45 and HL-53 | 0.960084033613445 |
| HL-8; HL-13; HL-25 and HL-53 | 0.960051207983193 |
| HL-1; HL-5; HL-26 and HL-40 | 0.960018382352942 |
| HL-1; HL-5; HL-31 and HL-35 | 0.960018382352942 |
| HL-1; HL-5; HL-33 and HL-53 | 0.960018382352942 |
| HL-1; HL-5; HL-45 and HL-47 | 0.960018382352942 |
| HL-1; HL-3; HL-8 and HL-35 | 0.960018382352941 |
| HL-1; HL-3; HL-10 and HL-25 | 0.960018382352941 |
| HL-1; HL-3; HL-10 and HL-36 | 0.960018382352941 |
| HL-1; HL-5; HL-26 and HL-36 | 0.960018382352941 |
| HL-1; HL-5; HL-27 and HL-36 | 0.960018382352941 |
| HL-1; HL-10; HL-32 and HL-47 | 0.960018382352941 |
| HL-3; HL-8; HL-11 and HL-45 | 0.960018382352941 |
| HL-3; HL-18; HL-26 and HL-51 | 0.960018382352941 |
| HL-5; HL-8; HL-10 and HL-26 | 0.960018382352941 |
| HL-5; HL-10; HL-11 and HL-12 | 0.960018382352941 |
| HL-5; HL-11; HL-13 and HL-21 | 0.960018382352941 |
| HL-8; HL-21; HL-23 and HL-53 | 0.960018382352941 |
| HL-8; HL-21; HL-26 and HL-56 | 0.960018382352941 |
| HL-8; HL-25; HL-26 and HL-47 | 0.960018382352941 |
| HL-8; HL-26; HL-32 and HL-35 | 0.960018382352941 |
| HL-10; HL-13; HL-26 and HL-28 | 0.960018382352941 |
| HL-10; HL-18; HL-26 and HL-32 | 0.960018382352941 |

**Table 10: bacterial species and combinations of 2 to 20 bacterial species giving the best AUC in the method of the invention.**

| combinations of bacterials species | AUC |
|---|---|
| HL-1 | 0.936055672 |
| HL-1 and HL-5 | 0.955685399 |
| HL-10, HL-1 and HL-5 | 0.964778099 |
| HL-8, HL-3, HL-53 and HL-26 | 0.975380777 |
| HL-10, HL-26, HL-8, HL-53 and HL-3 | 0.976070116 |
| HL-53, HL-8, HL-13, HL-3, HL-26 and HL-37 | 0.97761292 |
| HL-37, HL-26, HL-10, HL-8, HL-21, HL-53 and HL-11 | 0.979713761 |
| HL-10, HL-5, HL-26, HL-25, HL-53, HL-22, HL-8 and HL-17 | 0.980370273 |
| HL-26, HL-37, HL-21, HL-10, HL-5, HL-17, HL-16, HL-8 and HL-3 | 0.981026786 |
| HL-15, HL-11, HL-27, HL-35, HL-8, HL-22, HL-47, HL-26, HL-10 and HL-37 | 0.983652836 |
| HL-28, HL-21, HL-5, HL-27, HL-26, HL-17, HL-3, HL-38, HL-40, HL-37 and HL-25 | 0.981026786 |
| HL-8, HL-45, HL-35, HL-53, HL-17, HL-26, HL-3, HL-18, HL-15, HL-10, HL-37 and HL-40 | 0.985556723 |
| HL-33, HL-13, HL-10, HL-28, HL-36, HL-17, HL-8, HL-3, HL-22, HL-53, HL-35, HL-5 and HL-27 | 0.982405462 |
| HL-56, HL-17, HL-21, HL-35, HL-40, HL-26, HL-12, HL-13, HL-45, HL-3, HL-5, HL-10, HL-8 and HL-27 | 0.983193277 |
| HL-31, HL-11, HL-25, HL-10, HL-35, HL-12, HL-28, HL-37, HL-5, HL-15, HL-33, HL-17, HL-51, HL-27 and HL-40 | 0.981026786 |
| HL-33, HL-51, HL-39, HL-27, HL-56, HL-31, HL-23, HL-10, HL-18, HL-4, HL-11, HL-8, HL-21, HL-45, HL-5 and HL-17 | 0.981617647 |
| HL-45, HL-27, HL-47, HL-5, HL-51, HL-8, HL-26, HL-3, HL-53, HL-37, HL-13, HL-11, HL-38, HL-17, HL-23, HL-1 and HL-28 | 0.982602416 |
| HL-31, HL-11, HL-33, HL-49, HL-38, HL-28, HL-36, HL-21, HL-22, HL-4, HL-37, HL-45, HL-27, HL-15, HL-51, HL-8, HL-17 and HL-56 | 0.982208508 |
| HL-39, HL-18, HL-56, HL-28, HL-36, HL-45, HL-49, HL-7, HL-17, HL-35, HL-33, HL-11, HL-5, HL-8, HL-10, HL-12, HL-15, HL-25 and HL-22 | 0.981551996 |
| HL-47, HL-5, HL-15, HL-36, HL-37, HL-35, HL-44, HL-11, HL-8, HL-17, HL-31, HL-18, HL-13, HL-21, HL-51, HL-4, HL-28, HL-45, HL-33 and HL-3 | 0.982536765 |

### References

1 Jorgensen, T. et al. A randomized non-pharmacological intervention study for prevention of ischaemic heart disease: baseline results Inter99. Eur J Cardiovasc Prev Rehabil 10, 377-386, doi:10.1097/01.hjr.0000096541.30533.82 (2003).
2 WHO. Obesity: preventing and managing the globalepidemic. Report of a WHO consultation. Tech. Rep. Ser. 894 (World Health Organisation, Geneva, 2000).
3 Treuth, M. S., Hunter, G. R. & Kekes-Szabo, T. Estimating intraabdominal adipose tissue in women by dual-energy X-ray absorptiometry. Am J Clin Nutr 62, 527-532 (1995).
4 Matthews, D. R. et al. Homeostasis model assessment: insulin resistance and beta-cell function from fasting plasma glucose and insulin concentrations in man. Diabetologia 28, 412-419 (1985).
5 Manichanh, C. et al. Reduced diversity of faecal microbiota in Crohn's disease revealed by a metagenomic approach. Gut 55, 205-211, doi:gut.2005.073817 [pii] 10.1136/gut.2005.073817 (2006).
6 Li, R. et al. SOAP2: an improved ultrafast tool for short read alignment. Bioinformatics 25, 1966-1967, doi:btp336 [pii] 10.1093/bioinformatics/btp336 (2009).
7 Oksanen, J. et al. vegan: Community Ecology Package. (2012).
8 Rajilic-Stojanovic, M. et al. Development and application of the human intestinal tract chip, a phylogenetic microarray: analysis of universally conserved phylotypes in the abundant microbiota of young and elderly adults. Environ Microbiol 11, 1736-1751, doi:EMI1900 [pii] 10 1111/j.1462-2920.2009.01900.x (2009).
9 Qin, J. et al. A human gut microbial gene catalogue established by metagenomic sequencing. Nature 464, 59-65, doi:nature08821 [pii] 10.1038/nature08821 (2010).
10 Arumugam, M. et al. Enterotypes of the human gut microbiome. Nature 473, 174-180, doi:nature09944 [pii] 10.1038/nature09944 (2011).
11 Benjamini, Y. H., Y. Controlling the false discovery rate: a practical and powerful approach to multiple testning. Journal of the Royal Statistical Society 57, 289-300 (1995).
12 Jensen, L. J. et al. eggNOG: automated construction and annotation of orthologous groups of genes. Nucleic Acids Res 36, D250-254, doi:gkm796 [pii] 10.1093/nar/gkm796 (2008).
13 Kanehisa, M., Goto, S., Sato, Y., Furumichi, M. & Tanabe, M. KEGG for integration and interpretation of large-scale molecular data sets. Nucleic Acids Res 40, D109-114, doi:gkr988 [pii] 10.1093/nar/gkr988 (2012).
14 Jiang, D., Huang, J. & Zhang, Y. The cross-validated AUC for MCP-logistic regression with high-dimensional data. Stat Methods Med Res, doi:0962280211428385 [pii] 10.1177/0962280211428385 (2011).

## Claims

1. A method for determining whether a subject has a reduced gut bacterial diversity, said method comprising:
a) detecting from a gut microbial DNA sample which has been obtained from said subject whether
all of the 50 SEQ ID NOs of at least one bacterial species as defined in Table 1 are absent in said sample; and
b) determining that the subject has a reduced gut bacterial diversity, if said all 50 SEQ ID NOs of said at least one bacterial species as defined in Table 1 are absent in said sample.

2. A method for determining whether a subject has a reduced gut bacterial diversity, said method comprising:
a) detecting from a gut microbial DNA sample which has been obtained from said subject whether
all 50 SEQ ID NOs of at least one bacterial species as defined in Table 2 are present in said sample; and
b) determining that the subject has a reduced gut bacterial diversity, if said all 50 SEQ ID NOs of said at least one bacterial species as defined in Table 2 are present in said sample.

3. A method according to claim 1, **characterised in that** it comprises a step of determining from a gut microbial DNA sample which has been obtained from said subject whether all 50 SEQ ID NOs of at least one bacterial species as defined in Table 1 selected from SEQ ID NO: 1-50, SEQ ID NO: 2801-2850, SEQ ID NO: 2601-2650, SEQ ID NO: 151-200, SEQ ID NO: 2651-2700, SEQ ID NO: 51-100, SEQ ID NO: 101-150, SEQ ID NO: 351-400, SEQ ID NO: 451-500, SEQ ID NO: 2201-2250, SEQ ID NO: 1051-1100, SEQ ID NO: 1251-1300, SEQ ID NO: 401-450, SEQ ID NO: 201-250, SEQ ID NO: 501-550, SEQ ID NO: 651-700, SEQ ID NO: 601-650, SEQ ID NO: 851-900, SEQ ID NO: 551-600 and SEQ ID NO: 1001-1050 are absent in said sample.

4. A method according to anyone of claims 1 to 3, **characterised in that** it comprises a step of detecting from a gut microbial DNA sample which has been obtained from said subject whether all 50 SEQ ID NOs of bacterial species as defined in Table 1 or Table 2 of the bacterial species combinations indicated in table 7, 8 and/or 9 are absent and/or present in said sample.

5. A method according to anyone of claims 1 to 4, **characterised in that** it comprises a step of detecting from a gut microbial DNA sample which has been obtained from said subject whether:
- SEQ ID NO: 1-50 and SEQ ID NO: 201-250 are absent in said sample, or;
- SEQ ID NO: 451-500, SEQ ID NO: 1-50 and SEQ ID NO: 201-250 are absent in said sample, or;
- SEQ ID NO: 351-400, SEQ ID NO: 101-150, SEQ ID NO: 1251-1300 and SEQ ID NO: 2601-2650 are absent in said sample, or;
- SEQ ID NO: 451-500, SEQ ID NO: 1251-1300, SEQ ID NO: 351-400, SEQ ID NO: 2601-2650 and SEQ ID NO: 101-150 are absent in said sample, or;
- SEQ ID NO: 2601-2650, SEQ ID NO: 351-400, SEQ ID NO: 601-650, SEQ ID NO: 101-150, SEQ ID NO: 1251-1300 and SEQ ID NO: 1801-1850 are absent in said sample, or;
- SEQ ID NO: 1801-1850, SEQ ID NO: 1251-1300, SEQ ID NO: 451-500, SEQ ID NO: 351-400, SEQ ID NO: 1001-1050, SEQ ID NO: 2601-2650 and SEQ ID NO: 501-550 are absent in said sample, or;
- SEQ ID NO: 451-500, SEQ ID NO: 201-250, SEQ ID NO: 1251-1300, SEQ ID NO: 1201-1250, SEQ ID NO: 2601-2650, SEQ ID NO: 1051-1100, SEQ ID NO: 351-400 and SEQ ID NO: 801-850 are absent in said sample, or;
- SEQ ID NO: 1251-1300, SEQ ID NO: 1801-1850, SEQ ID NO: 1001-1050, SEQ ID NO: 451-500, SEQ ID NO: 201-250, SEQ ID NO: 801-850, SEQ ID NO: 751-800, SEQ ID NO: 351-400 and SEQ ID NO: 101-150 are absent in said sample, or;
- SEQ ID NO: 501-550, SEQ ID NO: 1301-1350, SEQ ID NO: 1701-1750, SEQ ID NO: 351-400, SEQ ID NO: 1051-1100, SEQ ID NO: 2301-2350, SEQ ID NO: 1251-1300, SEQ ID NO: 451-500 and SEQ ID NO: 1801-1850 are absent, and SEQ ID NO: 701-750 are present, in said sample;
- SEQ ID NO: 1351-1400, SEQ ID NO: 1001-1050, SEQ ID NO: 201-250, SEQ ID NO: 1301-1350, SEQ ID NO: 1251-1300, SEQ ID NO: 801-850, SEQ ID NO: 101-150, SEQ ID NO: 1951-2000, SEQ ID NO: 1801-1850 and SEQ ID NO: 1201-1250 are absent, and SEQ ID NO: 1851-1900 are present, in said sample;
- SEQ ID NO: 351-400, SEQ ID NO: 2201-2250; SEQ ID NO: 1701-1750, SEQ ID NO: 2601-2650, SEQ ID NO: 801-850, SEQ ID NO: 1251-1300, SEQ ID NO: 101-150, SEQ ID NO: 851-900, SEQ ID NO: 451-500, SEQ ID NO: 1801-1850 and SEQ ID NO: 1951-2000 are absent, and SEQ ID NO: 701-750 are present, in said sample;
- SEQ ID NO: 1601-1650, SEQ ID NO: 601-650; SEQ ID NO: 451-500, SEQ ID NO: 1351-1400, SEQ ID NO: 1751-1800, SEQ ID NO: 801-850, SEQ ID NO: 351-400, SEQ ID NO: 101-150, SEQ ID NO: 1051-1100, SEQ ID NO: 2601-2650, SEQ ID NO: 1701-1750, SEQ ID NO: 201-250 and SEQ ID NO: 1301-1350 are absent in said sample, or;
- SEQ ID NO: 1601-1650, SEQ ID NO: 601-650; SEQ ID NO: 451-500, SEQ ID NO: 1351-1400, SEQ ID NO: 1751-1800, SEQ ID NO: 801-850, SEQ ID NO: 351-400, SEQ ID NO: 101-150, SEQ ID NO: 1051-1100, SEQ ID NO: 2601-2650, SEQ ID NO: 1701-1750, SEQ ID NO: 201-250 and SEQ ID NO: 1301-1350 are absent in said sample, or;
- SEQ ID NO: 1501-1550, SEQ ID NO: 501-550; SEQ ID NO: 1201-1250, SEQ ID NO: 451-500, SEQ ID NO: 1701-1750, SEQ ID NO: 551-600, SEQ ID NO: 1351-1400, SEQ ID NO: 1801-1850, SEQ ID NO: 201-250, SEQ ID NO: 1601-1650, SEQ ID NO: 801-850, SEQ ID NO: 2501-2550, SEQ ID NO: 1301-1350 and SEQ ID NO: 1951-2000 are absent, and SEQ ID NO: 701-750 are present, in said sample;
- SEQ ID NO: 1601-1650, SEQ ID NO: 2501-2550, SEQ ID NO: 1301-1350, SEQ ID NO: 1501-1550, SEQ ID NO: 1101-1150, SEQ ID NO: 451-500, SEQ ID NO: 851-900, SEQ ID NO: 151-200, SEQ ID NO: 501-550, SEQ ID NO: 351-400, SEQ ID NO: 1001-1050, SEQ ID NO: 2201-2250, SEQ ID NO: 201-250 and SEQ ID NO: 801-850 are absent in said sample, and SEQ ID NO: 701-750, SEQ ID NO: 1901-1950 and SEQ ID NO: 2751-2800 from table 2 are present or;
- SEQ ID NO: 2201-2250, SEQ ID NO: 1301-1350, SEQ ID NO: 2301-2350, SEQ ID NO: 201-250, SEQ ID NO: 2501-2550, SEQ ID NO: 351-400, SEQ ID NO: 1251-1300, SEQ ID NO: 101-150, SEQ ID NO: 2601-2650, SEQ ID NO: 1801-1850, SEQ ID NO: 601-650, SEQ ID NO: 501-550, SEQ ID NO: 801-850, SEQ ID NO: 1101-1150, SEQ ID NO: 1-50 and SEQ ID NO: 1351-1400 are absent, and SEQ ID NO: 1851-1900 are present, in said sample;
- SEQ ID NO: 1501-1550, SEQ ID NO: 501-550, SEQ ID NO: 1601-1650, SEQ ID NO: 1351-1400, SEQ ID NO: 1751-1800, SEQ ID NO: 1001-1050, SEQ ID NO: 1051-1100, SEQ ID NO: 151-200, SEQ ID NO: 1801-1850, SEQ ID NO: 2201-2250, SEQ ID NO: 1301-1350, SEQ ID NO: 701-750, SEQ ID NO: 2501-2550, SEQ ID NO: 351-400 and SEQ ID NO: 801-850 are absent, and SEQ ID NO: 701-750, SEQ ID NO: 2401-2450, SEQ ID NO: 1851-1900 and SEQ ID NO: 2751-2800 are present, in said sample;
- SEQ ID NO: 851-900, SEQ ID NO: 2751-2800, 1351-1400, SEQ ID NO: 1751-1800, SEQ ID NO: 2201-2250, SEQ ID NO: 801-850, SEQ ID NO: 1701-1750, SEQ ID NO: 1601-1650, SEQ ID NO: 501-550, SEQ ID NO: 201-250, SEQ ID NO: 351-400, SEQ ID NO: 451-500, SEQ ID NO: 551-600, SEQ ID NO: 1201-1250 and SEQ ID NO: 1051-1110 are absent, and SEQ ID NO: 1901-1950, SEQ ID NO: 2401-2450, SEQ ID NO: 301-350, SEQ ID NO: 701-750 and SEQ ID NO: 2751-2800 are present, in said sample;
- SEQ ID NO: 2301-2350, SEQ ID NO: 201-250, SEQ ID NO: 1751-1800, SEQ ID NO: 1801-1850, SEQ ID NO: 1701-1750, SEQ ID NO: 2151-2200, SEQ ID NO: 501-550, SEQ ID NO: 351-400, SEQ ID NO: 801-850, SEQ ID NO: 1501-1550, SEQ ID NO: 851-900, SEQ ID NO: 601-650, SEQ ID NO: 1001-1050, SEQ ID NO: 2501-2550, SEQ ID NO: 151-200, SEQ ID NO: 1351-1400, SEQ ID NO: 2201-2250, SEQ ID NO: 1601-1650 and SEQ ID NO: 101-150 are absent, and SEQ ID NO: 701-750 are present, in said sample.

6. A method according to any of claims 1 to 5, **characterized in that** it comprises a step of detecting from a gut microbial DNA sample which has been obtained from said subject whether all of the said 50 SEQ ID NOs: 1-50 are absent in said sample.

7. A method according to any of claims 1 to 6, **characterized in that** the presence or absence of the bacterial genes is detected by the use of a nucleic microarray.

8. A method according to claim 7, **characterized in that** the nucleic microarray is an oligonucleotide microarray comprising at least one oligonucleotide specific for at least 50 bacterial genes having a sequence selected from SEQ ID NOs 1-2900.

9. Method for the *in vitro* risk assessment of developing metabolic disorders, preferentially type II diabetes, hyperglycemic syndrome, heart diseases, insulin resistance or hepatic stasis, comprising the steps of:
a) Determining whether a subject has a reduced gut bacterial diversity with a method according to any of claims 1 to 8; and
b) If said subject has a reduced gut bacterial diversity, assessing that said subject is at risk to develop said metabolic disorders.

10. Method for the *in vitro* risk assessment of developing immune disorders, preferentially sensitivity to nosocomial pathogens in elderly, allergic asthma in neonatal subjects, atopic dermatitis or type I diabetes in a subject, comprising the steps of:
a) Determining whether a subject has a reduced gut bacterial diversity with a method according to any of claims 1 to 8; and
b) If said subject has a reduced gut bacterial diversity, assessing that said subject is at risk to develop said immune disorders.

## Patentansprüche

1. Verfahren zur Bestimmung, ob ein Proband eine reduzierte Darmbakterienvielfalt hat, wobei das Verfahren Folgendes umfasst:
a) Erfassung aus einer mikrobiellen Darm-DNA-Probe, die von dem Probanden erhalten wurde, ob
alle 50 SEQ ID NOs von zumindest einer Bakterienart, wie in Tabelle 1 definiert, in der Probe fehlen; und
b) Bestimmung, dass der Proband eine reduzierte Darmbakterienvielfalt aufweist, wenn alle 50 SEQ ID NOs der mindestens einen Bakterienart, wie in Tabelle 1 definiert, in der Probe fehlen.

2. Verfahren zur Bestimmung, ob ein Proband eine reduzierte Darmbakterienvielfalt hat, wobei das Verfahren Folgendes umfasst:
a) Erfassung aus einer mikrobiellen Darm-DNA-Probe, die von dem Probanden erhalten wurde, ob
alle 50 SEQ ID NOs von zumindest einer Bakterienart, wie in Tabelle 2 definiert, in der Probe vorhanden sind; und
b) Bestimmung, dass der Proband eine reduzierte Darmbakterienvielfalt aufweist, wenn alle 50 SEQ ID NOs der mindestens einen Bakterienart, wie in Tabelle 2 definiert, in der Probe vorhanden sind.

3. Verfahren nach Anspruch 1, **dadurch gekennzeichnet, dass** es einen Schritt zum Bestimmen aus einer mikrobiellen Darm-DNA-Probe umfasst, die von dem Probanden erhalten wurde, ob alle 50 SEQ ID NOs von zumindest einer Bakterienart wie in Tabelle 1 definiert, die aus SEQ ID NO: 1-50, SEQ ID NO: 2801-2850, SEQ ID NO: 2601-2650, SEQ ID NO: 151-200, SEQ ID NO: 2651-2700, SEQ ID NO: 51-100, SEQ ID NO: 101-150, SEQ ID NO: 351-400, SEQ ID NO: 451-500, SEQ ID NO: 2201-2250, SEQ ID NO: 1051-1100, SEQ ID NO: 1251-1300, SEQ ID NO: 401-450, SEQ ID NO: 201-250, SEQ ID NO: 501-550, SEQ ID NO: 651-700, SEQ ID NO: 601-650, SEQ ID NO: 851-900, SEQ ID NO: 551-600, SEQ ID NO: 1001-1050 ausgewählt, in der Probe fehlen.

4. Verfahren nach einem der Ansprüche 1 bis 3, **dadurch gekennzeichnet, dass** es einen Schritt zum Erfassen aus einer mikrobiellen Darm-DNA-Probe umfasst, die vom Probanden erhalten wurde,
ob alle 50 SEQ ID NOs der Bakterienarten wie in Tabelle 1 oder Tabelle 2 der Bakterienartkombinationen wie in Tabelle 7, 8 und/oder 9 angegeben definiert, in der Probe fehlen und/oder vorhanden sind.

5. Verfahren nach einem der Ansprüche 1 bis 4, **dadurch gekennzeichnet, dass** es einen Schritt zum Erfassen aus einer mikrobiellen Darm-DNA-Probe umfasst, die von dem Probanden erhalten wurde, ob die
- SEQ ID NO: 1-50 und SEQ ID NO: 201-250 in der Probe fehlen, oder;
- SEQ ID NO: 451-500, SEQ ID NO: 1-50 und SEQ ID NO: 201-250 in der Probe fehlen, oder;
- SEQ ID NO: 351-400, SEQ ID NO: 101-150, SEQ ID NO: 1251-1300 und SEQ ID NO: 2601-2650 in der Probe fehlen, oder;
- SEQ ID NO: 451-500, SEQ ID NO: 1251-1300, SEQ ID NO: 351-400, SEQ ID NO: 2601-2650 und SEQ ID NO: 101-150 in der Probe fehlen, oder;
- SEQ ID NO: 2601-2650, SEQ ID NO: 351-400, SEQ ID NO: 601-650, SEQ ID NO: 101-150, SEQ ID NO: 1251-1300 und SEQ ID NO: 1801-1850 in der Probe fehlen, oder;
- SEQ ID NO: 1801-1850, SEQ ID NO: 1251-1300, SEQ ID NO: 451-500, SEQ ID NO: 351-400, SEQ ID NO: 1001-1050, SEQ ID NO: 2601-2650 und SEQ ID NO: 501-550 in der Probe fehlen, oder;
- SEQ ID NO: 451-500, SEQ ID NO: 201-250, SEQ ID NO: 1251-1300, SEQ ID NO: 1201-1250, SEQ ID NO: 2601-2650, SEQ ID NO: 1051-1100, SEQ ID NO: 351-400 und SEQ ID NO: 801-850 in der Probe fehlen, oder;
- SEQ ID NO: 1251-1300, SEQ ID NO: 1801-1850, SEQ ID NO: 1001-1050, SEQ ID NO: 451-500, SEQ ID NO: 201-250, SEQ ID NO: 801-850, SEQ ID NO: 751-800, SEQ ID NO: 351-400 und SEQ ID NO: 101-150 in der Probe fehlen, oder;
- SEQ ID NO: 501-550, SEQ ID NO: 1301-1350, SEQ ID NO: 1701-1750, SEQ ID NO: 351-400, SEQ ID NO: 1051-1100, SEQ ID NO: 2301-2350, SEQ ID NO: 1251-1300, SEQ ID NO: 451-500 und SEQ ID NO: 1801-1850 fehlen, und SEQ ID NO: 701-750 in der Probe vorhanden sind;
- SEQ ID NO: 1351-1400, SEQ ID NO: 1001-1050, SEQ ID NO: 201-250, SEQ ID NO: 1301-1350, SEQ ID NO: 1251-1300, SEQ ID NO: 801-850, SEQ ID NO: 101-150, SEQ ID NO: 1951-2000, SEQ ID NO: 1801-1850 und SEQ ID NO: 1201-1250 fehlen, und SEQ ID NO: 1851-1900 in der Probe vorhanden sind;
- SEQ ID NO: 351-400, SEQ ID NO: 2201-2250; SEQ ID NO: 1701-1750, SEQ ID NO: 2601-2650, SEQ ID NO: 801-850, SEQ ID NO: 1251-1300, SEQ ID NO: 101-150, SEQ ID NO: 851-900, SEQ ID NO: 451-500, SEQ ID NO: 1801-1850 und SEQ ID NO: 1951-2000 fehlen, und SEQ ID NO: 701-750 in der Probe vorhanden sind;
- SEQ ID NO: 1601-1650, SEQ ID NO: 601-650; SEQ ID NO: 451-500, SEQ ID NO: 1351-1400, SEQ ID NO: 1751-1800, SEQ ID NO: 801-850, SEQ ID NO: 351-400, SEQ ID NO: 101-150, SEQ ID NO: 1051-1100, SEQ ID NO: 2601-2650, SEQ ID NO: 1701-1750, SEQ ID NO: 201-250 und SEQ ID NO: 1301-1350 in der Probe fehlen, oder;
- SEQ ID NO: 1601-1650, SEQ ID NO: 601-650; SEQ ID NO: 451-500, SEQ ID NO: 1351-1400, SEQ ID NO: 1751-1800, SEQ ID NO: 801-850, SEQ ID NO: 351-400, SEQ ID NO: 101-150, SEQ ID NO: 1051-1100, SEQ ID NO: 2601-2650, SEQ ID NO: 1701-1750, SEQ ID NO: 201-250 und SEQ ID NO: 1301-1350 in der Probe fehlen, oder;
- SEQ ID NO: 1501-1550, SEQ ID NO: 501-550; SEQ ID NO: 1201-1250, SEQ ID NO: 451-500, SEQ ID NO: 1701-1750, SEQ ID NO: 551-600, SEQ ID NO: 1351-1400, SEQ ID NO: 1801-1850, SEQ ID NO: 201-250, SEQ ID NO: 1601-1650, SEQ ID NO: 801-850, SEQ ID NO: 2501-2550, SEQ ID NO: 1301-1350 und SEQ ID NO: 1951-2000 fehlen, und SEQ ID NO: 701-750 in der Probe vorhanden sind;
- SEQ ID NO: 1601-1650, SEQ ID NO: 2501-2550, SEQ ID NO: 1301-1350, SEQ ID NO: 1501-1550, SEQ ID NO: 1101-1150, SEQ ID NO: 451-500, SEQ ID NO: 851-900, SEQ ID NO: 151-200, SEQ ID NO: 501-550, SEQ ID NO: 351-400, SEQ ID NO: 1001-1050, SEQ ID NO: 2201-2250, SEQ ID NO: 201-250 und SEQ ID NO: 801-850 in der Probe fehlen, und SEQ ID NO: 701-750, SEQ ID NO: 1901-1950 und SEQ ID NO: 2751-2800 aus der Tabelle 2 vorhanden sind, oder;
- SEQ ID NO: 2201-2250, SEQ ID NO: 1301-1350, SEQ ID NO: 2301-2350, SEQ ID NO: 201-250, SEQ ID NO: 2501-2550, SEQ ID NO: 351-400, SEQ ID NO: 1251-1300, SEQ ID NO: 101-150, SEQ ID NO: 2601-2650, SEQ ID NO: 1801-1850, SEQ ID NO: 601-650, SEQ ID NO: 501-550, SEQ ID NO: 801-850, SEQ ID NO: 1101-1150, SEQ ID NO: 1-50 und SEQ ID NO: 1351-1400 fehlen, und SEQ ID NO: 1851-1900 in der Probe vorhanden sind;
- SEQ ID NO: 1501-1550, SEQ ID NO: 501-550, SEQ ID NO: 1601-1650, SEQ ID NO: 1351-1400, SEQ ID NO: 1751-1800, SEQ ID NO: 1001-1050, SEQ ID NO: 1051-1100, SEQ ID NO: 151-200, SEQ ID NO: 1801-1850, SEQ ID NO: 2201-2250, SEQ ID NO: 1301-1350, SEQ ID NO: 701-750, SEQ ID NO: 2501-2550, SEQ ID NO: 351-400 und SEQ ID NO: 801-850 fehlen, und SEQ ID NO: 701-750, SEQ ID NO: 2401-2450, SEQ ID NO: 1851-1900 und SEQ ID NO: 2751-2800 in der Probe vorhanden sind;
- SEQ ID NO: 851-900, SEQ ID NO: 2751-2800, 1351-1400, SEQ ID NO: 1751-1800, SEQ ID NO: 2201-2250, SEQ ID NO: 801-850, SEQ ID NO: 1701-1750, SEQ ID NO: 1601-1650, SEQ ID NO: 501-550, SEQ ID NO: 201-250, SEQ ID NO: 351-400, SEQ ID NO: 451-500, SEQ ID NO: 551-600, SEQ ID NO: 1201-1250 und SEQ ID NO: 1051-1110 fehlen, und SEQ ID NO: 1901-1950, SEQ ID NO: 2401-2450, SEQ ID NO: 301-350, SEQ ID NO: 701-750 und SEQ ID NO: 2751-2800 in der Probe vorhanden sind;
- SEQ ID NO: 2301-2350, SEQ ID NO: 201-250, SEQ ID NO: 1751-1800, SEQ ID NO: 1801-1850, SEQ ID NO: 1701-1750, SEQ ID NO: 2151-2200, SEQ ID NO: 501-550, SEQ ID NO: 351-400, SEQ ID NO: 801-850, SEQ ID NO: 1501-1550, SEQ ID NO: 851-900, SEQ ID NO: 601-650, SEQ ID NO: 1001-1050, SEQ ID NO: 2501-2550, SEQ ID NO: 151-200, SEQ ID NO: 1351-1400, SEQ ID NO: 2201-2250, SEQ ID NO: 1601-1650 und SEQ ID NO: 101-150 fehlen, und SEQ ID NO: 701-750 in der Probe vorhanden sind.

6. Verfahren nach einem der Ansprüche 1 bis 5, **dadurch gekennzeichnet, dass** es einen Schritt zum Erfassen aus einer mikrobiellen Darm-DNA-Probe umfasst, die von dem Probanden erhalten wurde, ob alle der 50 SEQ ID NOs: 1-50 in der Probe fehlen.

7. Verfahren nach einem der Ansprüche 1 bis 6, **dadurch gekennzeichnet, dass** das Vorhandensein oder Fehlen der bakteriellen Gene mit Verwendung eines Nuklein-Microarray erfassen wird.

8. Verfahren nach Anspruch 7, **dadurch gekennzeichnet, dass** das Nuklein-Microarray ein Oligonukleotid-Microarray ist, das zumindest ein für zumindest 50 bakterielle Gene spezifisches Oligonukleotid umfasst, die eine aus den SEQ ID NOs 1-2900 gewählte Sequenz haben.

9. Verfahren zur *in vitro* Risikobewertung von sich entwickelnden Stoffwechselstörungen, vorzugsweise Typ II Diabetes, hyperglykämisches Syndrom, Herzerkrankungen, Insulinresistenz oder hepatischer Stase, das die folgenden Schritte umfasst:
a) Bestimmung, ob ein Proband eine reduzierte Darmbakterienvielfalt aufweist, und zwar mit einem Verfahren nach einem der Ansprüche 1 bis 8; und
b) wenn der Proband eine reduzierte Darmbakterienvielfalt aufweist, Bewertung, dass der Proband Risiko läuft, die Stoffwechselstörungen zu entwickeln.

10. Verfahren zur *in vitro* Risikobewertung von sich entwickelnden Immunerkrankungen, vorzugsweise Empfindlichkeit gegenüber nosokomialen Krankheitserregern bei älteren Menschen, Allergieasthma bei Neugeborenen, Atopie-Dermatitis oder Typ I Diabetes bei einem Probanden, das die folgenden Schritte umfasst:
a) Bestimmung, ob ein Proband eine reduzierte Darmbakterienvielfalt aufweist, und zwar mit einem Verfahren nach einem der Ansprüche 1 bis 8; und
b) wenn der Proband eine reduzierte Darmbakterienvielfalt aufweist, Bewertung, dass der Proband Risiko läuft, die Immunerkrankungen zu entwickeln.

## Revendications

1. Procédé pour déterminer si un sujet a une diversité bactérienne intestinale réduite, ledit procédé comprenant :
a) la détection, à partir d'un échantillon d'ADN microbien intestinal qui a été obtenu auprès dudit sujet, si
toutes les 50 SEQ ID NO d'au moins une espèce bactérienne comme défini dans le Tableau 1 sont absentes dans ledit échantillon ; et
b) la détermination du fait que le sujet a une diversité bactérienne intestinale réduite si toutes lesdites 50 SEQ ID NO de ladite au moins une espèce bactérienne comme défini dans le Tableau 1 sont absentes dans ledit échantillon.

2. Procédé pour déterminer si un sujet a une diversité bactérienne intestinale réduite, ledit procédé comprenant :
a) la détection, à partir d'un échantillon d'ADN microbien intestinal qui a été obtenu auprès dudit sujet, si
toutes les 50 SEQ ID NO d'au moins une espèce bactérienne comme défini dans le Tableau 2 sont présentes dans ledit échantillon ; et
b) la détermination du fait que le sujet a une diversité bactérienne intestinale réduite si toutes lesdites 50 SEQ ID NO de ladite au moins une espèce bactérienne comme défini dans le Tableau 2 sont présentes dans ledit échantillon.

3. Procédé selon la revendication 1, **caractérisé en ce qu'**il comprend une étape de détermination, à partir d'un échantillon d'ADN microbien intestinal qui a été obtenu à partir dudit sujet, de si toutes les 50 SEQ ID NO d'au moins une espèce bactérienne comme défini dans le Tableau 1, choisies parmi les SEQ ID NO : 1-50, SEQ ID NO : 2801-2850, SEQ ID NO : 2601-2650, SEQ ID NO : 151-200, SEQ ID NO : 2651-2700, SEQ ID NO : 51-100, SEQ ID NO : 101-150, SEQ ID NO : 351-400, SEQ ID NO : 451-500, SEQ ID NO : 2201-2250, SEQ ID NO : 1051-1100, SEQ ID NO : 1251-1300, SEQ ID NO : 401-450, SEQ ID NO : 201-250, SEQ ID NO : 501-550, SEQ ID NO : 651-700, SEQ ID NO : 601-650, SEQ ID NO : 851-900, SEQ ID NO : 551-600 et SEQ ID NO : 1001-1050, sont absentes dans ledit échantillon.

4. Procédé selon l'une quelconque des revendications 1 à 3, **caractérisé en ce qu'**il comprend une étape de détection, à partir d'un échantillon d'ADN microbien intestinal qui a été obtenu auprès dudit sujet, de si toutes les 50 SEQ ID NO d'espèce bactérienne comme défini dans le Tableau 1 ou le Tableau 2 de combinaisons d'espèces bactériennes indiquées dans les Tableaux 7, 8 et/ou 9 sont absentes et/ou présentes dans ledit échantillon.

5. Procédé selon l'une quelconque des revendications 1 à 4, **caractérisé en ce qu'**il comprend une étape de détection, à partir d'un échantillon d'ADN microbien intestinal qui a été obtenu auprès dudit sujet, de si :
- les SEQ ID NO : 1-50 et les SEQ ID NO : 201-250 sont absentes dans ledit échantillon, ou ;
- les SEQ ID NO : 451-500, les SEQ ID NO : 1-50 et les SEQ ID NO : 201-250 sont absentes dans ledit échantillon, ou ;
- les SEQ ID NO : 351-400, les SEQ ID NO : 101-150, les SEQ ID NO : 1251-1300 et les SEQ ID NO : 2601-2650 sont absentes dans ledit échantillon, ou ;
- les SEQ ID NO : 451-500, les SEQ ID NO : 1251-1300, les SEQ ID NO : 351-400, les SEQ ID NO : 2601-2650 et les SEQ ID NO : 101-150 sont absentes dans ledit échantillon, ou ;
- les SEQ ID NO : 2601-2650, les SEQ ID NO : 351-400, les SEQ ID NO : 601-650, les SEQ ID NO : 101-150, les SEQ ID NO : 1251-1300 et les SEQ ID NO : 1801-1850 sont absentes dans ledit échantillon, ou ;
- les SEQ ID NO : 1801-1850, les SEQ ID NO : 1251-1300, les SEQ ID NO : 451-500, les SEQ ID NO : 351-400, les SEQ ID NO : 1001-1050, les SEQ ID NO : 2601-2650 et les SEQ ID NO : 501-550 sont absentes dans ledit échantillon, ou ;
- les SEQ ID NO : 451-500, les SEQ ID NO : 201-250, les SEQ ID NO : 1251-1300, les SEQ ID NO : 1201-1250, les SEQ ID NO : 2601-2650, les SEQ ID NO : 1051-1100, les SEQ ID NO : 351-400 et les SEQ ID NO : 801-850 sont absentes dans ledit échantillon, ou ;
- les SEQ ID NO : 1251-1300, les SEQ ID NO : 1801-1850, les SEQ ID NO : 1001-1050, les SEQ ID NO : 451-500, les SEQ ID NO : 201-250, les SEQ ID NO : 801-850, les SEQ ID NO : 751-800, les SEQ ID NO : 351-400 et les SEQ ID NO : 101-150 sont absentes dans ledit échantillon, ou ;
- les SEQ ID NO : 501-550, les SEQ ID NO : 1301-1350, les SEQ ID NO : 1701-1750, les SEQ ID NO : 351-400, les SEQ ID NO : 1051-1100, les SEQ ID NO : 2301-2350, les SEQ ID NO : 1251-1300, les SEQ ID NO : 451-500 et les SEQ ID NO : 1801-1850 sont absentes, et les SEQ ID NO : 701-750 sont présentes, dans ledit échantillon ;
- les SEQ ID NO : 1351-1400, les SEQ ID NO : 1001-1050, les SEQ ID NO : 201-250, les SEQ ID NO : 1301-1350, les SEQ ID NO : 1251-1300, les SEQ ID NO : 801-850, les SEQ ID NO : 101-150, les SEQ ID NO : 1951-2000, les SEQ ID NO : 1801-1850 et les SEQ ID NO : 1201-1250 sont absentes, et les SEQ ID NO : 1851-1900 sont présentes, dans ledit échantillon ;
- les SEQ ID NO : 351-400, les SEQ ID NO : 2201-2250; les SEQ ID NO : 1701-1750, les SEQ ID NO : 2601-2650, les SEQ ID NO : 801-850, les SEQ ID NO : 1251-1300, les SEQ ID NO : 101-150, les SEQ ID NO : 851-900, les SEQ ID NO : 451-500, les SEQ ID NO : 1801-1850 et les SEQ ID NO : 1951-2000 sont absentes, et les SEQ ID NO : 701-750 sont présentes, dans ledit échantillon ;
- les SEQ ID NO : 1601-1650, les SEQ ID NO : 601-650, les SEQ ID NO : 451-500, les SEQ ID NO : 1351-1400, les SEQ ID NO : 1751-1800, les SEQ ID NO : 801-850, les SEQ ID NO : 351-400, les SEQ ID NO : 101-150, les SEQ ID NO : 1051-1100, les SEQ ID NO : 2601-2650, les SEQ ID NO : 1701-1750, les SEQ ID NO : 201-250 et les SEQ ID NO : 1301-1350 sont absentes dans ledit échantillon, ou ;
- les SEQ ID NO : 1601-1650, les SEQ ID NO : 601-650; les SEQ ID NO : 451-500, les SEQ ID NO : 1351-1400, les SEQ ID NO : 1751-1800, les SEQ ID NO : 801-850, les SEQ ID NO : 351-400, les SEQ ID NO : 101-150, les SEQ ID NO : 1051-1100, les SEQ ID NO : 2601-2650, les SEQ ID NO : 1701-1750, les SEQ ID NO : 201-250 et les SEQ ID NO : 1301-1350 sont absentes dans ledit échantillon, ou ;
- les SEQ ID NO : 1501-1550, les SEQ ID NO : 501-550, les SEQ ID NO : 1201-1250, les SEQ ID NO : 451-500, les SEQ ID NO : 1701-1750, les SEQ ID NO : 551-600, les SEQ ID NO : 1351-1400, les SEQ ID NO : 1801-1850, les SEQ ID NO : 201-250, les SEQ ID NO : 1601-1650, les SEQ ID NO : 801-850, les SEQ ID NO : 2501-2550, les SEQ ID NO : 1301-1350 et les SEQ ID NO : 1951-2000 sont absentes, et les SEQ ID NO : 701-750 sont présentes, dans ledit échantillon ;
- les SEQ ID NO : 1601-1650, les SEQ ID NO : 2501-2550, les SEQ ID NO : 1301-1350, les SEQ ID NO : 1501-1550, les SEQ ID NO : 1101-1150, les SEQ ID NO : 451-500, les SEQ ID NO : 851-900, les SEQ ID NO : 151-200, les SEQ ID NO : 501-550, les SEQ ID NO : 351-400, les SEQ ID NO : 1001-1050, les SEQ ID NO : 2201-2250, les SEQ ID NO : 201-250 et les SEQ ID NO : 801-850 sont absentes dans ledit échantillon, et les SEQ ID NO : 701-750, les SEQ ID NO : 1901-1950 et les SEQ ID NO : 2751-2800 du Tableau 2 sont présentes ou ;
- les SEQ ID NO : 2201-2250, les SEQ ID NO : 1301-1350, les SEQ ID NO : 2301-2350, les SEQ ID NO : 201-250, les SEQ ID NO : 2501-2550, les SEQ ID NO : 351-400, les SEQ ID NO : 1251-1300, les SEQ ID NO : 101-150, les SEQ ID NO : 2601-2650, les SEQ ID NO : 1801-1850, les SEQ ID NO : 601-650, les SEQ ID NO : 501-550, les SEQ ID NO : 801-850, les SEQ ID NO : 1101-1150, les SEQ ID NO : 1-50 et les SEQ ID NO : 1351-1400 sont absentes, et les SEQ ID NO : 1851-1900 sont présentes, dans ledit échantillon ;
- les SEQ ID NO : 1501-1550, les SEQ ID NO : 501-550, les SEQ ID NO : 1601-1650, les SEQ ID NO : 1351-1400, les SEQ ID NO : 1751-1800, les SEQ ID NO : 1001-1050, les SEQ ID NO : 1051-1100, les SEQ ID NO : 151-200, les SEQ ID NO : 1801-1850, les SEQ ID NO : 2201-2250, les SEQ ID NO : 1301-1350, les SEQ ID NO : 701-750, les SEQ ID NO : 2501-2550, les SEQ ID NO : 351-400 et les SEQ ID NO : 801-850 sont absentes, et les SEQ ID NO : 701-750, les SEQ ID NO : 2401-2450, les SEQ ID NO : 1851-1900 et les SEQ ID NO : 2751-2800 sont présentes, dans ledit échantillon ;
- les SEQ ID NO: 851-900, les SEQ ID NO : 2751-2800, 1351-1400, les SEQ ID NO : 1751-1800, les SEQ ID NO : 2201-2250, les SEQ ID NO: 801-850, les SEQ ID NO: 1701-1750, les SEQ ID NO: 1601-1650, les SEQ ID NO: 501-550, les SEQ ID NO: 201-250, les SEQ ID NO : 351-400, les SEQ ID NO : 451-500, les SEQ ID NO : 551-600, les SEQ ID NO: 1201-1250 et les SEQ ID NO: 1051-1110 sont absentes, et les SEQ ID NO : 1901-1950, les SEQ ID NO : 2401-2450, les SEQ ID NO : 301-350, les SEQ ID NO : 701-750 et les SEQ ID NO : 2751-2800 sont présentes, dans ledit échantillon ;
- les SEQ ID NO : 2301-2350, les SEQ ID NO : 201-250, les SEQ ID NO : 1751-1800, les SEQ ID NO : 1801-1850, les SEQ ID NO : 1701-1750, les SEQ ID NO : 2151-2200, les SEQ ID NO : 501-550, les SEQ ID NO : 351-400, les SEQ ID NO : 801-850, les SEQ ID NO : 1501-1550, les SEQ ID NO : 851-900, les SEQ ID NO : 601-650, les SEQ ID NO : 1001-1050, les SEQ ID NO : 2501-2550, les SEQ ID NO : 151-200, les SEQ ID NO : 1351-1400, les SEQ ID NO : 2201-2250, les SEQ ID NO : 1601-1650 et les SEQ ID NO : 101-150 sont absentes, et les SEQ ID NO : 701-750 sont présentes, dans ledit échantillon.

6. Procédé selon l'une quelconque des revendications 1 à 5, **caractérisé en ce qu'**il comprend une étape de détection, à partir d'un échantillon d'ADN microbien intestinal qui a été obtenu auprès dudit sujet, de si toutes lesdites SEQ ID NO : 1-50 sont absentes dans ledit échantillon.

7. Procédé selon l'une quelconque des revendications 1 à 6, **caractérisé en ce que** la présence ou l'absence des gènes bactériens est détectée par l'utilisation d'une puce nucléique.

8. Procédé selon la revendication 7, **caractérisé en ce que** la puce nucléique est une puce d'oligonucléotides comprenant au moins un oligonucléotide spécifique d'au moins 50 gènes bactériens ayant une séquence choisie parmi les SEQ ID NO : 1-2900.

9. Procédé d'évaluation *in vitro* d'un risque de développement de troubles métaboliques, de préférence du diabète de type II, du syndrome hyperglycémiant, de maladies cardiaques, d'une résistance à l'insuline ou d'une stase hépatique, comprenant les étapes de :
a) détermination de si un sujet a une diversité bactérienne intestinale réduite au moyen d'un procédé selon l'une quelconque des revendications 1 à 8 ; et
b) si ledit sujet a une diversité bactérienne intestinale réduite, évaluation que ledit sujet présente un risque de développer lesdits troubles métaboliques.

10. Procédé d'évaluation *in vitro* d'un risque de développement de troubles immunitaires, de préférence d'une sensibilité à des pathogènes nosocomiaux chez les sujets âgés, d'un asthme allergique chez les sujets nouveau-nés, d'une dermatite atopique ou du diabète de type I chez un sujet, comprenant les étapes de :
a) détermination de si un sujet a une diversité bactérienne intestinale réduite au moyen d'un procédé selon l'une quelconque des revendications 1 à 8 ; et
b) si ledit sujet a une diversité bactérienne intestinale réduite, évaluation que ledit sujet présente un risque de développer lesdits troubles immunitaires.
